# EUROPEAN PATENT APPLICATION

(11) **EP 2 647 647 A2**
(43) Date of publication of application: **09.10.2013**
(21) Application number: 12190104.5
(22) Date of filing: 06.08.2008
(51) Int. Cl.: C07K 14/435, C07K 7/08, C07K 14/36, C07K 5/078, C07D 513/22

(54) **Thiopeptide precursor protein, gene encoding it and uses thereof**

(30) Priority: 09.08.2007 EP 07114121
(62) Divisional of application: 08802965.7
(71) Applicant: Novartis AG, 4056 Basel (CH)
(72) Inventor: Morris, Rowan, 4056 Basel (CH)
(74) Representative: Didelon, Frédéric

(57) **Abstract**

The present invention relates to the precursor proteins for thiopeptide biosynthesis, and the corresponding structural genes and uses thereof. The present invention also relates to methods for genetically manipulating the thiopeptide precursor protein or host cells expressing a gene encoding said thiopeptide precursor protein to produce thiopeptide compounds or their derivatives. The present invention further relates to the cloning and characterization of genes involved in thiopeptide biosynthesis and their use in thiopeptide compounds production

SEQUENCE LISTING

<110>  Novartis AG

<120>  thiopeptide gene cluster

<130>  I315

<160>  65

<170>  PatentIn version 3.2

<210>  1
<211>  14
<212>  PRT
<213>  nonomuraea sp.

<400>  1

Ser Cys Asn Cys Val Cys Gly Phe Cys Cys Ser Cys Ser Pro
1               5                   10


<210>  2
<211>  14
<212>  PRT
<213>  nonomuraea sp.

<400>  2

Ser Ser Asn Cys Phe Cys Tyr Pro Cys Cys Ser Cys Ser Ser
1               5                   10


<210>  3
<211>  14
<212>  PRT
<213>  nonomuraea sp.

<400>  3

Ser Cys Asn Cys Phe Cys Tyr Ile Cys Cys Ser Cys Ser Ser
1               5                   10


<210>  4
<211>  14
<212>  PRT
<213>  nonomuraea sp.

<400>  4

Ser Cys Asn Cys Val Cys Gly Val Cys Cys Ser Cys Ser Pro
1               5                   10


<210>  5
<211>  13
<212>  PRT
<213>  nonomuraea sp.

<400>  5

Ser Cys Thr Thr Cys Val Cys Thr Cys Ser Cys Cys Thr
1               5                   10


<210>  6
<211>  17

<212>  PRT
<213>  nonomuraea sp.

<400>  6

Ile Ala Ser Ala Ser Cys Thr Thr Cys Ile Cys Thr Cys Ser Cys Ser
1               5                   10                  15

Ser

<210>  7
<211>  12
<212>  PRT
<213>  nonomuraea sp.

<400>  7

Ser Cys Thr Thr Cys Glu Cys Cys Cys Ser Cys Ser
1               5                   10

<210>  8
<211>  13
<212>  PRT
<213>  nonomuraea sp.

<400>  8

Ser Cys Thr Thr Cys Val Cys Val Cys Ser Cys Cys Thr
1               5                   10

<210>  9
<211>  13
<212>  PRT
<213>  nonomuraea sp.

<400>  9

Ser Cys Val Gly Ser Ala Cys Ala Ser Ser Ser Ser Ser
1               5                   10

<210>  10
<211>  12
<212>  PRT
<213>  nonomuraea sp.

<220>
<221>  misc_feature
<222>  (6)..(6)
<223>  Xaa can be any naturally occurring amino acid

<220>
<221>  misc_feature
<222>  (8)..(8)
<223>  Xaa can be any naturally occurring amino acid

<400>  10

Ser Cys Thr Thr Cys Xaa Cys Xaa Cys Ser Cys Ser
1               5                   10

<210>  11

<211>  15
<212>  PRT
<213>  nonomuraea sp.

<400>  11

Ser Cys Thr Thr Ser Ser Val Ser Ser Ser Ser Ser Ser Ser Ser
1               5                   10                  15


<210>  12
<211>  16
<212>  PRT
<213>  nonomuraea sp.

<400>  12

Ser Cys Thr Thr Ser Gly Cys Thr Ser Ser Ser Ser Ser Ser Ser Ser
1               5                   10                  15


<210>  13
<211>  17
<212>  PRT
<213>  nonomuraea sp.

<400>  13

Ser Cys Thr Thr Ser Gly Ser Ala Cys Ser Ser Ser Ser Ser Ser Ser
1               5                   10                  15


Ser



<210>  14
<211>  15
<212>  PRT
<213>  nonomuraea sp.

<400>  14

Ser Cys Val Gly Ser Ala Cys Ala Cys Ser Ser Ser Ser Ser Ser
1               5                   10                  15


<210>  15
<211>  42
<212>  DNA
<213>  nonomuraea sp.

<400>  15
tcctgcaact gcgtgtgcgg cttctgctgc tcctgcagcc cg                          42


<210>  16
<211>  42
<212>  DNA
<213>  nonomuraea sp.

<400>  16
tcgtgtaact gcttctgcta catctgctgc tcctgctcga gc                          42


<210>  17
<211>  174
<212>  DNA
<213>  nonomuraea sp.

<400> 17
atgagcgagc tggaatccaa gctgaacctt agcgacctgc ccatggacgt cttcgagatg 60

gccgacagcg gcatggaggt cgaatccctc accgcaggcc acgggatgcc cgaggtaggc 120

gcttcctgca actgcgtgtg cggcttctgc tgctcctgca gcccgtccgc gtag 174


<210> 18
<211> 150
<212> DNA
<213> nonomuraea sp.

<400> 18
atggacctca gcgatctgcc catggacgtc ttcgaactcg ccgatgacgg tgttgcggtc 60

gagtccctta ctgctggtca cggcatgacc gaagtgggag cttcgtgtaa ctgcttctgc 120

tacatctgct gctcctgctc gagcgcctga 150


<210> 19
<211> 57
<212> PRT
<213> nonomuraea sp.

<400> 19

Met Ser Glu Leu Glu Ser Lys Leu Asn Leu Ser Asp Leu Pro Met Asp
1               5                   10                  15


Val Phe Glu Met Ala Asp Ser Gly Met Glu Val Glu Ser Leu Thr Ala
            20                  25                  30


Gly His Gly Met Pro Glu Val Gly Ala Ser Cys Asn Cys Val Cys Gly
        35                  40                  45


Phe Cys Cys Ser Cys Ser Pro Ser Ala
    50                  55


<210> 20
<211> 49
<212> PRT
<213> nonomuraea sp.

<400> 20

Met Asp Leu Ser Asp Leu Pro Met Asp Val Phe Glu Leu Ala Asp Asp
1               5                   10                  15


Gly Val Ala Val Glu Ser Leu Thr Ala Gly His Gly Met Thr Glu Val
            20                  25                  30


Gly Ala Ser Cys Asn Cys Phe Cys Tyr Ile Cys Cys Ser Cys Ser Ser
        35                  40                  45


Ala



<210> 21

<211>  414
<212>  PRT
<213>  nonomuraea sp.

<400>  21

Met Pro Thr Thr Leu Pro Lys Thr Leu Arg Gly Ser Asp Gly Ala Leu
1               5                   10                  15

Ala Glu Gln Pro Leu Leu Ile Tyr Val Asn Val Pro Phe Cys Asn Ser
            20                  25                  30

Lys Cys His Phe Cys Asp Trp Val Thr Glu Val Pro Leu Ala Asp Leu
        35                  40                  45

Arg Leu Thr Pro Asp Ser Ser Pro Arg Arg Arg Tyr Val Ala Ala Leu
    50                  55                  60

Val Lys Gln Ile Glu Thr His Ala Pro Thr Leu Gly Gly Leu Gly Tyr
65                  70                  75                  80

Arg Pro Glu Val Met Tyr Trp Gly Gly Gly Thr Ala Ser Ile Leu Thr
                85                  90                  95

Ile Glu Glu Ile Glu Ala Val Ala Gly Ala Leu Ala Ala Arg Phe Asp
            100                 105                 110

Leu Gly Gly Leu Val Glu Ala Thr Ile Glu Gly Ser Pro Glu Ser Met
        115                 120                 125

Asp Pro Gly Lys Leu Lys Leu Phe Arg Ala Ile Gly Phe Asn Arg Ile
    130                 135                 140

Ser Ile Gly Val Gln Ala Phe Asp Asp Ala Arg Leu Arg Arg Ile Gly
145                 150                 155                 160

Arg Val His Ser Ala Glu Gln Ala Glu Arg Ala Val Arg Met Ala Ala
                165                 170                 175

Asp Ala Gly Phe Asp Asn Ile Asn Ile Asp Leu Ile Val Gly Phe Pro
            180                 185                 190

Gly Gln Gly Ala Asp Glu Val Ser Arg Thr Ile Gln His Ala Val Thr
        195                 200                 205

Leu Pro Val Asn His Phe Ser Val Tyr Pro Tyr Arg Pro Thr Gly Gly
    210                 215                 220

Thr Val Met Arg Arg Gln Val Gly Arg Gly Met Gly Arg Ile Asp Val
225                 230                 235                 240

Glu Glu Gln Leu Arg Ser Tyr Asp His Ala Arg Asp Leu Leu Ala Arg
                245                 250                 255

His Gly Phe Glu Glu Tyr Ala Thr Ala Tyr Phe Gly Ala Pro Arg Cys
            260                 265                 270

Glu Ser Asp Glu Val Tyr Tyr Lys Leu Thr Met Asp Trp Ile Gly Phe
        275                 280                 285

Gly Ser Gly Ala Asn Ser Leu Ile Gly Thr Arg Phe Leu Leu Asn Asp
    290                 295                 300

Arg Gly Gly Leu His Arg Phe Ser Thr Ala Pro His Arg Phe Asp Ser
305                 310                 315                 320

Asp Thr Pro Ala Ser Ala Pro His Leu Thr Arg His Phe Leu Ala Gln
                325                 330                 335

Ala Leu Thr Thr Val Asp Gly Met Asp Ala Arg Thr Phe Gln Leu Arg
            340                 345                 350

Thr Gly Arg Ser Leu Arg Ala Ala Cys Glu Glu Pro Ala Val Arg Arg
        355                 360                 365

Met Leu Glu Gln Ile Asn Arg Arg Gly Arg Leu Ile Val Asp Ser Arg
    370                 375                 380

Gly Ile Arg Leu His Arg Asp Asp Met Ala Ser Ala Tyr Ile Thr Met
385                 390                 395                 400

Asn Ser Val Asp Leu Tyr Ala Ala Thr Glu Gln Pro Gly Gly
                405                 410

<210>  22
<211>  1242
<212>  DNA
<213>  nonomuraea sp.

<400>  22
atgccgacca cactcccgaa gaccctgcgc gggtcggacg gcgcgctcgc cgagcagccg      60

ctgctcatct acgtgaatgt gccgttctgc aactcaaaat gtcacttctg cgactgggtg     120

accgaggttc cgctggcgga tctgcggctc acgccggact cgtcgccacg ccgccgctac     180

gtcgccgcgc tggtcaagca gatcgagacg cacgcgccga cgctgggcgg actcggctac     240

cggcccgagg tcatgtactg gggcggcggc acggccagca tcctcaccat cgaggagatc     300

gaggcggtgg ctggggcgct ggcggcgcgg ttcgacctcg gcggcctggt cgaggccacc     360

atcgagggca gcccggagtc catggacccc ggcaagctga agctgttccg tgcgatcggg     420

ttcaaccgga tcagcatcgg ggtgcaggcg ttcgacgacg cgcgcctgcg ccgcatcggc     480

cgggtgcaca gcgccgagca ggcggagcgc gcggtccgga tggccgccga cgccggcttc     540

gacaacatca acatcgacct catcgtcggg ttccccgggc agggcgccga cgaggtgtcg     600

cgcacgatcc agcacgcggt gacgctgccg gtcaaccact tctcggtcta cccctaccgg     660

ccgaccggcg gcaccgtgat gcgccggcag gtgggccgcg gcatgggccg gatcgacgtg 720

gaggagcagc tccggtcgta cgaccacgcc cgcgacctgc tcgcccggca cgggttcgag 780

gagtacgcca ccgcctactt cggcgctccc cgctgcgagt ccgacgaggt ctactacaag 840

ctcacgatgg actggatcgg gttcggctcc ggggccaact ccctcatcgg gacccgcttc 900

ctgctgaacg accggggcgg gctgcaccgc ttcagcacgg ccccgcaccg cttcgactcc 960

gacaccccgg cctcggcccc ccacctgaca cgccacttcc tggcccaggc gctgaccacg 1020

gtcgacggca tggacgcgcg caccttccag ctgcgcaccg gccgttcgct gcgggcggcg 1080

tgcgaggagc ccgcggtgcg ccggatgctc gaacagatca accgccgcgg caggctgatc 1140

gtcgactcac gcggcatccg gctgcaccgc gacgacatgg cctcggccta catcaccatg 1200

aacagcgtcg atctgtacgc cgcgaccgag cagcccggcg ga 1242

<210> 23
<211> 827
<212> PRT
<213> nonomuraea sp.

<400> 23

Val Pro Pro Ala Gln Ala Gly Gly Thr Pro Pro Gly Val Arg Arg Gln
1               5                   10                  15

Ile Met Glu Val Gly Arg Glu Ile Val Val Arg Val Ala Gly Leu Pro
            20                  25                  30

Ala Ala Val Leu Ala Asp Leu Arg Leu Pro His Thr Ala Glu Leu Val
        35                  40                  45

Thr His Leu Ser Ala Glu Arg Arg Arg Leu Ala Ala Glu Ala Ala Ala
    50                  55                  60

Leu Ser Gly Glu Leu Phe Asp Leu Ile Gly Ala Ala Gly Ser Ala Arg
65                  70                  75                  80

Ala Ala Leu Val Gly Leu Arg Arg Ala Leu Ala Pro Gly His Arg Pro
                85                  90                  95

Pro Ser Pro Arg Leu Ile Gly Leu Cys Pro Leu Pro Ala Pro Leu Ala
            100                 105                 110

Glu Arg Val Thr Ala Trp Val Arg Ala Arg Tyr Asp Trp Glu Asp Arg
        115                 120                 125

Arg Arg Asp Leu Ala Gly Thr Leu Asp Lys Glu Arg Ala Asp Ala Leu
    130                 135                 140

Asp Arg Val Arg Ala Ala Cys Val Ala Pro Ala Phe Arg Arg Gly Leu
145                 150                 155                 160

Ala Leu Ser Gly Gly Glu Leu Thr Ser Thr Leu Glu Arg Trp Leu Ala

165                 170                 175

Asp Pro Gly Arg Ala Pro Arg Gln Gly Lys Val Leu Arg Leu Val Lys
            180                 185                 190

Tyr Leu Thr Arg Ala Ala Ala Lys Thr Ser Pro Tyr Gly Ser Phe Met
        195                 200                 205

Val Ser Ala Leu Ala Gly Arg Pro Gly Asp Asp Pro Pro Ile Pro Glu
    210                 215                 220

Leu Leu Thr Val Ala Glu Pro Pro Gly Ala Phe Leu Asp Ala Val Gly
225                 230                 235                 240

Asp Ala Leu Leu Ala Asp Pro Ala Leu Ala Gly Gln Val Pro Leu Arg
                245                 250                 255

Pro Asn Pro Ser Leu Thr Trp Thr Ala Gln Gly Leu Leu Phe Val Arg
            260                 265                 270

Thr Val Arg Ala Ala Ala Gly Glu Gln Ala Gly Pro Lys Glu Glu Ile
        275                 280                 285

Ala Thr Ala Gly Arg Ala Ala Ala Leu Glu Leu Cys Leu Arg His Ala
    290                 295                 300

Glu Ser Arg Pro Thr Ala Pro Gln Leu Ala Glu Leu Leu Ala Glu Ala
305                 310                 315                 320

Gly Ala Asp Pro Gly Glu Ala Ala Ala Phe Val Asp Arg Leu Val Ala
                325                 330                 335

Ala Gln Leu Leu Leu Pro Cys Pro Pro Val His Asp Asp Asp Pro Asp
            340                 345                 350

Pro Phe Gly Ala Trp Ala Arg Gln Val Cys Ala Pro Glu Leu Arg Glu
        355                 360                 365

Leu Ala Ala Ala Ser Arg Pro Val Ala Ala Ala Val Asp Ser Pro Gly
    370                 375                 380

Gln Arg Arg Ala Arg Ile Ala Glu Ala Ala Ala Ala Val Ala Asp Arg
385                 390                 395                 400

Leu Gly Ile Asp Pro Pro Ala Asp Pro Ala His Glu His Asp Val Ser
                405                 410                 415

Thr Gly Arg Pro Ala Pro Pro Pro Leu Pro Ala Gly Val Leu Ala Asp
            420                 425                 430

Leu Asp Ala Val Arg Arg Trp Leu Ala Val Phe Asp Trp Lys Val Pro
        435                 440                 445

Val Arg Val Gly Val Gly Ala Phe Cys Arg Glu Arg Phe Gly Ser Gly
    450                 455                 460

Ser Arg Thr Pro Phe Leu Glu Ala Cys Arg Ala Ala Thr Ala Ala Leu
465                 470                 475                 480

Pro His Leu Phe Gly Pro Ala Ala Met Pro Trp Phe Leu Asp Leu Thr
                485                 490                 495

Gly Asp Gly Arg Leu Arg Glu Leu Asp Arg Leu Arg Thr Arg Ala Arg
            500                 505                 510

Asp Leu Ala Arg Ser Gly Val Leu Asp Arg Arg Arg Val Leu Ala Asp
        515                 520                 525

Thr Ala Asp Trp Pro Ala Trp Leu Thr Trp Pro Ala Ser Thr Gly Phe
    530                 535                 540

Tyr Leu Gln Thr Leu Pro Gly Gly Val Val Leu Asn Ala Val His Ala
545                 550                 555                 560

Gly His Gly Arg Ala Ala Gly Arg Val His His Leu Leu Ala Arg Ala
                565                 570                 575

Gly Ala Ala Pro Pro Arg Pro Pro Arg Ser Gly Leu Pro Arg Ala Glu
            580                 585                 590

Phe Gly Gly Arg Phe Gly Ser Ala Leu Asn Thr Arg Thr Pro Ser Thr
        595                 600                 605

Arg Tyr Glu Ile Asp His Pro Gly Ala Thr Ser Gly Arg Asp Pro Arg
    610                 615                 620

His Arg Val Pro Leu Gly Thr Leu Met Val Val His Asp Pro Asp Thr
625                 630                 635                 640

Asp Leu Val His Leu His Ser Asp Arg Leu Gly Arg Val Glu Pro Val
                645                 650                 655

His Leu Gly Met Met Gly Glu Leu Gly Leu Pro Ala Val Ala Gly Phe
            660                 665                 670

Leu Glu Arg Ala Phe Ala Pro Thr Tyr Leu Phe His Pro Ser Val Pro
        675                 680                 685

Pro Phe Ile Ser Leu Arg Asp Leu Ala Gly Ala Ser Ser Ala Arg Arg
    690                 695                 700

Phe Pro Arg Val Ser Val Gly Asp Val Val Val Gln Arg Ala Arg Trp
705                 710                 715                 720

Thr Val Pro Ala Gly Leu Val Pro Ala Arg Thr Gly Ala Asp Gly Asp
                725                 730                 735

His Leu Leu Ala Leu Ala Glu Trp Arg Arg Glu His Gly Ile Pro Glu
            740                 745                 750

Arg Cys Phe Val Arg Gly Trp Lys Pro Gly Ala Ala Leu Gly Lys Ala
        755                 760                 765

Arg Lys Pro Leu Tyr Val Asp Phe Ala Ser Trp His Leu Val Ala Leu
    770                 775                 780

Phe Glu Arg Glu Ala Arg Thr Asn Ala Ala Leu Val Ile Asp Glu Ala
785                 790                 795                 800

Leu Pro Asp Pro Leu Ala Asp Gly Ala Pro Ala His Val Thr Glu Tyr
                805                 810                 815

His Val Glu Ile Gly Asp Arg Glu Arg Glu Gln
            820                 825

<210> 24
<211> 299
<212> PRT
<213> nonomuraea sp.

<400> 24

Met Thr Glu Arg Pro Gly Trp Phe Ala Ala His Val His Tyr Arg Gly
1               5                   10                  15

Asp Leu Asp Ala Leu Leu Arg Glu Ala Val Ala Pro Leu Val Arg Ala
            20                  25                  30

Leu Ala Ala Asp Phe Phe Phe Leu Arg Tyr Trp Asp Gly Gly Ser His
        35                  40                  45

Leu Arg Leu Arg Leu Arg Gly Gly Asp Gln Val Ala Val Ala Arg His
    50                  55                  60

Leu Asp Ala Tyr Leu Ala Ala His Pro Ala Pro Glu Thr Thr Ser Gln
65                  70                  75                  80

Glu Glu Tyr Ala Arg Val Ala Pro Val Leu Ala Ala Arg Glu Gly Met
                85                  90                  95

Thr Gly His Leu Thr Thr Leu Arg Pro Asn Asn Thr Val Glu Phe Ala
            100                 105                 110

Ala Tyr Arg Pro Glu Thr Ala Lys Tyr Gly Thr Gly Asp Ala Leu Arg
        115                 120                 125

Ala Val Glu Arg His Phe Val Glu Ser Ser Arg Tyr Ala Leu Asp Val

130                 135                 140

Leu Asp Arg Arg Pro Thr Gly Asn Gln Arg Glu Leu Ala Val Leu Gly
145                 150                 155                 160

Val Leu Leu Leu Ala Trp Tyr Ala Ala Arg Pro Leu Asp Glu Pro Pro
                165                 170                 175

Gly Glu Pro Val Asp Asp Glu Leu Ile Gly Ala Val Glu Ala Leu Cys
            180                 185                 190

Arg Gly Trp Arg Gly Gly Arg Asp Leu Pro Glu Glu Leu Val Ala Glu
        195                 200                 205

Glu Tyr Gly Arg Val Arg Glu Arg Val Ala Gly Leu Ala Gly Ala Leu
    210                 215                 220

Arg Asp Leu Val Pro Asp Pro Asp Ala Pro Gly Ser Ser Met Arg Ala
225                 230                 235                 240

Trp Ala Ala Thr Phe Asp Arg Leu Ala Ala Ala Leu Pro Pro Pro Asp
                245                 250                 255

Arg Leu Arg Val Leu Asp Thr Cys Ala His Leu Ala Ala Asn Arg Leu
            260                 265                 270

Gly Val Ser Met Ala Ala Glu Val Arg Leu Arg Leu Leu Ala Ala Arg
        275                 280                 285

Ala Leu Arg Glu Val Ala Pro Val Gly Arg Arg
    290                 295

<210>  25
<211>  333
<212>  PRT
<213>  nonomuraea sp.

<400>  25
   Met Ser Trp Arg Arg Val Asp Val Ala Tyr His Asp Pro Asp Leu Asp
   1               5                   10                  15
   Gly Leu Ile Leu Ala Thr Arg Pro Leu Leu Ala Gly Thr Pro Gly Arg
               20                  25                  30
   Gly Trp Phe Gln Arg His Trp Val Arg Gly Pro His Leu Glu Leu Trp
           35                  40                  45
   Phe Asp Ala Ala Gln Pro Ser Trp Glu Arg Ile Arg Asp Val Leu Glu
       50                  55                  60
   Pro Trp Leu Arg Val Asn Pro Ser Arg Ala Arg Ile Asp Arg Asp Arg
   65                  70                  75                  80
   Leu Leu Ala Gln His Arg His Leu Ala Ala Ala Glu Arg Ile Asp Glu
                   85                  90                  95
   Pro Leu Leu Pro Phe Tyr Ala Asp Asn Thr Leu His Arg Ala Ala Pro
               100                 105                 110
   Arg Ser Arg Ala His Val Leu Gly Gly Pro Ala Ala Glu Glu Leu Phe
           115                 120                 125
   His Asp Phe His Thr Thr Ala Ser Ala Val Ala Tyr Asp Glu Leu Asp
       130                 135                 140
   Ala Val Arg Ala Gly Glu Ser Arg Leu Val Met Ala Leu Asp Leu Met
   145                 150                 155                 160
   Val Ala Ala Ala His Ala His Ala Glu Gly Gly Val Arg Gly Gly Phe
                   165                 170                 175
   Val Ser Phe Arg Ser His Ala Glu Ala Phe Leu Ala Ser Ala Pro Gly
               180                 185                 190
   Leu Arg Glu Arg Trp Asp Ala Glu Tyr Ala Ala Arg Ala Gly Ala Leu
           195                 200                 205
   Arg Ala Arg Ile Thr Ala Val Val Ala Gly Ile Pro Arg Gly Arg Ala
       210                 215                 220
   Trp Ala Gly Leu Leu Asp Arg Phe Ala Asp Arg Gly Asp Glu Leu Ile
   225                 230                 235                 240
   Ala Ser Gly Ala Leu Leu Val Glu Pro Ala Gly Pro Asp Ala Val Ala
                   245                 250                 255
   Arg Pro Asp Thr Ala Phe His Arg Ala Leu Arg Gly Asn Arg Thr Trp
               260                 265                 270
   His Glu Glu Val Leu Arg Ser Ala Pro Phe Arg Arg Tyr Arg Leu Leu
           275                 280                 285
   Leu Asn Leu Thr Tyr Leu Gln Leu Ser Arg Leu Gly Val Asn Ala Val
       290                 295                 300
   Gln Arg Ala Leu Leu Cys His Phe Ala Ala Ser Ala Val Glu Gln Glu
   305                 310                 315                 320
   Tyr Gly Val Ser Ala Ile Glu Ile Ala Met Gly Gly Ala
                   325                 330

<210>  26
<211>  455
<212>  PRT
<213>  nonomuraea sp.

<400> 26
   Val Met Ser Val Gln Val Glu Pro Ala Val Thr His Arg Trp Ala Leu
   1               5                   10                  15
   Arg Pro Gly Val Tyr Arg Ala Thr Ala Pro Gly Gly Asp Leu Met Leu
               20                  25                  30
   Ala Ala Trp Pro His Ala Thr Pro Leu Gly Arg Ala Thr Pro Glu Leu
           35                  40                  45
   Leu Ala Leu Leu Asp Gly Leu Ala Ala Gly Ser Leu Pro Leu Asp Glu
       50                  55                  60
   Pro Gly Asp Leu Ala Pro Ala Val Glu Leu Leu Arg Ala Gly Gly Trp
   65                  70                  75                  80
   Leu Arg Lys Thr Val Ala Tyr Ala Gly Arg Asp Leu Tyr Thr Ile Thr
                   85                  90                  95
   Pro Val Ala Ala Pro Thr Asp Ala Pro Pro Pro Pro Arg Asp Glu Pro
               100                 105                 110
   Ala Leu Ser Arg Phe Ala Leu Leu Arg Asn Thr Ala Asp Gly Leu Val
           115                 120                 125
   Leu Glu Met Pro Gly Ser Trp Cys Asp Val His Val His Asp Pro Ala
       130                 135                 140
   Val Ala Ala Leu Leu Ala Asp Pro Ser Gly Asp Gly Gly Leu Pro Ala
   145                 150                 155                 160
   Glu Ala Ala Ala Ala Leu Arg Ala Asp Leu Ala Ala Ala Gly Met Leu
                   165                 170                 175
   Met Glu Arg Glu Glu Pro Phe Glu Arg Arg Gln Trp Ser Thr His Glu
               180                 185                 190
   Leu Trp Phe His Glu Arg Ser Arg Leu Gly Asn Arg Gly Trp Phe Gly
           195                 200                 205
   Ala His Phe Gly Gly Thr Phe Trp Ala Arg Gly Arg His Asp Pro Pro
       210                 215                 220
   Pro Ala Arg Pro Ser Pro Tyr Pro Gly Pro Ala Val Glu Leu Ala Arg
   225                 230                 235                 240
   Pro Asp Leu Ala Ala Leu Arg Arg Thr Asp Pro Thr Leu Thr Ala Val
                   245                 250                 255
   Leu Glu Asp Arg Glu Ser Val Arg Asp His Asp Asp Asp Asp Pro Ile
               260                 265                 270
   Ser Ala Glu Gln Leu Gly Glu Leu Leu Tyr Arg Cys Ala Arg Val Arg
           275                 280                 285
   Leu Leu Arg His Leu Glu Gly Phe Glu Tyr Ala Gly Lys Pro Tyr Pro
       290                 295                 300
   Ala Gly Gly Ser Ala Tyr Glu Leu Glu Val Tyr Pro Val Val Arg Leu
   305                 310                 315                 320
   Ala Arg Gly Leu Asp Pro Gly Met Tyr His Tyr Asp Ala His Asp His
                   325                 330                 335
   Arg Leu Arg Gln Val Arg Pro Ala Gly His Pro Ala Val Arg Arg Met
               340                 345                 350
   Leu Lys Val Ala Thr Glu Ser Ser Val Ala Lys Ala Pro Pro Gln Val
           355                 360                 365
   Leu Leu Val Ile Ser Ala Arg Val Gly Arg Ile Leu Trp Lys Tyr Glu
       370                 375                 380
   Ala Met Gly Tyr Ala Leu Met Leu Lys His Val Gly Val Leu Gln Gln
   385                 390                 395                 400
   Thr Leu Tyr Ala Val Ala Thr Ala Met Asp Leu Ala Pro Cys Ala Leu
                   405                 410                 415
   Gly Ser Gly Asp Asp Leu Ala Phe Thr Ala Ala Thr Gly Arg Asp Arg
               420                 425                 430
   Leu Val Glu Cys Ala Val Gly Glu Phe Met Val Gly Ser Arg Arg Lys
           435                 440                 445
   Glu Pro Ala Ala Trp Glu Leu
       450                 455

<210>  27
<211>  608
<212>  PRT
<213>  nonomuraea sp.

<400>  27

Met Gly Ala Leu Asn Leu Leu Leu Arg Pro Asp Ala His Tyr Ala Glu
1               5                   10                  15

Val Asp Gly Gly Val Tyr Val Leu Ser His Gln Gly Glu Thr Phe Ile
            20                  25                  30

Ser Gly Thr Ser Val His Arg Trp Leu Asp Arg Leu Ala Pro Leu Leu
        35                  40                  45

Asp Gly Thr Arg Thr Leu Asp Gln Leu Thr Ala Gly Leu Pro Ala Asp
    50                  55                  60

Arg Ala Gly Phe Val Ala Arg Leu Val Gly Thr Leu Thr Glu Arg Gly
65                  70                  75                  80

Leu Val Arg Glu Val Gly Pro Ala Val Ser Asp Thr Leu Thr Asp Ala
                85                  90                  95

Glu Arg Gly Glu His Arg Ala Leu Leu Ala Tyr Leu Gly Tyr Phe His
            100                 105                 110

Asp Ser Pro Gly Arg Val Leu Glu Asp Val Arg Asp Thr Pro Thr Ala
        115                 120                 125

Ile Val Gly Ser Gly Pro Leu Ala Ala Ala Val Val Arg Ala Cys Ala
    130                 135                 140

Ala Ala Gly Leu Arg Arg Val Glu Ile Gly Glu Glu Ala Gly Ala Ala
145                 150                 155                 160

Arg Val Val Val His Val Ala Glu Arg Ala Glu Pro Glu Arg Ala Ala
                165                 170                 175

Arg Leu Glu Arg Arg Cys Ala Ala Glu Gly Val Leu Leu Ala Gln Val
            180                 185                 190

Met Pro Gly Pro Asp Gly Ile Trp Trp Gln Pro Ala Ala Arg Gly Gly
        195                 200                 205

Cys Trp Ala Ser Ala Trp Arg Arg His Arg Ala Leu Ala Gly Val Asp
    210                 215                 220

Arg Thr Gly Thr Pro Leu Asp Pro Val Ala Val Ala Val Val Ala Gly
225                 230                 235                 240

Gln Val Ala His Asp Val Phe Arg Val Leu Ala Gly Leu Arg Glu Glu
                245                 250                 255

Ala Pro Pro Arg Leu Val Val Leu Asp Pro Arg Thr Leu Ala Ser Thr
            260                 265                 270

Ala His Pro Val Val Ala His Pro Phe Glu Leu Pro Ala Ala Pro Leu
        275                 280                 285

Asp Glu Ala Ala Phe Leu Asp Arg Val Ala Ala Leu Arg Ala Ala Pro
    290                 295                 300

Ala Leu Ser Glu Ala Glu Phe Ser Arg Arg Ala Lys Gly Leu Met Asp
305                 310                 315                 320

Ser Thr Val Gly Leu Phe Ala Glu Ile Asp Glu Gly Asp Leu Ala Gln

325                 330                 335

Leu Pro Leu His Val Thr Ala Thr Thr Val Ala Asp Pro Cys Gly Leu
            340                 345                 350

Ile Gly Ser Ala Pro Arg Pro Val Val Thr Gly Ala Gly Leu Thr Phe
        355                 360                 365

Glu Glu Ala Arg Tyr Arg Ala Ala Arg Ala Ala Leu Ala Leu Ala Gly
    370                 375                 380

Thr Leu Ala Leu Asp Arg Arg Arg Leu Val Asp Gly Arg Val Trp Ala
385                 390                 395                 400

Tyr Asp Leu Ala Asp Gly Ala Ala Arg Leu Val Pro Ala Asp Glu Val
                405                 410                 415

Phe Ala Ser Thr Ala Gly Gly Ser Thr Ala Gly Val Pro Leu Gly Ala
            420                 425                 430

Ala Ala Ala Tyr Ser Trp Ala Glu Ala Val Ala Glu Gly Leu Val Ala
        435                 440                 445

Gln Ala Ala Ala Leu Thr Leu Ala Gly Ala Gly His Ala Gly Glu Pro
    450                 455                 460

Tyr Gly Arg Ala Asp Leu Ala Gly Ala Pro Ala Tyr Leu Leu Ala Met
465                 470                 475                 480

Val Arg Ala Leu Gly Glu Glu Leu Thr Val His Asp Val Thr Gly Pro
                485                 490                 495

Leu Gly Val Pro Val Val Val Gly Thr Leu Ser Gly Gly Ala Thr Ala
            500                 505                 510

Cys Gly Ala Gly Met Thr Thr Asp Ala Ala Val Thr Ala Cys Leu Arg
        515                 520                 525

Asp Leu Leu Leu Leu Arg Gln Ala Glu Ile Asn Asp Gln Pro Val Tyr
    530                 535                 540

Ala Pro Pro Ser Gly Arg Pro Leu Pro Gly His Leu Arg Gly Asn His
545                 550                 555                 560

Pro Ala Pro Ala Val Pro Gly Thr Asp Ala Asp Arg Leu Ala Ala Arg
                565                 570                 575

Leu Ala Glu Arg Gly His Arg Pro Leu Ala Val Pro Leu Asp His Asp
            580                 585                 590

Gln Ala Val His Ala Val Leu Pro Phe Ala Val Arg Val Val Cys Arg
        595                 600                 605

<210>  28
<211>  578
<212>  PRT
<213>  nonomuraea sp.

<400>  28

Met Ile Arg Leu Pro Asp His Gly Glu Leu Ala Val Pro Ile Gly Asp
1               5                   10                  15

Gly Pro Met Leu Pro Leu Arg Thr Glu Leu Gly Arg Val Val Ile Gly
            20                  25                  30

Pro Leu Glu Arg Pro Gly Val Pro Gly Cys Arg Ala Cys Val Arg Ile
        35                  40                  45

Arg Ala Ala Arg Val Gln Pro Asp Ala Arg Lys Ala Glu Ala Val Arg
    50                  55                  60

Ala Arg His Ala Pro Ala Pro Ser Gln Trp Leu Thr Pro Leu Ala Thr
65                  70                  75                  80

Asp Leu Val Arg Val Leu Val Ala Ser Glu Ser Ala Ala Leu Ala Ala
                85                  90                  95

Asp Thr Asp Pro Arg Thr Ala Gly Ala Val Leu Glu Ile Asp Leu Ala
            100                 105                 110

Thr Leu Glu Ile Thr Arg His Arg Phe Leu Pro Asp Pro Leu Cys Pro
        115                 120                 125

His Cys Gly Gly Leu Pro Pro Asp Gly Pro Ala Glu Leu Thr Leu Arg
    130                 135                 140

Pro Arg Arg Lys Leu Gly Gly Ser Pro Arg Thr Arg Pro Val Glu Leu
145                 150                 155                 160

Asn Ala Leu Leu Glu Thr Tyr Val Asp Gly Arg Val Gly Met Ile Arg
                165                 170                 175

Pro Leu Lys Ser Gly Val Gln Gly Gly Leu Thr Val Ala Ser Ala Met
            180                 185                 190

Leu Pro Ile Arg Ala Gly His Gly Leu Glu Pro Gly Val Gly Arg Thr
        195                 200                 205

Arg Ser Tyr Thr Ala Ser Arg Leu Val Ala Val Leu Glu Ala Leu Glu
    210                 215                 220

Arg Tyr Gly Gly Val Ser Pro Gly Gly Arg Arg Thr Ser Val Thr Ala
225                 230                 235                 240

Ala Tyr Arg Asp Ile Ser Gly His Ala Val His Pro Asp Thr Phe Gly
                245                 250                 255

Thr His Pro Glu Glu Asn Tyr Asp Arg Pro Gly Phe Ala Phe Arg Arg
            260                 265                 270

Phe Thr Glu Asp Thr Val Cys Arg Trp Val Trp Gly Tyr Ser Phe Ala
        275                 280                 285

Lys Ala Gln Pro Val Leu Val Pro Glu Asn Gln Val Tyr Tyr Tyr Ala
    290                 295                 300

Arg Asn Leu Pro Asp Gly Glu Gln Pro Phe Val Phe Glu Val Ser Asn
305                 310                 315                 320

Gly Cys Ala Leu Gly Ser Cys Leu Glu Glu Ala Ile Leu His Gly Leu
                325                 330                 335

Leu Glu Val Val Glu Arg Asp Ala Phe Leu Leu Thr Trp His Ala Arg
            340                 345                 350

Arg Glu Val Pro Val Leu Asn Pro Ala Leu Ala Ser Asp Pro Val Leu
        355                 360                 365

Pro Met Gln Ala Ala Ala Ile Thr Ala Glu Thr Gly His Arg Val Leu
    370                 375                 380

Cys Phe Asp Thr Thr Ala Glu His Gly Ile Pro Ser Val Trp Ala Met
385                 390                 395                 400

Ala Val Asp Val Glu His Arg Ser Asp Arg Pro Ala Thr Ala His Ala
                405                 410                 415

Ala Gly Ala Ala Leu Thr Leu Glu Gln Ala Ala Met Asn Ala Leu Ser
            420                 425                 430

Glu Leu Gly Pro Leu Leu Ala Asp Val Ile Arg Arg Tyr Pro Glu Glu
        435                 440                 445

Arg Glu Arg Ala Glu Ala Met Val Arg Asp Pro Glu Gln Val Ala Thr
    450                 455                 460

Met His Asp His Ser Leu Leu Tyr Ala Val Pro Ala Ala Ala Glu Arg
465                 470                 475                 480

Leu Ser Phe Leu Thr Gly Arg Ala Asp Gly Pro Arg Thr Gly Phe Gly
                485                 490                 495

Ser Ala Arg Phe Thr Gly Asp Asp Leu Thr Ala Asp Leu Arg Ala Met
            500                 505                 510

Ile Asp Ala Val Ile Asp Ala Gly Met Asp Val Val Val Val Asp Gln

515                 520                 525

Thr Thr Pro Glu His Leu Ala Gly Gly Phe Arg Cys Val Lys Val Leu
    530                 535                 540

Val Pro Gly Ala Leu Pro Met Thr Phe Gly His Gln Tyr Arg Arg Leu
545                 550                 555                 560

Asp Asn Leu Pro Arg Leu Ala Ala Ala Arg Thr Thr Asp Pro His Pro
                565                 570                 575

Phe Pro

<210>  29
<211>  857
<212>  PRT
<213>  nonomuraea sp.

<400>  29

Met Ala Val Ile Glu Arg Glu Glu Ser Asp Gly Pro Pro Val Ala Val
1               5                   10                  15

Arg Thr Cys Gly Val Ala Ala Ala Glu Leu Glu Ser Leu Arg Trp Asp
            20                  25                  30

Glu Ser Tyr Ala Leu Val Thr Arg Leu Leu Ala Glu Thr Ala Arg Val
        35                  40                  45

Ala Ala Asp Gly Arg Arg Leu Ala Val Ala Leu Gly Asp Val Ile Gly
    50                  55                  60

Gly Leu Gly Pro Ser Gly Ser Arg Pro Val Leu Val Gly Leu Arg Arg
65                  70                  75                  80

Ala Leu His Thr Gly Arg Leu Pro Ser Gly Arg Glu Trp Gly Pro Glu
                85                  90                  95

Ala Gly Ala Ala Leu Pro Tyr Gly Leu Arg Gly Glu Val Glu Glu Trp
            100                 105                 110

Val Arg Arg Ala Arg Glu Cys Ala Gly Leu Arg Ala Arg Leu Pro Glu
        115                 120                 125

Val Val Ala Arg Glu Ser Leu Glu Lys Glu Glu Arg Leu Arg Ala Ala
    130                 135                 140

Ala Ala Asp Pro Ala Phe Arg Arg Gly Leu Ala Leu Ala Gly Gly Glu
145                 150                 155                 160

Leu Ala Val Asp Leu Glu Thr Trp Leu Ala Asp Pro Ala Arg Arg Pro
                165                 170                 175

Lys Pro Gln Lys Leu Leu Arg Leu Ala Lys Tyr Leu Val Arg Ala Ala
            180                 185                 190

Val Lys Thr Ser Pro Tyr Ser Thr Phe Thr Ser Thr Gly Arg Ala Val
        195                 200                 205

Trp Gly Gly Gly Glu Arg His Val Glu Arg Val Val Pro Val Leu Glu
    210                 215                 220

Leu Asp Gly Val Gln Thr Phe Ser Asp Asp Arg Val Arg Val Asn Pro
225                 230                 235                 240

Ser Val Thr Val Ala Gly Gly Thr Ala Glu Phe Ile Gly Pro Pro Pro
                245                 250                 255

Gly Glu Ala Leu Val Ser Ile Gly Val Thr Glu Ala Val Ala Ala Cys
            260                 265                 270

Leu Arg Val Ala Glu Gly Gly Glu Trp Val Pro Arg Gly Arg Phe Ala
        275                 280                 285

Glu Ala Leu Gly Ala Glu Pro Ala Ala Val Thr Lys Phe Leu Asp Lys
    290                 295                 300

Leu Leu Ala Val Gly Leu Leu Glu Ala Arg Pro Asp Gly Asp Pro Pro
305                 310                 315                 320

Pro His Leu Leu Asp Gly Ile Arg Arg Thr Glu Arg Ala Thr Asp Pro
                325                 330                 335

Ser Thr Phe Arg His Glu Leu Gly Arg Leu Ile Ala Ala Ala Arg Ala
            340                 345                 350

Ala Ala Arg Ala Thr Ala Pro Gly Ala Asp Thr Ala Lys Asn Ala Ala
        355                 360                 365

Pro Ala Gly Arg Ala Thr Arg Gly Asp Gly Gly Ala Asp Ala Glu Arg
    370                 375                 380

Ala Thr Arg Gly Asp Ser Asp Ala Asp Asp Gly Arg Ala Thr Arg Gly
385                 390                 395                 400

Asp Gly Gly Ala Asp Asp Arg Gly Ala Ala Arg Gly Asp Gly Gly Val
                405                 410                 415

Gly Arg Ala Val Val His Glu Val Ala Val Cys Val Glu Pro Val Ala
            420                 425                 430

Arg Leu Asp Pro Ala Arg Trp Arg Gly Gly Leu Ala Asp Leu Asp Val
        435                 440                 445

Val Arg Arg Trp Leu Ala Val Phe Asp Ala Lys His Pro Met Arg Leu
    450                 455                 460

Ala Val Ala Ala Tyr Leu Thr Thr Arg Tyr Gly Pro Asp Pro Ala Val
465                 470                 475                 480

Pro Phe Leu Thr Leu His Arg His Ile Gln Arg Glu Leu Ala Gly Thr
                485                 490                 495

Gly Ala Ala Gly Arg Glu Leu Arg Ala Phe Leu Gly Ser Ser Ala Ala
            500                 505                 510

Trp Thr Gln Pro Leu Gly Thr Ser Ala Leu Pro Arg Ile Arg Glu Leu
        515                 520                 525

Glu Arg Leu Arg Ala Glu Ala Arg Ser Leu Ala Leu Asp Ala Glu Asp
    530                 535                 540

Pro Asp Gly Thr Cys Arg Val Thr Pro Gly Gln Leu Ala Ala Gln Leu
545                 550                 555                 560

Glu Ser Trp Pro Ser Trp Ile Ala Ile Pro Ala Ser Ser Ala Cys Tyr
                565                 570                 575

Val Gln Ala Val Ala Gly Thr Leu Val Leu Asn Val Val His Gly Gly
            580                 585                 590

His Gly Arg Ala Leu Arg Arg Leu Asp His Leu Leu Glu Arg Ala Gly
        595                 600                 605

Gly His Pro Glu Pro Pro Arg Ile Glu Asp Pro Asp Gly Ala Val Tyr
    610                 615                 620

Ala Glu Phe Ser Gly Asp Leu Gly Ser Thr Leu Asn Ala Arg Pro Pro
625                 630                 635                 640

Ser Thr Arg Tyr Glu Ile Asp Tyr Pro His Ser Pro Gly Thr Arg Pro
                645                 650                 655

Pro Asp Leu Arg Leu Pro Leu Thr Asp Leu His Val Thr Leu Cys Pro
            660                 665                 670

Asp Ser Gly Leu Pro Glu Leu Arg Ser Lys Arg Leu Gly Arg Arg Val
        675                 680                 685

Val Pro Leu His Leu Gly Leu Ala Ala Glu Phe Arg Leu Pro Pro Ala
    690                 695                 700

Ala Arg Phe Ile Glu Arg Val Phe Gly Pro Gly Tyr Leu Leu His Pro
705                 710                 715                 720

Ser Ser Pro Pro Leu Val Arg Met Gly Arg Val Pro Ser Glu Val Thr

725                    730                    735

Arg Tyr Pro Arg Val Glu Ala Gly Arg Val Val Val Gln Arg Arg Arg
            740                 745                 750

Trp Leu Ala Pro Ala Ala Thr Leu Pro Val Arg Ala Lys Gly Glu Gly
        755                 760                 765

Asp Ala Ala Tyr Leu Thr Arg Leu Ile Ala Trp Ala Asp Glu Tyr Gly
    770                 775                 780

Val Pro His Arg Ser Phe Val Arg Ala Trp Pro Glu Gln Thr Gly Asp
785                 790                 795                 800

Lys Gly Gln Asp Lys Ala Arg Lys Pro Leu Phe Leu Asp Leu Ala Asn
                805                 810                 815

Leu Phe Leu Val Lys Asn Phe Glu Arg Gln Ile Arg Gly Cys Ala Phe
            820                 825                 830

Ala Leu Phe Glu Glu Ala Leu Pro Asp Pro Gly Pro Glu Arg Val Thr
        835                 840                 845

Glu Tyr Leu Ile Glu Val Gly Gly Arg
    850                 855

<210>  30
<211>  271
<212>  PRT
<213>  nonomuraea sp.

<400>  30

Met Ser Trp Val Ser Val His Val Phe Ser Gln Gly Pro Leu Asp Asp
1               5                   10                  15

Met Ile Thr Gly Leu Ala Gly Pro Leu Leu Gly Asp Asp Gly Phe Phe
            20                  25                  30

Leu Arg Tyr Trp Glu Gly Gly Pro His Leu Arg Ile Arg Val Arg Ser
        35                  40                  45

His Pro Ala Glu Gln Val Val Glu Arg Ala Glu Ala Tyr Leu Arg Glu
    50                  55                  60

His Pro Ser Glu Ala Val Ile Asp Pro Glu Glu Tyr Ala Arg Leu Ala
65                  70                  75                  80

Ala Thr Leu Ala Ala Arg Glu Gly Val Pro Gly Tyr Thr Arg Arg Leu
                85                  90                  95

Tyr Pro Asn Asp Ser Ala His Leu Ile Pro Tyr Gln Pro Glu His Asp
            100                 105                 110

Arg Tyr Gly Thr Gly Arg Ser Leu Asp Ala Val Glu Arg His Phe His
        115                 120                 125

Asp Ser Ser Val Ile Ala Leu Gln Leu Ile Gly Ala Gly Leu Gly His
    130                 135                 140

Glu Arg Arg Ser Met Ala Trp Leu Thr Met Leu Leu Val Ala Trp Gln
145                 150                 155                 160

Val Ala Gly Glu Gly Arg Ala Phe Gly Pro Pro Ala Asp Ala Gly Val
                165                 170                 175

Gln Val Pro His Glu Arg Met Ala Ala Leu Val Glu His Ala Arg Ala
            180                 185                 190

Val Thr Gln Glu Pro Ala Pro Ser Glu Pro Arg Gly Val Val Ala Ala
        195                 200                 205

Trp Tyr Ala Ser Ile Ala Arg Leu Val Glu Glu Leu Ala Ala Ala Gly
    210                 215                 220

Phe Asp Ala Ala Arg Thr Gly Ala Thr Val Asp Leu Ala Ala His Leu
225                 230                 235                 240

Leu Ala Asn Arg Leu Gly Ile Arg Val Gln Glu Glu Ala Arg Leu Arg
                245                 250                 255

His Leu Ala Ser Gly Ala Thr Arg Glu Ser Glu Val Gly Val Gly
            260                 265                 270

<210>  31
<211>  319
<212>  PRT
<213>  nonomuraea sp.

<400>  31

Met Thr Trp Thr Arg Leu Arg Val Asp Tyr His Asp Gly Pro Met Asp
1               5                   10                  15

Asp Leu Ile Leu Asp Ala Leu Arg Pro Ala Trp His Glu Ile Arg Gly
            20                  25                  30

Tyr Phe Leu Arg His Trp Val Cys Gly Pro His Leu Arg Ile Phe Val
        35                  40                  45

Asp Gly Asp Ala Thr Pro Ile Val Arg Ala Val Glu Arg His Leu Ala
    50                  55                  60

Ala Cys Pro Ser Ala Gly Thr Thr Asp Pro Gln Ala Leu Leu Pro Leu
65                  70                  75                  80

His Glu Arg Leu Ala Glu Leu Glu Gly Glu Arg Gly Pro Leu Leu Pro

85                  90                  95

Trp Ala Pro Asp Asn Thr Val Thr Ala Glu Pro Pro Gly Leu Asp Thr
            100                 105                 110

Glu Leu Asp Arg Phe Leu Ala Asp Phe Tyr Ala Asp Thr Thr Glu Ala
        115                 120                 125

Ala Phe Asp Ala Leu Gly Arg Val Arg Ala Gly Thr Pro Leu Pro Gly
    130                 135                 140

Ile Ala Phe Asp Leu Val Val Ala Thr Ala His Asp Leu Ser Glu Gly
145                 150                 155                 160

Gly Leu Pro Thr Ala Arg Thr Ser Leu Arg Ser His Ala Glu Ala Tyr
                165                 170                 175

Leu Ser Arg Leu Pro Gly Gly Val Arg Ala Lys Trp Gln Ala His Tyr
            180                 185                 190

Glu Arg Asn Gln Glu Pro Leu Thr Ala Arg Ile Lys Ala Leu Thr Gly
        195                 200                 205

Ala Gly Glu Pro Gly Ala Trp Leu Arg Thr Ile Arg Ala Thr Arg Asp
    210                 215                 220

Arg Gly Arg Thr Leu Ile Asp Glu Gly Arg Leu Ser Leu Gly Tyr Ala
225                 230                 235                 240

Thr Asp Gly Pro Ser Thr Arg Pro Pro Leu Ala Ala Val Ser Pro Phe
                245                 250                 255

His Arg Asn Leu Glu Thr Asp Glu Arg Trp Leu Ala Leu Lys Asp Thr
            260                 265                 270

Pro Ala Phe Ala Ala Tyr Arg Leu Ala Leu Asn Cys Ala Tyr Leu His
        275                 280                 285

Leu Thr Arg Leu Gly Leu Thr Pro Asp Gln Arg Phe Leu Ile Cys His
    290                 295                 300

Leu Ala Ala Ser Ala Ala Asp Asp Val Tyr Arg Ala Val Ala Ser
305                 310                 315

<210>  32
<211>  428
<212>  PRT
<213>  nonomuraea sp.

<400>  32

Met Thr Gly Glu Arg Tyr Glu Leu Arg Ala Gly Val His Leu Ala Arg
1               5                   10                  15

Leu Asp Ala Gly Val Arg Leu Phe Ala Trp Pro His Ala Glu Thr Leu
            20                  25                  30

Gly Glu Leu Thr Arg Asp Glu Leu Asp Leu Leu Arg Asp Leu Ala Ala
        35                  40                  45

Gly Pro Arg Thr Ala Gly Gly Pro Leu Val Glu Arg Leu Arg Ala Gly
    50                  55                  60

Gly Trp Leu Lys Ile Thr Ile Thr Arg Asp Gly Val Pro Leu Tyr Thr
65                  70                  75                  80

Leu Ala Pro Leu Arg Pro Pro Pro Pro Arg Pro Ala Val Pro Glu Arg
                85                  90                  95

Pro Val Leu Ser Arg Phe Ala Thr Leu Arg Arg Glu Gly Asp Ala Leu
            100                 105                 110

Val Ala Gly Ser Pro Leu Ala Trp Cys Asp Leu Tyr Val His Asp Pro
        115                 120                 125

Gly Leu Val Gly Asp Val Val Arg Pro Gly Gly Glu Val Leu Ala Asp
    130                 135                 140

Leu Val Trp Ala Gly Leu Ala Val Pro Glu Glu Ala Glu Asp Arg Glu
145                 150                 155                 160

Leu Arg Phe Arg Gln Trp Ala Pro His Glu Leu Glu Phe His Asp Arg
                165                 170                 175

Ser Arg Val Gly His Arg Gly Tyr Leu Gly Asp Gly Phe Gly Gly Thr
            180                 185                 190

Phe Trp Ala Arg Gly Arg Phe Asp Pro Leu Pro Ala Arg Pro Arg Pro
        195                 200                 205

Tyr Pro Gly Arg Ala Ile Glu Leu Pro Thr Pro Asp Leu Asp Glu Leu
    210                 215                 220

Arg Leu Lys Asp Pro Ser Leu Thr Asp Val Leu Glu Gly Arg Arg Ser
225                 230                 235                 240

Val Arg Glu His Asp Asp Glu Arg Pro Ile Thr Leu Asp Leu Leu Gly
                245                 250                 255

Glu Leu Leu Tyr Arg Thr Ala Arg Val Arg Glu Cys Trp Thr Ala Asp
            260                 265                 270

Gly Val Glu Tyr Thr Arg Lys Pro Tyr Pro Ser Gly Gly Ser Val Tyr
        275                 280                 285

Glu Leu Glu Ile Tyr Pro Val Val Arg Asn Val Ala Gly Leu Glu Pro
    290                 295                 300

Gly Met Tyr His Tyr Asp Gly His Asp His Val Leu Arg Pro Val Arg
305                 310                 315                 320

Glu Ala Gly His Pro Ala Val Arg Arg Leu Leu Thr Val Ala Gly His
                325                 330                 335

Gly Ala Ala Thr Gly Arg Arg Pro Gln Val Leu Leu Ala Val Ser Ser
            340                 345                 350

Arg Ala Gly Arg Leu Leu Trp Lys Tyr Glu Gly Met Gly Tyr Ala Leu
        355                 360                 365

Thr Leu Lys His Val Gly Val Leu Tyr Gln Thr Leu Tyr Cys Val Ala
    370                 375                 380

Thr Ala Met Gly Leu Ala Ala Cys Gly Leu Gly Ser Gly Asp Ser Ala
385                 390                 395                 400

Ala Phe Ala Glu Ala Thr Gly Arg Asp Pro Leu Glu Glu Cys Ala Val
                405                 410                 415

Gly Glu Phe Met Ile Gly Thr Ala Glu Ala Pro Arg
            420                 425

<210>  33
<211>  630
<212>  PRT
<213>  nonomuraea sp.

<400>  33
   Leu Asn Asp Phe Lys Pro Arg Leu Pro Ala Asp Ile Arg Phe Ala Pro
   1               5                   10                  15
   Thr Ser Gly Gly Val Ala Phe Leu Thr Arg Glu Gly Val Val Arg Met
               20                  25                  30
   Asn Gly Ala Ser Val His Leu Trp Val Glu Arg Leu Ala Val His Leu
           35                  40                  45
   Asp Gly Ser Arg Thr Val Ala Glu Leu Thr Ala Glu Leu Pro Asp Glu
       50                  55                  60
   Arg Arg Ala Phe Val Thr Ser Leu Leu Thr Thr Leu Arg Glu Arg Gly
   65                  70                  75                  80
   Leu Val Glu Asp Ala Gly Glu Arg Gly Val Leu Pro Val Ala Ala Gly
                   85                  90                  95
   Thr Ala Cys Ser Leu Leu Ala Asp Ala Asp Arg Phe Gly Glu Leu Ala
               100                 105                 110
   Pro Ala Leu Ala Asp Ala Leu Arg Arg Leu Gly Leu Glu Val Leu Thr
           115                 120                 125
   Glu Pro Ser Gly Gly Ala Leu Val His Val Ala Ala Ala Asp Ala Asp
       130                 135                 140
   Gly Leu Ala Ala Ala Gly Arg Leu Asp Arg Leu Ser Ala Glu Arg Gly
   145                 150                 155                 160
   Leu Pro Ile Ala His Val Leu Leu Arg Glu Gly Glu Ala Trp Trp Ser
                   165                 170                 175
   Glu Thr Gly Val Val Gly Arg Asp Val Pro Pro Trp Ala Ala Gly Trp
               180                 185                 190
   Arg Arg Leu Ala Ala Met Glu Ala Val Pro Arg Thr Ala Gly His Val
           195                 200                 205
   Ser Val Ser Pro Val Ala Ala Ala Val Val Ala Gly Gln Val Val Ala
       210                 215                 220
   Asp Leu Gly Arg Cys Leu Ser Gly Thr Pro Arg Val Ser Gly Gly Pro
   225                 230                 235                 240
   Arg Leu His Val Val Asp Leu Asp Gly Phe Gly Met Arg Thr His Ser
                   245                 250                 255
   Phe Leu Pro His Leu Ser Ala Leu Pro Pro Gly Thr Gly Val Ser Ala
               260                 265                 270
   Gly Pro Leu Gly Glu Glu Glu Phe Ser Arg Arg Ala Ala Arg Leu Met
           275                 280                 285
   Asp Ser Arg Thr Gly Val Phe Ala Glu Ile Glu Glu Gly Glu Tyr Gly
       290                 295                 300
   Gln Leu Pro Leu His Val Ser Val Thr Thr Val Ala Asp Pro Met Gly
   305                 310                 315                 320
   Ala Leu Gly Gly Ala Leu Ala Arg Val Ile Gly Val Gly Ala Asp Phe
                   325                 330                 335
   Ala Thr Ala Arg Tyr Arg Ala Ala Leu Lys Ala Leu Thr Val His Gly
               340                 345                 350
   Leu Leu Ala Leu Asp Pro Arg Arg Leu Val Thr Ala Glu Gly Thr Pro
           355                 360                 365
   Leu Thr Gly Ala Gly Glu Pro Ala Ser Gly Phe Pro Ala Asp Pro Gly
       370                 375                 380
   Ala Arg Glu Arg Pro Ile Gly Gly Phe Ser Ala Asp Pro Gly Ala Gly
   385                 390                 395                 400
   Glu Arg Pro Asp Asp Gly Leu Leu Asp Asp Pro Leu Val Gly Asp Val
                   405                 410                 415
   Ala Ala Gly Arg Val Glu Ala Phe Val Arg Gly Arg Asp Leu Val Glu
               420                 425                 430
   Gly Gly Glu Val Leu Leu Pro Ala Arg Ala Val Phe Leu Asp Pro His
           435                 440                 445
   Ala Thr Gly Ala Ala Ala Gly Tyr Ser Ala Glu Glu Ala Leu Val Ala
       450                 455                 460
   Gly Leu Leu Asp His Cys Ala Ala Leu Ala Gly Leu Asp Gly Thr Leu
   465                 470                 475                 480
   Pro Pro Val Asp Val Gly Gly Ala Arg Ala Ala Asp Asp Glu Thr Ala
                   485                 490                 495
   Arg Ser Ala Ala Leu Leu Asp Ala Ile Gly Leu Pro Tyr Gln Val Leu
               500                 505                 510
   Asp Ala Thr Gly Pro Ser Gly Val Pro Val His Ala Gly Thr Leu Gly
           515                 520                 525
   Gly Arg Leu Val Ala Arg Ala Ala Gly Val Thr Pro Leu Ala Ala Leu
       530                 535                 540
   Arg Ala Thr Leu Glu Gly Ile Leu Ala Ala Tyr Gln Gly Ile Pro Gly
   545                 550                 555                 560
   Pro Ala Pro Leu Pro Asp Leu Cys Pro Ala Val Ala Gln Glu Pro Pro
                   565                 570                 575
   Val Val Leu Asp Thr Gly Ala Ala Val Leu Gly Ala Gly Glu Leu Ala
               580                 585                 590
   Ala Ala Leu Ala Gly Ala Gly Leu Arg Val Ser Ala Val Pro Leu Asp
           595                 600                 605
   His Asp Arg Glu Val His Ala Leu Met Pro His Val Val Arg Val Val
       610                 615                 620
   Thr Glu Pro Leu His Gly
   625                 630

<210>  34
<211>  610
<212>  PRT
<213>  nonomuraea sp.

<400> 34

Val Gly Thr Gly Leu Leu Ala Glu Glu Ile Thr Ile Ala Leu Lys Asp
1               5                   10                  15

Arg Asp Gly Arg Ala Glu Val Thr Ile Thr Ala Cys Asp Gly Trp Ala
            20                  25                  30

Arg Gly Pro Arg Arg Asp Gly Ala Ala Trp Leu Pro Val Arg Val Glu
        35                  40                  45

Tyr Gly Val Ala Val Ile Gly Pro Leu Glu Arg Pro Gly Glu Pro Gly
    50                  55                  60

Cys Val Thr Cys Phe Glu Thr Arg Arg Asp Arg Val Arg Thr Asp Ser
65                  70                  75                  80

Ala Asp Arg Ala Ala Val Leu Ala Val His Gly Pro Glu Leu Ala Gly
                85                  90                  95

Arg Ala Ser Pro Tyr Leu Gly Arg Leu Ala Ala Arg Thr Ile Ala Ala
            100                 105                 110

Leu Ala Ala His Ala Ala Glu Gly Pro Gly Cys Ala Val Trp Tyr Val
        115                 120                 125

Asp Leu Arg Thr Leu Thr Ala Glu Arg His Thr Phe Leu Pro Glu Pro
    130                 135                 140

His Cys Pro Asp Cys Gly Asp Leu Pro Asp Asp Asp Arg Ala Ser Ala
145                 150                 155                 160

Ala Phe Glu Pro Gly Ala Ala Pro Arg Pro Pro Gly Tyr Arg Val Arg
                165                 170                 175

Asn Val Pro Ala Glu His Gly Ala Leu Leu Asp Thr Tyr Val Asp Gly
            180                 185                 190

Glu Cys Gly Leu Ile Arg Pro Leu Val Arg Asp Thr Leu Gly Gly Leu
        195                 200                 205

Val Ile Ala Gly Ala Met Leu Pro Leu Arg Phe Glu Gly Gly Ser Glu
    210                 215                 220

Pro Gly Val Gly Arg Thr Arg Gly Tyr Arg Ala Ser Glu Val Thr Ala
225                 230                 235                 240

Val Leu Glu Ala Leu Glu Arg Trp Gly Gly Val Glu Pro Gly Gly Lys
                245                 250                 255

Arg Thr Arg Val Arg Ala Ser Tyr Ala Glu Val Ala Ala Asp Ala Leu
            260                 265                 270

Asp Pro Arg Thr Leu Gly Glu His Asp Thr Ser Ser Tyr Glu Met Glu
        275                 280                 285

Gly Phe Ala Phe Arg Pro Phe Ala Glu Asp Ala Val Cys Asp Trp Val
    290                 295                 300

Trp Gly Tyr Ser Phe Ala Arg Arg Ser Pro Ile Leu Val Pro Glu Ser
305                 310                 315                 320

Leu Ala Tyr Tyr Tyr Val Arg Gly Arg Glu Arg Pro Phe Leu Phe Glu
                325                 330                 335

Ile Ser Asn Gly Cys Ala Leu Gly Gly Ser Met Ala Glu Ala Leu Leu
            340                 345                 350

Tyr Gly Leu Leu Glu Thr Val Glu Arg Asp Ala Phe Leu Met Thr Trp
        355                 360                 365

Tyr Gly Arg Leu Pro Val Pro Arg Ile Asp Pro Gly Thr Ala Arg Asp
    370                 375                 380

Arg Thr Ile Pro Leu Gln Ala Ala Ala Ile Ile Ala Glu Thr Gly Tyr
385                 390                 395                 400

Arg Val Glu Leu Tyr Asp Thr Thr Met Glu His Gly Ile Pro Ser Val
                405                 410                 415

Trp Ala Met Gly Val Arg Gln Asp Gly Asp Pro Gly Arg Pro Arg Leu
            420                 425                 430

Val Cys Ala Ala Gly Ala His Leu Val Pro Glu Lys Ala Val Leu Ser
        435                 440                 445

Ala Leu Ser Glu Leu Gly Pro Leu Leu Ala Asp Leu Ile Arg Arg Tyr
    450                 455                 460

Pro Asp Glu Val His Arg Ala Gln Glu Met Val Thr Asn Pro Asp Leu
465                 470                 475                 480

Val Ala Thr Met His Asp His Ser Thr Leu Tyr Gly Ala Asp Ala Ala
                485                 490                 495

Phe Asp Arg Leu Ser Phe Leu Thr Gly Gly Thr Gly Val Arg Gly Leu
            500                 505                 510

Pro Asp Met Glu Ala Phe Thr Val Pro Gly Asp Leu Asp Val Met Arg
        515                 520                 525

Val Val Asp Arg Phe Leu Ala Glu Gly Met Asp Val Val Val Ile Asp
    530                 535                 540

Gln Thr Thr Pro Glu His Arg Ala Arg Gly Phe Ser Cys Val Lys Val
545                 550                 555                 560

Leu Val Pro Gly Thr Leu Pro Met Thr Phe Gly His Arg Asn Arg Arg
                565                 570                 575

Val Arg Gly Leu Thr Arg Pro Leu Glu Leu Pro Tyr Arg Leu Gly His
            580                 585                 590

Arg Asp Thr Pro Leu Thr Pro Ala Asp Leu Asn Pro Asp Pro His Pro
        595                 600                 605

Phe Pro
    610

<210>  35
<211>  2484
<212>  DNA
<213>  nonomuraea sp.

<400>  35
gtgccgccgg cccaggccgg cggcactccg cccggggtga ggagacagat catggaggtg      60

ggccgcgaaa tcgtggtccg cgtggcgggg ctgcccgccg ccgtgctcgc cgacctgcgc     120

ctgccgcaca ccgccgagct cgtcacgcac ctgtccgctg agcggcggcg gctcgccgcc     180

gaggcggctg cgctgtccgg cgagctgttc gacctgatcg gcgccgccgg ctccgcccgc     240

gccgccctgg tgggcctgcg gcgcgcgctc gcccccgggc accgcccccc gtcgccgcga     300

ctgatcgggc tgtgtcccct gcccgccccg ctggccgagc gggtcaccgc ctgggtgcgg     360

gcccggtacg actgggagga ccggcgccgc gaccttgccg gcacgctgga caaggagcgc     420

gccgacgcgc tcgaccgcgt acgcgccgcc tgcgtcgccc cggcgttccg gcggggtctg     480

gcgctgtccg gtggggagct gaccagcacg ctggagcgct ggctcgccga ccccggccgt     540

gcgccccggc aggggaaggt gctccgcctg gtcaagtacc tgacccgagc tgcggccaag     600

accagcccgt acggctcctt catggtcagc gcgctggcgg ggcggcccgg cgacgaccct     660

cccatcccgg agctgctcac cgtggccgag ccgccggggg cgttcctcga cgccgtcggc     720

gacgcgctgc tcgccgaccc ggcactggcc ggccaggtgc cgctgcgccc caaccccagc     780

ctgacgtgga ccgcccaagg actgcttttc gtccggaccg tccgcgcggc ggcgggagag     840

caggccgggc cgaaggaaga gatcgccacc gccgggcgcg ccgccgcgct ggagctgtgc     900

ctgcggcacg ccgagtcccg gcccaccgcg ccgcagctgg cggagctgct ggccgaggcg     960

ggggcggatc cgggcgaggc ggccgcgttc gtcgaccggc tggtggcggc gcagctcctg    1020

ctgccgtgtc cgccggtgca cgacgacgat cccgacccgt tcggtgcctg ggcccgccag    1080

gtgtgcgccc cggagctgcg cgagctggcg gccgcgtcgc ggcccgtggc cgccgccgtg    1140

gactcgcccg gccagcgccg cgccaggatc gccgaagccg ccgccgccgt ggcggaccgg    1200

ctcggcatcg acccgcccgc cgatccggcg cacgagcacg acgtcagcac cggccgtccg    1260

gcgccgccac cgctgcccgc gggcgtgctc gccgacctgg acgcggtccg gcgctggctc 1320
gccgtgttcg actggaaggt gcccgtccgg gtgggggtcg gcgcgttctg ccgcgaacgt 1380
ttcggctcag ggtcccgcac gcccttcctg gaggcgtgcc gggcggcgac ggcggcgctg 1440
ccgcacctgt tcggccccgc cgcgatgccg tggttcctcg acctgaccgg tgacggtcgc 1500
ctgcgtgagc tggaccgtct ccggacgcgc gcccgcgatc tcgcccgctc cggcgtcctc 1560
gaccggcggc gggtgctggc tgacacggcc gactggcccg cctggctgac ctggcccgcc 1620
tccactggtt tctacctgca gaccctgccc ggtggggtgg tgctgaacgc ggtgcatgcc 1680
gggcacggcc gcgcggcggg tcgcgtccac cacctcctcg cccgggcggg cgccgcgccg 1740
ccgcgcccgc cccgctccgg cctgccgcgt gccgagttcg gcggcaggtt cggctccgcg 1800
ctcaacaccc gcacgccgag cacgcggtac gagatcgacc accccggcgc gacgtccggc 1860
cgcgaccccc ggcaccgtgt cccgctcggc acgctcatgg tggtgcacga cccggacacc 1920
gacctggtcc acctgcacag cgaccggctc ggccgcgtcg aaccggtgca cctcggcatg 1980
atgggcgagc tggggctgcc cgccgtggcc ggtttcctcg aacgcgcctt cgcgcccacc 2040
tacctgttcc acccgagcgt gccgccgttc atctcgctgc gtgacctggc cggcgcctcc 2100
tcggcgcggc gcttccccag ggtgagtgtc ggcgacgtgg tcgtgcagcg cgcccgctgg 2160
acggtgcccg ccggcctggt gcccgcgcgt accggggcgg acggtgacca cctgctcgcg 2220
ctggccgagt ggcggcgcga gcacggcatc cccgagcggt gtttcgtgcg cggctggaag 2280
ccgggcgcag cgctgggcaa ggcgcgcaag ccgctctacg tcgacttcgc ctcctggcat 2340
ctggtcgccc tgttcgaacg ggaggcgcga accaacgccg ccctggtcat cgacgaggcc 2400
ctgccggacc cgctggccga cggcgccccc gcgcacgtca ccgagtacca cgtcgagatc 2460
ggcgaccggg agagagagca atga 2484

<210> 36
<211> 900
<212> DNA
<213> nonomuraea sp.

<400> 36
atgaccgaac ggcccggctg gttcgcggcg catgtgcact accgcggcga cctcgacgcg 60
ctgctgcgcg aagcggtcgc gccgctggtg cgcgcgcttg ccgccgactt cttcttcctg 120
cgctactggg acgggggcag ccacctgcgg ctgcggctgc gcggcgggga ccaggtcgcg 180
gtcgcccggc acctggacgc ctaccttgcc gcccaccccg cgccggagac cacctcccag 240
gaggagtacg cccgcgtcgc cccggtgctg gccgcccgcg agggcatgac gggccacctg 300
acgacgctgc ggccgaacaa caccgtggag ttcgcggcgt accggccgga gaccgcgaag 360
tacggcaccg gcgacgcgct acgcgccgtc gagcggcatt tcgtcgagtc cagccggtac 420
gcgcttgacg tccttgaccg ccgcccgacc ggcaaccaac gcgagctcgc agtgctcggc 480
gtcctgctgc tcgcctggta cgccgcccgc ccactcgatg agccgcctgg tgagccggtc 540
gatgacgagc tgatcggcgc ggtcgaggcg ctgtgccggg gctggcgcgg cggccgggac 600

ctgcccgagg agctcgtggc ggaggagtac ggccgggtgc gggagcgtgt ggccgggctg 660

gccggcgcgc tgcgtgacct cgttccggac ccggacgcgc ccggcagcag catgcgcgcg 720

tgggcggcga cgttcgaccg gctcgccgcc gcgctgcccc cgcccgaccg gctgcgggtg 780

ctcgacacgt gcgcgcatct ggcggccaac cggctgggcg tgtccatggc ggccgaggtg 840

cggctgcggc tgctcgccgc gcgggccctg cgcgaggtcg cgcccgtggg gaggcgatga 900

<210> 37
<211> 1002
<212> DNA
<213> nonomuraea sp.

<400> 37
atgagctggc gacgcgtcga cgtggcctac cacgatcccg accttgacgg gctgatcctg 60

gccacccggc cgctgctggc cgggacgccc ggccgcggct ggttccagcg gcactgggtc 120

cgcggtccgc acctggagct gtggttcgac gccgcgcagc cgtcgtggga gcggatccgc 180

gacgtgctgg agccctggct gcgcgtcaac ccgtcgcgcg cccggatcga ccgagaccgg 240

ctgctggcgc agcaccggca cctcgccgcc gccgaacgga tcgacgagcc gctgctgccg 300

ttctacgccg acaacacgct gcaccgggcc gctccgcgct cccgcgcaca cgtgctcggt 360

ggtccggcgg ccgaggagct gttccacgac ttccacacca ccgccagcgc cgtggcttac 420

gacgagctcg acgccgtgcg ggcgggagag tcgcggctgg tgatggcact ggacctgatg 480

gtcgccgccg cgcacgcgca cgccgagggc ggcgtccgcg gcgggttcgt gtcgttccgc 540

tcgcacgcgg aggcgttcct ggcgagcgcg cccggcctgc gcgagcgctg ggacgccgag 600

tacgccgccc gggcgggggc gctgcgagcg cggatcaccg cggtggtcgc cggaatcccg 660

cgcggccggg cgtgggccgg gctgctcgac cggttcgcgg accgcggcga cgagctgatc 720

gcctccggcg cgctgctggt cgagcccgcc gggccggacg cggtcgcccg gcccgacacc 780

gccttccacc gggcgctgcg cggcaaccgg acctggcacg aggaggtgct gcgctcagca 840

ccgttccgcc gctaccggct gctgctgaac ctgacctacc tgcagctttc ccggctcggg 900

gtgaacgccg tgcagcgggc gctgctgtgc cacttcgccg cgtcggcggt cgagcaggag 960

tacggggtgt ccgccatcga gatcgccatg ggaggcgcgt ga 1002

<210> 38
<211> 1368
<212> DNA
<213> nonomuraea sp.

<400> 38
gtgatgtcag tacaggtcga gcccgcagtg acgcaccggt gggcactgcg gcccggcgtc 60

taccgggcca ccgcgccagg cggcgatctc atgctcgccg cctggccgca cgccacgccg 120

ctcggccggg ccacccccga gctgctggcc ctgctggacg gcctggccgc cggatcgctg 180

ccgctcgacg agcccggcga cctcgcgccc gccgtcgagc tgctgcgcgc gggtggctgg 240

ctgcgcaaga cggtggcgta cgcgggacgc gacctctaca ccatcacccc cgtcgccgcc 300

cccaccgacg cgccgccacc gccgcgggac gaaccggcgc tgtcgcggtt cgcgctgctg 360

cgcaacaccg cggacgggct ggtgctggag atgcccggct cctggtgcga cgtccacgtg 420

cacgacccgg cggtggccgc gctgctcgcc gacccgtccg gcgacggcgg cctgcccgcc 480

gaggccgcgg ccgcgctgcg cgccgacctg gccgccgccg gcatgctgat ggagcgggag 540

gagccgttcg agcggcggca gtggagcacc cacgagctgt ggttccatga acgcagccgg 600

ctcggcaacc ggggctggtt cggcgcgcac ttcggcggca cgttctgggc ccgcggccgg 660

cacgacccgc cgcccgcgcg gccgtccccc taccccggcc cggccgtcga gctggcccgg 720

ccggacctcg cggcgctgcg gcgcaccgac cccacgctca ccgccgtgct ggaggaccgc 780

gaaagcgtcc gcgaccacga cgacgacgat ccgatcagcg ctgagcagct cggcgagctg 840

ctctaccggt gcgcgcgggt gcggctgctg cgtcacctgg aaggcttcga gtacgccggc 900

aagccgtacc ccgccggtgg ctcggcgtac gagctagagg tgtacccggt cgtgcggctg 960

gcccgcggcc tcgaccccgg catgtaccac tacgacgcgc acgaccatcg gctgcgccag 1020

gtccggcccg ccggccaccc ggcggtgcgc aggatgctga aggtcgccac ggagtcgtcc 1080

gtcgccaagg cgccgcccca ggtgctgctg gtgatcagcg cgagagtcgg ccggatcctg 1140

tggaagtacg aggcgatggg gtacgcgctc atgctgaagc acgtcggcgt gctgcaacag 1200

acgctgtacg cggtcgccac cgccatggac ctggccccgt gcgcgctcgg cagcggggac 1260

gatctcgcct tcaccgccgc caccggccgg gaccggctgg tggagtgcgc ggtcggcgag 1320

ttcatggtcg gcagcaggcg gaaggagccc gcggcatggg agctctga 1368

<210> 39
<211> 1827
<212> DNA
<213> nonomuraea sp.

<400> 39
atgggagctc tgaacctcct cctgcgcccc gacgcgcact acgccgaggt cgacggcggc 60

gtctacgtgc tgagccacca gggcgagacc ttcatctcgg gcacctcggt gcaccggtgg 120

ctcgaccgcc tcgcgccgct gctcgacggc acccgcacgc tcgaccagct caccgccggc 180

ctgcccgccg accgggccgg cttcgtggcc agacttgtcg gcacgctcac cgaacgcggc 240

ctggtgcgcg aggtcggccc ggcggtgtcg gacacgctca ccgacgccga acgcggcgag 300

caccgtgccc tgctggccta cctgggctac ttccacgact cgcccggccg cgtcctcgag 360

gacgtgcgcg acaccccgac cgcgatcgtg gggtccggtc cgctggccgc cgcggtggtc 420

cgcgcctgcg ccgccgccgg gctgcgccgg gtggagatcg gcgaggaggc gggcgcggcc 480

cgggtggtcg tccacgtggc cgagcgcgcc gagcccgaac gggcggcgcg gctggaacgg 540

cggtgcgccg ccgagggggt gctgctcgca caggtcatgc cggggccgga cggcatctgg 600

tggcagcccg cggcccgcgg cggctgctgg gcgagcgcgt ggcgccgcca ccgggcgctc 660

gcgggcgtgg atcggacggg cacgccgctc gatccggtgg cggtcgcggt ggtggccggc 720

caggtggcac acgacgtctt ccgcgtcctc gccggactgc gcgaggaggc gccgccccgg 780

ttggtggtgc tcgatccgcg gacgctggcg agcaccgcgc acccggtggt ggcgcacccg 840

ttcgagctgc ccgccgcgcc gctggacgag gcggcgttcc tcgaccgggt cgccgcgctg 900

cgggcagcgc ccgcgctgag cgaggcggag ttctcccggc gggccaaggg gctcatggac 960

tcgacagtgg ggctgttcgc cgagatcgac gagggcgacc tcgcgcagtt gccgctgcac 1020

gtcacggcga ccacggtggc cgacccgtgc gggctgatcg gctccgcgcc gcggcccgtg 1080

gtcaccggcg cggggctcac gttcgaggag gcccgctacc gcgcggcgcg cgccgcgctc 1140

gccctggccg gcactctcgc gctcgaccgg cggaggctgg tcgacggccg ggtgtgggcg 1200

tacgaccttg cggacggggc cgcccggctg gttccggccg acgaggtctt cgcaagcacg 1260

gcgggcggga gcacggcggg cgtgccgctc ggcgcggcgg cagcctactc gtgggccgag 1320

gcggtcgcgg agggcctggt cgcgcaggcc gccgcgctca ctctcgccgg cgccgggcac 1380

gcgggcgagc cgtatggacg ggcggacctc gccggggcgc ctgcgtatct cctcgccatg 1440

gtgcgggcgc tcggcgagga gctgaccgtg cacgacgtca ccgggccgct gggcgtgccg 1500

gtcgtggtgg ggacgctgtc cggcggggcg accgcctgcg gcgcgggcat gaccacggac 1560

gccgccgtca ccgcgtgcct gcgcgacctg ctgctgctgc gccaggccga gatcaacgac 1620

cagcccgtct acgccccacc atccggcagg cccttgcccg ggcatctacg cgggaaccac 1680

ccggcgcccg ccgtgcccgg cacggacgcc gaccggctgg cggcccgcct ggccgagcgg 1740

gggcaccgcc ctctggcggt gccgctggat catgaccagg ccgtgcacgc ggtcctgccc 1800

ttcgccgtcc gggtggtgtg ccgatga 1827

<210> 40
<211> 1737
<212> DNA
<213> nonomuraea sp.

<400> 40
atgatccgcc tgcccgacca cggcgagctt gccgtgccga tcggcgacgg gccgatgctg 60

ccgctgcgca ccgaactcgg ccgggtggtc atcggcccgc tggagcggcc cggcgtgccc 120

ggatgccggg cctgcgtgcg catccgcgcg gcgcgggtcc agcccgacgc ccgcaaggcc 180

gaggccgtac gcgcccggca cgcccccgcg cccagccagt ggctcacccc gctcgccacc 240

gacctggtgc gcgtcctggt cgcgagcgag agcgctgccc tggcagccga caccgacccc 300

cgcaccgcgg gtgccgtgct ggagatcgac ctcgccacgc tggaaatcac caggcaccgg 360

ttcctgcccg acccgctctg cccgcactgc ggcggtctgc cgccggacgg cccggccgag 420

ctgaccctgc ggccccgccg caagctcggc ggcagtcccc ggaccaggcc ggtcgagctg 480

aacgcgctgc tggagacgta cgtggacggc cgggtcggga tgatccggcc gctgaagtcg 540

ggcgtgcagg gcggactgac ggtggccagc gccatgctgc cgatccgcgc gggccacggc 600

ctggaaccgg gggtcggccg tacccgcagc tacaccgcga gcaggctggt cgccgtgctg 660

gaggcgctgg agcgctatgg cggcgtcagc cccggcggcc gccgcaccag cgttaccgcc 720

gcctaccgcg acatctccgg ccacgccgtc catcccgaca ccttcggcac acaccccgag 780

gagaactacg acaggcccgg cttcgccttc cggcgcttca ccgaggacac cgtgtgccgg 840

tgggtgtggg gctactcctt cgccaaggcc cagcccgtgc tggtgccgga gaaccaggtc 900

tactactacg ccagaaacct gccggacggc gagcagccgt tcgtcttcga ggtctccaac 960

ggctgcgcgc tcggctcgtg cctggaggag gcgatcctgc acggcctgct cgaggtggtc 1020

gagcgggacg cgttcctgct cacctggcac gcccggcggg aggttccggt gctgaacccc 1080

gcgctggcga gcgacccggt cctgcccatg caggccgccg cgatcaccgc cgagaccggg 1140

caccgggtgc tgtgcttcga caccaccgcc gagcacggca tccccagcgt gtgggcgatg 1200

gccgtggacg tcgaacaccg gtcggaccgg cccgccaccg cccacgccgc cggcgcggcg 1260

ctcacgctgg agcaggccgc catgaacgcg ctgagcgagc tcggcccgct gctggccgac 1320

gtgatccgcc gctaccccga ggagcgggaa cgggccgagg ccatggtccg cgaccccgag 1380

caggtcgcca ccatgcacga ccactccctg ctctacgcgg ttcccgccgc ggccgaacgg 1440

ctgtcgttcc tcaccggccg cgccgacggc ccgcggaccg gcttcggctc ggcccggttc 1500

accggcgacg acctcaccgc cgatctgcgg gccatgatcg acgcggtgat cgacgccggg 1560

atggacgtgg tggtcgtcga ccagaccacg ccggagcacc tggcgggcgg gttccgctgc 1620

gtgaaggtgc tggtgcccgg cgcgctgccg atgacgttcg gccaccagta ccggcggctg 1680

gacaacctgc cgcggctggc ggccgcgcgt accaccgacc cgcacccgtt cccatga 1737

<210> 41
<211> 2574
<212> DNA
<213> nonomuraea sp.

<400> 41
atggcggtta ttgagcggga agaaagcgac ggcccgccgg tcgctgtccg cacgtgcggg 60

gtcgcggccg cggagctgga atccctgcga tgggacgagt cctatgcgct ggtgacccgg 120

ctgctcgcgg agaccgcccg ggtggcggcg gacgggcggc ggctggccgt ggcgctcggt 180

gacgtcatcg gagggctcgg cccgtccggg tcccggcccg tcctcgtggg gctgcggcgg 240

gcgctgcaca ccgggcgtct cccctccggg cgggagtggg ggcccgaggc cggggcggcg 300

ctgccgtacg ggctgagggg cgaggtggag gagtgggtac ggcgtgcgcg cgagtgcgcc 360

gggctgcgcg cccgcctgcc cgaggtcgtc gcccgggagt ccctggagaa ggaggaacgg 420

ctgcgcgccg ccgcggccga ccccgccttc cgcagggggc tggcgctggc cggcggcgag 480

ctggcggtcg acctggagac gtggctcgcg gaccccgcgc ggcgccccaa gccgcagaag 540

ctgctcaggc tggccaaata cctggtcagg gccgccgtga agaccagccc gtacagcacg 600

ttcacgagca ccgggcgggc ggtgtggggc ggtggcgagc ggcacgtgga gcgcgtggtg 660

ccggtgctgg aactcgacgg cgtccagacg ttctcggacg atcgggtcag ggtgaatccg 720

tcggtcaccg tcgcgggcgg gacggcggag ttcatcgggc cgcctcccgg ggaggcgctg 780

gtgtccatcg ggtgacggaa ggcggtggcc gcgtgcctgc gcgtcgcgga gggcggggag 840

tgggtgccgc ggggccggtt cgcggaggct ctgggggctg agcctgccgc agtcacgaaa 900

tttctggaca agttgcttgc ggttgggctc ctggaggccc ggccggacgg cgacccgccc 960

ccgcacctgc tggacgggat ccggcgcacg gaacgcgcca ccgacccgtc caccttccgc 1020

cacgaactcg gccgcctgat cgccgccgcc cgcgccgccg cccgcgccac cgcccccggc 1080

gctgacacgg ccaagaacgc cgcccctgcc gggcgcgcca cccgcggcga tggcggtgct 1140

gatgccgagc gcgccacccg cggcgatagc gatgctgatg acgggcgcgc cacccggggc 1200

gacggcggtg ctgatgacag gggcgccgcg cggggcgacg gcggtgtggg gcgggcggtt 1260

gtgcacgagg tcgcggtctg cgtcgagccc gtggcgaggc tggatccggc gcgatggcgt 1320

ggcgggcttg ccgatctcga cgtcgtacgc cgctggctgg ccgttttcga cgccaagcac 1380

cccatgcgcc tcgcggtcgc cgcctacctc accacccggt acggccccga cccggccgtc 1440

cccttcctca cgctccaccg ccacatccag cgcgagctgg ccggcaccgg cgcggcaggc 1500

cgggagttgc gcgcgttcct cggctcgtcg gccgcctgga cccagccgct aggcacctcc 1560

gccctgccca ggatcaggga gctggagcgc ctcagagccg aggcgagaag cctggcgctg 1620

gacgcggagg accccgacgg cacctgccgc gtcacccccg gacagctggc cgcgcagctg 1680

gagtcgtggc cgtcttggat cgcgatcccc gcctcctcgg cctgctacgt acaggcggtg 1740

gccggcaccc tggtgctcaa cgtcgtccac ggcgggcacg gccgagccct gcgcaggctc 1800

gaccacctcc tcgaacgcgc cggcggccac cccgagccgc cccggatcga ggacccggac 1860

ggcgcggtct acgccgagtt ctccggcgac ctgggctcca cgctcaacgc gcgcccgccg 1920

agcacccgat acgagatcga ctacccccac tcgcccggca cgcgcccgcc cgacctgcgg 1980

ctgccgctca cggacctgca cgtgacgctc tgcccggaca gcggcctgcc cgagctgcgt 2040

tccaagcggc tcggccgccg ggtcgtgccg ttgcatctcg ggctggccgc cgagttccgg 2100

cttccgccgg ccgcgcggtt catcgaacgc gtcttcggcc ccggctacct gctccatccc 2160

agctcgccgc cgctggtgcg gatgggccgg gtcccctcgg aggtcacccg gtatccacgc 2220

gtcgaggccg ggcgcgtggt ggtgcagcgg cgtcgctggc tggcgcctgc ggccacgctg 2280

ccggtgcgcg cgaagggcga gggcgacgcg gcgtacctca cgcggctgat cgcctgggcg 2340

gacgagtacg gcgtaccgca ccgatcgttc gtccgcgcct ggccggagca gaccggcgac 2400

aaagggcagg acaaggcgcg caaaccgctc ttcctggacc tggcgaacct gttcctggtc 2460

aagaacttcg agcggcagat acgtggctgc gcgttcgcgc tcttcgagga ggcgctgccc 2520

gacccgggac cggagcgggt gaccgaatac ctgatcgagg tgggaggccg atga 2574

<210> 42
<211> 816
<212> DNA
<213> nonomuraea sp.

<400> 42
atgagctggg tgagcgtgca cgtcttctcg caggggccgc tcgacgacat gatcacgggc 60

ctggccgggc cgctcctcgg cgacgacggc ttcttcctgc gctactggga gggcggcccg 120

catctgcgga tcagggtcag gtcccacccg gcggagcagg tcgtcgagcg ggccgaggcg 180

tatctgcgcg agcacccgtc agaggcggtg atcgacccgg aggagtacgc caggctcgcc 240

gcgaccctcg ccgcccgcga aggcgtcccc ggctatacgc ggcgcctcta tccgaacgac 300

tccgcccacc tcatcccgta ccagccggaa cacgaccgct acggcacggg ccgctcgctc 360

gacgccgtgg agcggcactt ccacgactcc agcgtgatcg cactccagct gatcggggcg 420

gggctcggac acgagcggcg gagcatggcg tggctgacca tgctgctggt cgcctggcag 480

gtcgcggggg agggcagggc gttcggcccg cccgctgacg ccggggttca ggtgccccac 540

gagcggatgg cggcgctggt cgagcacgcc agggcggtga cacaggagcc tgcgccgagc 600

gagccccgtg gcgtggtggc cgcctggtac gcctcgatcg cgcggctggt ggaggaactg 660

gcggcggcgg ggttcgacgc ggcgcggacg ggggcgaccg tcgatctggc cgcgcatctg 720

ctggccaaca ggctggggat ccgggtgcag gaggaggccc ggctgcgtca cctggcgtcc 780

ggggccacga gggagtcgga ggtgggggtc ggatga 816

<210> 43
<211> 960
<212> DNA
<213> nonomuraea sp.

<400> 43
atgacctgga cgcggctgcg ggtggactat cacgacggcc cgatggacga cctcatcctg 60

gacgcgctcc gcccggcctg gcatgaaata cggggatatt tcctgcgcca ctgggtgtgc 120

ggcccgcacc tgcggatctt cgtggacggc gacgcgacgc cgatcgtccg ggcggtcgag 180

cgccacctgg ccgcctgccc gtccgccggc accaccgacc cgcaggcgct gctccccctg 240

cacgagcgcc tggccgagct ggaaggcgaa cgaggcccgc tgctgccatg ggcccccgac 300

aacaccgtca ccgccgaacc cccggggctc gacaccgagc tcgaccggtt cctcgcggac 360

ttctacgccg acaccaccga ggccgcgttc gacgccctcg gccgggtcag agccggcacg 420

ccgctgccgg gcatcgcctt cgacctggtg gtcgccaccg cgcacgacct gtcggagggc 480

ggcctgccca ctgccaggac ctcgctgcgc tcgcacgccg aggcttacct gtcgcgcctg 540

ccgggaggcg tccgcgccaa gtggcaggcg cactacgaac gcaaccagga gccgctgacc 600

gccaggatca aggccctgac cggcgccgga gagcccggcg cgtggctgcg tacgatccgg 660

gccacccgcg accggggccg taccctcatc gacgagggac ggctgagcct cggctacgcc 720

accgacggcc cttccacgcg gcccccgctc gccgcggtct cccccttcca ccggaacctg 780

gagaccgacg aacgctggct cgccctcaag gacacccccg ccttcgccgc ctaccgcctg 840

gccctgaact gcgcgtacct ccacctcacc cggctcggcc tcacgcccga ccagcgcttc 900

ctgatctgcc acctcgccgc gagcgccgcc gacgacgtct accgggccgt ggcctcatga 960

<210> 44
<211> 1287
<212> DNA
<213> nonomuraea sp.

<400> 44
atgaccgggg aacggtacga gctgcgcgcc ggcgtgcacc tcgcccggct ggacgcgggg 60

gtccgcctgt tcgcctggcc gcacgccgag accctcggcg agctcacgcg ggatgagctc 120

gacctgctcc gcgacctggc cgccggaccc cgcaccgccg ggggaccgct cgtcgaacgc 180

ctgcgcgccg gaggctggct caagatcacg attacccggg acggcgtgcc cctctacacg 240

ctcgccccgc tgcgcccgcc gccgccacgg ccggcggtgc cggagcggcc ggttctgtcc 300

aggttcgcca cgctgcggcg cgagggggac gcgctggtcg ccggctcccc gctggcctgg 360

tgcgacctgt acgtccacga ccccggcctg gtcggcgacg tggtccggcc cggcggcgag 420

gtcctggccg acctggtgtg ggccgggctg gccgtgcccg aggaggccga ggaccgggag 480

ctgcggttcc ggcagtgggc gccgcacgag ctggagttcc atgaccggag ccgggtgggg 540

caccgcggct acctgggcga tgggttcggc gggacgttct gggcccgcgg ccggttcgac 600

ccgctgcccg cccggccccg gccctacccc ggccgggcga tcgagctgcc cacgcccgac 660

ctggacgaac tgcggctcaa ggacccctcg ctcacggacg tgctggaggg ccgcaggtcc 720

gtacgcgagc acgacgacga gcgcccgatc accctcgacc tgctcggcga gctgctgtac 780

cgcacggcca gggtgcggga gtgctggacg gccgacggcg tcgaatacac ccgcaagccg 840

tacccctccg gagggtccgt ctacgagctg gagatctacc ccgtcgtcag gaacgtggcc 900

gggctcgaac ccggcatgta ccactacgac gggcacgacc acgtgctcag gccggtacgt 960

gaggccggcc atcccgccgt acgccggctg ctgaccgtcg ccgggcacgg cgccgccacc 1020

ggccgccgcc cccaggtcct gctcgcggtc tccagccgcg ccggccggct gctgtggaag 1080

tacgaaggca tggggtacgc gctgacgctc aagcacgtgg gtgtgctcta ccagacgctc 1140

tactgcgtgg ccacggcgat gggcctggcg gcctgcgggc tcggcagcgg cgactcggcc 1200

gcgttcgccg aggcgaccgg gcgggacccg ttggaggagt gcgcggtcgg cgaattcatg 1260

atcggaaccg cggaggcacc acgttga 1287

<210> 45
<211> 1893
<212> DNA
<213> nonomuraea sp.

<400> 45
ttgaacgact tcaaacccag actccccgcg gacatacgct tcgcgcccac ctccggcggt 60

gtcgccttcc tcacccgcga aggcgtcgtg cggatgaacg gggcctccgt ccacctgtgg 120

gtggagcggc tggccgtgca cctcgacggg agcaggaccg tggccgagct gaccgcggag 180

ctgcccgacg agcggcgggc gttcgtgacg agcctgctga ccacgctgcg cgagcgcggg 240

ctcgtggagg acgcgggaga gcgcggcgtg ctgcccgtgg cggccggcac cgcgtgctcg 300

ctgctggccg acgcggaccg gttcggcgag ctggcgcccg ccctggccga cgccctgcgc 360

aggctggggc tggaggtgct gacagagccg tcgggcgggg cgctcgtgca cgtcgcggcg 420

gccgacgcgg acgggctggc cgccgccggg cgcctcgacc ggctgagcgc cgagcgcggg 480

ctgcccatcg cgcacgtgct gctgcgcgag ggcgaggcgt ggtggagcga gaccggcgtc 540

gtcgggcgcg acgtcccgcc gtgggcggcc gggtggcggc ggctggcggc catggaggcc 600

gtcccgcgta ccgccgggca cgtctccgtg tcgcccgtcg ccgcggccgt ggtcgccggg 660

caggtggtgg cggacctggg gcgctgcctg tccggaacgc cccgcgtgtc cggcggtccg 720

cggctgcacg tggtggacct ggacgggttc gggatgcgga cgcactcctt cctgccgcac 780

ctgtcggcgc tgccgcccgg cacgggggtg agcgcggggc cgctcggcga ggaggagttc 840

tcgcggcggg cggccaggtt gatggactcg cggacggggg tgttcgccga gatcgaggag 900

ggggagtacg ggcagctccc gctgcacgtg tcggtgacca cggtggccga tccgatgggg 960

gccctcgggg gtgcgctggc gcgggtgatc ggcgtggggg cggacttcgc caccgcgcga 1020

taccgggcgg cgcttaaggc actgaccgtg cacgggctgc tcgccctcga cccccgccgc 1080

ctggtcacgg ccgaggggac gcccctgacc ggcgcggggg aacccgccag cggcttcccg 1140

gctgatccgg gcgcgaggga acggccgatc ggtggctttt cggctgatcc gggtgcgggt 1200

gagcggccgg acgacggtct cctggacgac ccgctggtgg gcgacgtcgc ggcgggacgg 1260

gtcgaggcgt tcgtgcgcgg ccgtgacctc gtcgagggcg gtgaggtgct gctgcccgcc 1320

cgcgcggtgt tcctggatcc gcacgccacc ggggccgccg cggggtactc ggccgaggag 1380

gcgctggtcg cggggttact ggaccactgt gcggcactgg ccgggctcga cgggacgctg 1440

ccgcccgtgg acgtgggcgg cgcgcgggcg gccgacgacg agaccgcgcg gtcggcggcg 1500

ctgctggacg cgatcgggct gccgtaccag gtgctggacg ccaccggccc gtcgggcgtg 1560

cccgtgcacg cggggacgct cggcgggcgg ctggtggcgc gggcggcggg cgtcacgccc 1620

ctggccgcgc tgcgggccac gctcgaaggg atcctggccg cgtaccaggg cattccgggc 1680

cccgcacccc tgcccgacct gtgcccggcc gtcgcgcagg agccgccggt cgtcctcgac 1740

acgggggcgg ccgtgctcgg cgccggtgag ctcgccgccg ccctggccgg cgccgggctg 1800

cgggtcagcg ccgttccgct ggatcacgac cgggaggtac acgcgctcat gccgcacgtc 1860

gtccgcgtcg tcacggagcc gctccatggc tga 1893

<210> 46
<211> 1833
<212> DNA
<213> nonomuraea sp.

<400> 46
gtggggacgg gcctgctggc cgaggagatc acgatcgcgc tgaaggaccg cgacgggcgg 60

gcagaggtga cgatcaccgc ctgcgacggc tgggcgcgcg gcccccggcg cgacggggcg 120

gcgtggttgc ccgtgcgggt cgagtacggg gtcgccgtca tcgggccgct ggaacgcccc 180

ggcgaacccg gctgcgtgac ctgcttcgag acccgccggg accgggtcag gaccgacagc 240

gccgatcggg ccgccgtcct ggccgtccac ggccccgaac tcgccgggcg ggcctccccg 300

tacctcggcc gcctggccgc ccgcacgatc gcggccctgg ccgcgcacgc cgccgagggc 360

cccggatgcg ccgtctggta cgtcgacctg cgcacgctca ccgccgaacg ccacacgttc 420

ctgccggaac cccactgccc cgactgcggc gacctgccgg acgacgaccg cgcgtccgcc 480

gccttcgagc ccggcgccgc cccgcgacct cccggctacc gggtcaggaa cgtgccggcc 540

gagcacggcg cgctgctcga cacgtacgtg gacggcgaat gcgggctgat caggcccctc 600
gtccgggaca cgctcggcgg cctggtgatc gccggggcga tgctgccgct gcggttcgag 660
ggcggttcgg agcccggcgt gggccgcacc cgcggctacc gggccagcga ggtcaccgcc 720
gtgctcgaag cgctcgaacg gtggggcggc gtcgagcccg gcggcaagcg cacccgcgtg 780
cgggcctcgt acgcggaggt ggccgcggac gcgctcgacc ctcgaacgct gggcgagcac 840
gacacctcct cctacgagat ggaggggttc gcgttccggc cgttcgccga ggacgccgtc 900
tgcgactggg tgtgggggta ctccttcgcc cggcgctcgc cgatcctcgt gccggagagc 960
ctggcctatt actacgtacg ggggcgggag cggccgttcc tcttcgagat ctcgaacggg 1020
tgcgcgctcg gcggcagcat ggcggaggcc ctgctctacg ggctgctgga gacggtcgag 1080
cgggacgcct ttctcatgac ctggtacggg cggctcccgg tgccgcggat cgaccccggc 1140
acggcacggg accgcaccat cccgctccag gccgccgcga tcatcgcgga gaccggttat 1200
cgggtggagc tgtacgacac gacgatggag cacggcatcc ccagcgtgtg ggcgatgggc 1260
gtgcggcagg acggcgatcc gggcaggccg cgcctggtgt gcgccgccgg ggcccacctg 1320
gtgccggaga aggccgtgct cagcgcgctg agcgagctgg ggccgctgct ggccgacctg 1380
atccggcggt atccggacga ggtgcaccga gcccaggaga tggtgacaaa tcctgatctg 1440
gtggcgacga tgcacgacca ctccaccctg tacggcgccg acgcggcctt cgaccggctc 1500
tcgttcctga ccggcggcac gggcgtgcgg gggctgcccg acatggaggc gttcaccgtg 1560
cccggtgatc tcgacgtcat gagggtggtg gaccgcttcc tcgccgaggg gatggacgtc 1620
gtcgtcatcg accagaccac gccggagcat cgggcccgcg gtttctcctg cgtcaaggtg 1680
ctggtgcccg gcacgctccc gatgaccttc ggccaccgca accgcagggt gcggggcctg 1740
acccgcccgc tggagctgcc gtaccggctc ggccaccgcg acacgcccct gacccccgcc 1800
gacctgaacc ccgacccaca ccccttccca tga 1833

<210> 47
<211> 237
<212> PRT
<213> nonomuraea sp.

<400> 47

Val Thr Ala Arg Arg Arg Asp Val Glu Arg Val Asp Arg Ile Val Asp
1               5                   10                  15

Ala Ala Ala Glu Leu Leu Pro Glu Arg Gly Tyr Arg Arg Leu Thr Val
            20                  25                  30

Glu Glu Val Ala Ser Arg Ala Gly Val Ser Lys Ser Ser Val Tyr Leu
        35                  40                  45

His Trp Asn Thr Lys Asp Glu Ile Phe Tyr Ala Val Leu Asp Arg Glu
    50                  55                  60

Phe Thr Val Leu Ala Gln Ala Ala Val Asp Arg Val Arg Arg Asp Pro

65                  70                  75                  80

Ala Glu Val Leu Ala His Arg Thr Ala Ala Asn Leu Phe Gln Ile Val
                85                  90                  95

Ala Cys Arg Pro Leu Leu Gln Ala Leu Leu Val Asp Asp Arg Thr Thr
            100                 105                 110

Leu Gly Ser Leu Gln Pro Ala Lys Ser Ala Val Phe Arg Ala Ser Leu
        115                 120                 125

Ala Asn Leu Asn Glu Leu Met Tyr Arg Tyr Leu His Ala Leu Arg Ile
    130                 135                 140

Asn Gly Leu Leu Cys Pro Glu Val Asp Pro Arg Ile Met Arg Lys Ala
145                 150                 155                 160

Ile Ser Glu Met Leu Arg Gly Met Thr Phe Ser Ala Gly Ala Lys Pro
                165                 170                 175

Leu Ala Glu Thr Arg Leu Ala Lys Leu Ser Gln Val Val Ala Val Thr
            180                 185                 190

Val Gln Arg Ala Phe Glu Pro Gly Asp Val Pro Glu Leu Asp Arg Ile
        195                 200                 205

Asn Ala Ala Ala Ala Glu Val Phe Lys Ala Phe Asp Glu Leu Met Pro
    210                 215                 220

Ala Glu Glu Thr Met Arg Ala Glu Arg Pro Val Ala Leu
225                 230                 235

<210>  48
<211>  633
<212>  PRT
<213>  nonomuraea sp.

<400>  48

Val Thr Val Val Arg Ala His Val Arg Gln Glu Pro Ala Ala Leu Val
1               5                   10                  15

Val Gly Arg Gly Leu Leu Ala Asp Arg Leu Ala Ala Val Thr Gly Arg
            20                  25                  30

Leu Asp Pro Gly Val Val Leu Ala Val Ser Asp Ala Pro Asp Pro Ala
        35                  40                  45

Val Leu Ala Glu Ala Arg Ala Thr Gly Arg Pro Val Leu Pro Val Leu
    50                  55                  60

Ala Glu Pro Gly Arg Val Arg Val Gly Pro Leu Glu Arg Pro Gly Glu
65                  70                  75                  80

Pro Gly Cys Ser Asp Cys Leu Ser Leu Arg Arg His Arg Ala Ala Ser
                85                  90                  95

Arg Ser Ala Glu Arg Ala Ala Ala Trp Leu Arg His Gly Pro Asp Leu
            100                 105                 110

Ala Ala Thr Pro Ser Pro Leu Leu Ala Pro Val Ala Leu Asp Leu Val
        115                 120                 125

Ala Glu Leu Val Thr Ala Leu Val Thr Ala Pro Ala Pro Ala His Arg
    130                 135                 140

Gly Ile Leu Leu Ala Asp Leu Ser Asp Leu Ser Val Thr Pro His Ala
145                 150                 155                 160

Phe Leu Pro Asp Pro Phe Cys Pro Arg Cys Gly Thr Leu Pro Asp Asp
                165                 170                 175

Asp Pro Ala Arg Ala Arg Ile Thr Leu Val Pro Arg Pro Lys Pro Ser
            180                 185                 190

Pro Asp Arg His Arg Val Trp Asp Ala Glu Gly Arg Leu Asp Arg Leu
        195                 200                 205

Arg Gln Ile Tyr Val Asp Asp His Cys Gly Leu Val His Ser Leu Asn
    210                 215                 220

Pro Ala Ala Leu Gly Ser Leu Ala Val Ala Gly Ala Pro Ile Arg Leu
225                 230                 235                 240

Arg Gly Thr Thr Ala Phe Glu Pro Gly Phe Gly Arg Ser Arg Ser Tyr
                245                 250                 255

Arg Arg Ser Ser Val Ile Ala Leu Leu Glu Ala Leu Glu Arg Tyr Gly
            260                 265                 270

Ala Ile Gly Pro Gly Gly Arg Arg Gly Thr Val Arg Ala Ser Tyr Ala
        275                 280                 285

Ser Leu Gly Asn Leu Ala Val Asp Pro Arg Ser Leu Gly Leu His Pro
    290                 295                 300

Pro Glu His Tyr Ala Leu Pro Gly Phe Pro Tyr Arg Pro Phe Thr Pro
305                 310                 315                 320

Glu Ala Val Cys Arg Trp Leu Trp Gly His Ser Phe Ala Thr Gly Gly
                325                 330                 335

Pro Val Leu Val Pro Glu Arg Asn Val His Tyr Gly Pro Ser Asp Asp
            340                 345                 350

Asp Arg Pro Phe Cys Tyr Glu Leu Ala Asn Gly Cys Ala Leu Gly Ser
        355                 360                 365

Cys Leu Glu Glu Ala Val Phe His Gly Ile Leu Glu Val Leu Glu Arg
    370                 375                 380

Asp Ala Phe Leu Leu Thr Trp Tyr Thr Arg Ala Arg Ala Pro Arg Leu
385                 390                 395                 400

Asp Leu Gly Thr Ala Arg Asp Pro Thr Ile Pro Leu Val Ala Ala Ala
                405                 410                 415

Ile Thr Ala Glu Thr Gly Tyr Leu Val Glu Cys Tyr Asp Ile Thr Pro
            420                 425                 430

Asp His Gly Val Pro Cys Val Trp Ala Leu Ala Arg His Pro Ser Asp
        435                 440                 445

Gly Glu Pro Ala Thr Ile Ser Ala Ala Ala Ala Gly Pro Gly Leu Glu
    450                 455                 460

Pro Ala Ala Ala Gly Ala Leu Ala Glu Leu Gly Pro Met Val Pro Thr
465                 470                 475                 480

Val Arg Asp His Phe Pro Gln His Ala Gly Arg Ala Arg Glu Leu Ala
                485                 490                 495

Ala Asp Gly Arg Arg Val Arg Ser Met Ile Asp His Tyr Leu Val Tyr
            500                 505                 510

Gly Val Arg Ser Ala Ala Arg Arg Leu Ser Phe Leu Thr Asp Gly Thr
        515                 520                 525

Glu Glu Val Pro Phe Ala Pro Ala Pro Asp Gly Phe Arg His Asp Asp
    530                 535                 540

Leu Thr Ala Asp Leu Arg Phe Leu Leu Asp Arg Leu Ala Asp Ser Gly
545                 550                 555                 560

Leu Asp Val Val Val Val Asp Leu Thr Thr Pro Glu His Arg Ala Gly
                565                 570                 575

Gly Leu Arg Cys Val Lys Val Leu Val Pro Gly Thr Val Pro Met Thr
            580                 585                 590

Phe Gly Glu Gln Asn Arg Arg Thr Trp Gly Leu Pro Arg Leu Leu Asp
        595                 600                 605

Pro Ala Val Val Arg Gly Arg Gly Met Pro Val Arg Gly His Ala Asp
    610                 615                 620

Leu Asn Pro Asp Pro His Pro Phe Pro

625                 630

<210>  49
<211>  829
<212>  PRT
<213>  nonomuraea sp.

<400>  49

Val Ser Arg His Ala Pro Pro Ala Val Ala Arg Val Gly Gly Leu Pro
1               5                   10                  15

Ala Ala Ala Leu His Gly Val Ala Cys Pro Glu Ala Thr Arg Leu Ala
            20                  25                  30

Gly Arg Val Val His Leu Thr Glu Glu Leu Thr Arg Arg Ala Ala Val
        35                  40                  45

Leu Ser Asp Ala Leu Tyr Glu Val Ile Gly Ala Ala Gly Ala His Lys
    50                  55                  60

Pro Ile Leu Val Ala Ile Arg Arg Asp Leu His Gly Leu Arg Arg Pro
65                  70                  75                  80

Lys Arg Val Glu Val Leu Pro Ala Pro Leu Ala Glu Pro Val Arg Thr
                85                  90                  95

Trp Ile Ser Leu Trp Glu Glu Arg Ala Arg Val His Ala Val Leu Pro
            100                 105                 110

Asp Val Leu Ala Arg Glu Glu Arg Leu Ala Trp Glu Ala Leu Arg Glu
        115                 120                 125

Leu Ala Ala Thr Pro Ala Val Arg His Gly Leu Ala His Ala Ser Pro
    130                 135                 140

Asp Leu Ser Ala Asp Leu Glu Lys Trp Leu Ala Asp Thr Gly Trp Arg
145                 150                 155                 160

Pro Arg Pro Ser Thr Leu Ala Ser Leu Leu Arg Tyr Val Lys Arg Val
                165                 170                 175

Ala Ala Lys Thr Ser Pro Phe Ser Thr Phe Thr Thr Val His Glu Val
            180                 185                 190

Arg Trp Val Asp Gly Gly Ala Ala Trp Gln Val Pro Asp Thr Ala Pro
        195                 200                 205

Thr Val Val Val Glu Ala Asp Val Gly Leu Arg Leu Leu Val Glu Ser
    210                 215                 220

Val Leu Pro Arg Met Pro Glu Val Ala Ala Ala Arg Val Val Arg Leu
225                 230                 235                 240

Ser Pro Ala Ala Tyr Ala Ser Gly Asp Arg Leu Ala Phe Pro Glu Pro
                245                 250                 255

Gly Gly Arg Met Arg Ala Val Asp Arg Thr Ser Ala Leu Asp Thr Leu
            260                 265                 270

Val Glu Leu Leu Arg Ala Glu Gln Arg Trp Asp Ala Ala Ile Ala Lys
        275                 280                 285

Leu Thr Gly Ala Gly Leu Ala Gly His Gly Ala Glu Asp Gly Thr Arg
    290                 295                 300

Asp Ser Ala Ser Asp Gly Thr Ala Ala Ala Glu Gln Val Leu Ser Gly
305                 310                 315                 320

Leu Val Arg Gly Gly Leu Val Glu Ala Val Val Pro Val Pro Gly Gln
                325                 330                 335

Ala Ala Arg Pro Phe Ala Arg Leu Ala Asp Trp Ala Ala Pro Ala Ala
            340                 345                 350

Pro Ala Gly Pro Leu His Arg Ile Gln Val Ala Leu Asp Gln Ala Gly
        355                 360                 365

Pro Leu Gly Asp Gly Asp Pro Leu Ala Ser Ala Cys Ala His Val Ala
    370                 375                 380

Arg Arg Leu Thr Ala Glu Leu Pro Ala Leu Gly Leu Pro Val Met Pro
385                 390                 395                 400

Val Pro Asp Leu Arg Arg Arg Val Leu Arg Glu Ser Ala Leu Gly Ala
                405                 410                 415

Pro Val Ala Cys Ala Leu Arg Glu Trp Arg Pro Val Leu Ala Asp Leu
            420                 425                 430

Gln Arg Val Arg Arg Trp Leu Ala Val His Asp Pro Met Leu Pro Leu
        435                 440                 445

Arg Leu Thr Leu Ala Asp Arg Val Arg Asp Trp Phe Gly Pro Gly Ser
    450                 455                 460

Ala Ala Pro Leu Leu Asp Val Tyr Ala Arg Val Arg Thr Ala Glu Pro
465                 470                 475                 480

Gly Thr Pro Leu Asp Pro Asp Phe Leu Glu His Pro Asp Pro Leu Ala
                485                 490                 495

Gly Val Thr Asp Pro Arg Leu Thr Arg Leu Arg Glu Leu Arg Ala Ala
            500                 505                 510

Ser Val Ala Ala Leu Thr Gly Gly Arg Ala Glu Glu Ala Leu Ser Glu
        515                 520                 525

Pro Pro Ala Trp Val Arg Asp Pro Gly Pro Val Thr Cys Tyr Val Gln
    530                 535                 540

Pro Phe Gln Gly Glu Asp Gly Leu Arg Leu Val Leu Asn Ala Ala His
545                 550                 555                 560

Gly Gly His Gly Arg Gly Ile Thr Arg Trp Thr Arg Leu Leu Gly Ala
                565                 570                 575

Glu Pro Pro Pro Gly Glu Pro Pro Ala Ala Glu Ser Pro Pro Gly Glu
            580                 585                 590

Ser Pro Pro Gly Glu Leu Pro Pro Gly Ala Cys Leu Val Ala Glu Leu
        595                 600                 605

Pro Gly Thr Phe Gly His Ser Leu Asn Leu His Ala Pro Ala Thr Gly
    610                 615                 620

Trp Glu Leu Thr Tyr Pro Gly Ala Val Asn Gln Ala Pro Pro Glu Arg
625                 630                 635                 640

Arg Val Pro Leu Ala Glu Leu Gln Val Arg His Asp Ala Gly Arg Gly
                645                 650                 655

Val Val Glu Leu Trp Trp Pro Arg Ala Gly Arg Arg Val Val Pro Val
            660                 665                 670

His Ala Gly Met Met Ser Glu Thr Leu Leu Pro Pro Leu Ala Arg Leu
        675                 680                 685

Leu Val Glu Ala Phe Gly Thr Thr His Leu Thr His Pro Thr Leu Pro
    690                 695                 700

Pro Val Val Arg Ala Ala Gly Pro Arg Ile Asp Leu Gly Arg Val Thr
705                 710                 715                 720

Leu Ala Arg Ala Gln Trp Thr Ala Arg Arg Glu Glu Ile Pro Arg Arg
                725                 730                 735

Gly Gly Asp Asp Ala Ala His Leu Val Ala Val His Ala Trp Leu Arg
            740                 745                 750

Ala Ala Gly Ile Pro Arg Arg Cys Phe Val Arg Val Arg Glu Pro Gln
        755                 760                 765

Val Arg Arg Asp Arg Leu Ala Phe Asp Lys Arg His Lys Pro Val Phe
    770                 775                 780

Val Asp Phe Gly Ser Trp Pro Ser Val Leu Glu Phe Asp Arg Ile Val

785                 790                 795                 800

Thr Arg Ala Thr Gly Asp Leu Glu Leu Thr Glu Ala Leu Pro Asp Gly
                805                 810                 815

Glu Arg Ala Val Glu Leu Ala Ile Glu Ile Gly Ala Arg
            820                 825

<210>  50
<211>  344
<212>  PRT
<213>  nonomuraea sp.

<400>  50

Val Thr Arg Tyr Pro Leu Pro Arg Pro His Pro Leu Gly Ile His Pro
1               5                   10                  15

Gly Tyr Ala Arg Leu Arg Glu Gly Cys Pro Val Ala Arg Val Asp Ser
            20                  25                  30

Pro Tyr Gly Pro Ala Trp Leu Ile Thr Arg Tyr Ala Asp Val Ala Ala
        35                  40                  45

Val Leu Thr Asp Ala Arg Tyr Ser Arg Ala Ala Ala Thr Glu Asp Asp
    50                  55                  60

Gly Gly Ile Leu Leu Asn Thr Asp Pro Pro Glu His Asp Arg Leu Arg
65                  70                  75                  80

Arg Leu Val Val Ala His Thr Gly Ala Ala Ala Val Glu Arg Leu Arg
                85                  90                  95

Pro Arg Ala Glu Glu Ile Ala Thr Thr Leu Ala Lys Arg Ile Pro Ala
            100                 105                 110

Glu Ala Glu Phe Val Ser Ala Phe Ala Glu Pro Tyr Ser His Arg Val
        115                 120                 125

Leu Ala Leu Phe Val Gly His Leu Val Gly Leu Pro Ala Gly Glu Leu
    130                 135                 140

Ser Pro Met Ala Thr Val Leu Thr Leu Ala Arg Val Pro Asp Arg Glu
145                 150                 155                 160

Arg Ala Ala Ala Phe Ala Glu Leu Arg Arg Arg Leu Arg Ser Gln Val
                165                 170                 175

Asp Asp Glu Thr Leu Ala Val Val Leu Asn Ile Val Phe Gly Gly His
            180                 185                 190

Ala Ala Val Val Ala Ala Leu Gly Tyr Cys Leu Leu Ala Ala Leu Ala
        195                 200                 205

Glu Pro Leu Pro Ser Leu Thr Gly Asp Val Arg Arg Ile Ala Glu Leu
    210                 215                 220

Val Glu Glu Thr Leu Arg Leu Ala Pro Pro Gly Asp Arg Thr Leu Leu
225                 230                 235                 240

Arg Arg Thr Leu Glu Pro Val Glu Leu Gly Gly Arg Glu Leu Pro Ala
                245                 250                 255

Gly Ala Leu Val Ile Pro Ser Ile Ala Ala Ala Gly Arg Asp Pro Asp
            260                 265                 270

Arg Pro Thr Gly Gly Pro Ala Gly Arg His Leu Ala Phe Gly Arg Gly
        275                 280                 285

Pro His Ala Cys Leu Gly Met Ala Leu Ala Arg Met Glu Leu Arg Val
    290                 295                 300

Ala Leu Gln Ala Leu Ala Glu His Val Pro Gly Leu Arg Pro Ala Val
305                 310                 315                 320

Ser Pro Ala Thr Leu Arg Arg Thr Cys Glu Glu Leu Ala Val Ser Pro
                325                 330                 335

Leu Ala Gly Ile Pro Ile Arg Val
            340

<210>  51
<211>  228
<212>  PRT
<213>  nonomuraea sp.

<400>  51

Met Val His Ala Phe Gly Val Arg Asn Arg Gly Arg Ile Val Ser Ala
1               5                   10                  15

Glu His His Tyr Arg Asp Arg Leu Arg Ala Leu Ser Ala Tyr Leu Arg
            20                  25                  30

Lys Val Asp Asn Glu Pro Glu Ala Ala Ala Glu Leu Arg Ala Asp Pro
        35                  40                  45

Glu Lys Ala Leu Arg Glu Ala Gly Val Asp Gln Ala Phe Asp Arg Pro
    50                  55                  60

Glu Ala Phe Arg Ala Phe Val Gly Lys Leu Ala Val Leu Ser Gly Glu
65                  70                  75                  80

Ala Trp Leu Ala Thr Val His Ser Met Ile Glu Leu Cys Glu Ile Gly
                85                  90                  95

Ala Asp Pro Gln Pro Pro Ala Gly Pro Asn Ile Ser Phe Arg Ile Ser

100                 105                 110

Ser Asp Gly Gly Val Thr Ala Ile Ala Asn Arg Gly Glu Ile Ala Lys
        115                 120                 125

Lys Val Gln Pro Asn Pro Phe His Ala Gly Ser Ala Ala Ala Ser Pro
    130                 135                 140

Gly Gly Arg Leu Arg Ile Tyr Pro Gly Tyr Ala Thr Ser Glu Leu Ser
145                 150                 155                 160

Ala Arg Leu Gly Glu Arg Tyr Leu Ser Thr Phe Tyr Gln Arg Thr Leu
                165                 170                 175

Leu Lys Arg Val Val Leu Asp Pro Gly Thr Val Val Glu Asp Ala Asp
            180                 185                 190

Ala Gly Glu Gly Ile Thr Val Asn Arg Ser Gln Tyr Arg Gly Val Gly
        195                 200                 205

Phe Glu Leu His Thr Arg Ala Asp Gly Ala Asp Arg Glu Ile Val Ala
    210                 215                 220

Ala Leu Val Arg
225

<210>  52
<211>  372
<212>  PRT
<213>  nonomuraea sp.

<400>  52

Met Leu Ser Ser Ala Leu Glu Val Asp Ile Asp Glu Ala Ala Val Ala
1               5                   10                  15

Ala Asp Leu Arg Glu Leu Ala Ala Ala Leu Asp Arg Ser Gly Tyr Gly
            20                  25                  30

Glu Ile Leu Thr Cys Phe Leu Pro His Lys Ala Gln Ala His Ile Trp
        35                  40                  45

Ala Gln Thr Ala Ala Lys Ile Asp Gly Pro Leu Arg Thr Leu Met Glu
    50                  55                  60

Leu Phe Leu Leu Gly Arg Ala Val Pro Gln Asp Asp Leu Pro Ala Arg
65                  70                  75                  80

Val Gly Asp Leu Ile Pro Gly Leu Ala Ala Thr Gly Leu Val Gln Thr
                85                  90                  95

Gly Gln Gly Ala Val Trp Leu Pro Asn Leu Ile Leu Leu Arg Pro Met
            100                 105                 110

Gly Gln Trp Leu Trp Cys Gln Arg Pro His Pro Ser Pro Thr Met Tyr
        115                 120                 125

Phe Gly Asp Asp Ser Leu Ala Leu Val His Arg Met Val Thr His Arg
    130                 135                 140

Gly Gly His Thr Leu Asp Leu Cys Ala Gly Pro Gly Val Gln Ala Leu
145                 150                 155                 160

Thr Ala Ser Leu Arg Ser Asp Arg Val Thr Ala Val Glu Ile Asn Pro
                165                 170                 175

Val Ala Ala Ala Leu Cys Arg Thr Asn Ile Ala Met Asn Gly Leu Gly
            180                 185                 190

Asp Arg Met Glu Val Arg Leu Gly Asp Leu Tyr Gly Ala Val Pro Gly
        195                 200                 205

Glu Val Phe Asp Asp Ile Val Ser Asn Pro Pro Leu Leu Pro Val Pro
    210                 215                 220

Glu Asp Val Gln Phe Ala Phe Val Gly Asp Gly Gly Arg Asp Gly Phe
225                 230                 235                 240

Asp Ile Ser Trp Thr Ile Leu Asp Gly Leu Pro Glu His Leu Ser Asp
                245                 250                 255

Arg Gly Ala Cys Arg Ile Val Gly Cys Val Leu Ser Asp Gly Tyr Val
            260                 265                 270

Pro Val Val Met Asp Gly Leu Gly Lys Trp Ala Val Glu His Asp Phe
        275                 280                 285

Asp Val Leu Leu Thr Val Thr Ala His Val Glu Ala His Arg Asp Ser
    290                 295                 300

Ser Phe Leu Arg Ser Met Ser Leu Met Ser Ser Ala Ile Ser Gly Glu
305                 310                 315                 320

Pro Ala Asp Val Leu Gln Glu Arg Tyr Ala Ala Asp Tyr Ala Glu Leu
                325                 330                 335

Gly Gly Ser His Val Ala Phe Tyr Glu Leu Cys Ala Arg Arg Gly Gly
            340                 345                 350

Gly Thr Ala Arg Leu Ala Asp Val Ser Ala Thr Lys Arg Ser Ala Glu
        355                 360                 365

Val Trp Phe Val
    370

<210>  53
<211>  122
<212>  PRT
<213>  nonomuraea sp.

<400>  53

Val Tyr Val Val Ile Val Ala Phe Asp Leu Lys Glu Ser Ser Val Asp
1               5                   10                  15

Phe Ala Glu Leu Arg Ala Trp Val Arg Asp Arg Ala Ala Asp Asp Tyr
            20                  25                  30

Ser Arg Leu Pro Gly Met Arg Phe Lys Thr Trp Phe Ser Asp Glu Arg
        35                  40                  45

Lys Arg Leu Trp Gly Ala Val Tyr Leu Val Glu Ser Met Leu Ser Phe
    50                  55                  60

Asp Arg Asp Asn Leu Pro Leu Leu Pro Asp Gly Arg Thr Gly Pro Val
65                  70                  75                  80

Gly Thr Arg Pro Thr Ser Val Met Val Leu Glu Leu Glu Ala Phe Val
                85                  90                  95

Thr Gly Pro Asp Gly Leu Asp Gly Ile Glu Ala Leu Ala Arg Gln Gly
            100                 105                 110

Leu Ser Met Thr Gly Gly Gly His Asp His
        115                 120

<210>  54
<211>  416
<212>  PRT
<213>  nonomuraea sp.

<400>  54

Met Thr Thr Glu Ala Thr Thr Ala Val Thr Thr Arg Ala Asn Ala Gly
1               5                   10                  15

Ala Leu Ser Leu Pro Ala Arg Gly Thr Asp Arg Pro Leu Leu Leu Tyr
            20                  25                  30

Val Asn Ile Pro Phe Cys Asn Ser Lys Cys His Phe Cys Asp Trp Val
        35                  40                  45

Val Asp Val Pro Val Ser Asp Leu Arg Leu Ala Pro Ala Ala Pro Arg
    50                  55                  60

Arg Val Asp Tyr Leu Ala Ala Leu Cys Thr Gln Ile Arg Thr His Ala
65                  70                  75                  80

Pro Ala Leu Arg Ala Ala Gly Tyr Arg Gly Glu Val Met Tyr Trp Gly
                85                  90                  95

Gly Gly Thr Ala Thr Val Leu Thr Ala Ala Glu Ile Glu Gln Val His
            100                 105                 110

Ala Cys Leu Ala Ala Glu Phe Asp Leu Thr Ala Leu Ala Glu Ala Thr
        115                 120                 125

Ile Glu Gly Ser Pro Glu Ser Val Asp Pro Ala Lys Leu Arg Leu Leu
    130                 135                 140

Arg Asp Leu Gly Phe Asp Arg Val Ser Leu Gly Val Gln Ser Phe Asp
145                 150                 155                 160

Glu Thr Arg Leu Arg Arg Ile Gly Arg Ala His Ser Ala Gly Gln Ala
                165                 170                 175

Val Arg Ser Val Glu Asp Ala His Ala Ala Gly Phe Asp Asn Ile Asn
            180                 185                 190

Ile Asp Leu Ile Val Gly Phe Pro Asp Gln Ser Leu Ala Glu Val Glu
        195                 200                 205

Gln Thr Val Arg Arg Ala Leu Asp Leu Pro Val Asn His Phe Ser Val
    210                 215                 220

Tyr Ser Tyr Arg Ala Thr Glu Gly Thr Val Met Arg Arg Gln Ile Glu
225                 230                 235                 240

Arg Ser Gly Thr Ala Ile Glu Leu Glu His Gln Leu Gln Ser Tyr Arg
                245                 250                 255

Leu Ala Gly Glu Met Leu Ala Ala Ala Gly His Pro Glu Tyr Ala Val
            260                 265                 270

Ser Tyr Phe Gly Ala Pro Arg Cys Leu Ala Asp Glu Ala Tyr Tyr Arg
        275                 280                 285

Leu Ser Met Asp Trp Ile Gly Phe Gly Thr Gly Ala Asn Ser Leu Ile
    290                 295                 300

Asn Gln Arg Tyr Leu Leu Asn Gly Arg Gly Arg Met Arg Asp Phe Thr
305                 310                 315                 320

Ser Arg Pro Gly Glu Phe Glu Val Asp Leu Pro Ala Gly Val Pro Ser
                325                 330                 335

Leu Thr Val Gln Trp Leu Pro Arg Ala Leu Gly Thr Ala Glu Gly Ile
            340                 345                 350

Asp Ala Ala Thr Phe Gln Arg Arg Thr Gly Thr Ser Leu Arg Ala Ala
        355                 360                 365

Cys Glu Glu Pro Glu Leu Asn Ala Phe Leu Thr Arg Val Asn Glu Phe
    370                 375                 380

Gly Arg Leu Val Val Asp Arg Asp Gly Ile Arg Leu Ala Asp Glu Asn
385                 390                 395                 400

Arg Ser Asp Val Leu Ser Arg Thr Phe Ala Ala Met Gly Trp Val Ser
                405                 410                 415

<210>  55
<211>  339
<212>  PRT
<213>  nonomuraea sp.

<400>  55

Val Glu Ala Met Arg Thr Leu Leu Ala Ala Ala Asp Leu Val Thr Pro
1               5                   10                  15

Met Ala Leu Arg Val Ser Ala Thr Leu Arg Leu Ala Asp His Leu Ala
            20                  25                  30

Ala Gly Thr Thr Gly Ala Ala Glu Leu Ala Ala Ala Ala Gly Val Asp
        35                  40                  45

Glu Ala Ala Leu Thr Arg Leu Leu Arg Tyr Leu Val Ala Arg Gly Val
    50                  55                  60

Phe Thr Glu Pro Ala Pro Gly Glu Phe Gly Leu Asn Pro Ala Ala Glu
65                  70                  75                  80

Leu Leu Arg Asp Gly Arg Pro Asp Arg Leu Arg Asp Trp Leu Asp Leu
                85                  90                  95

Thr Gly Pro Ile Gly Arg Ala Asp Leu Ala Phe Gly Ser Leu Leu Asp
            100                 105                 110

Val Val Arg Thr Gly Lys Pro Gly Tyr Pro Met Ile His Gly Arg Gly
        115                 120                 125

Phe Trp Asp Asp Leu Ala Ala Glu Pro Ala Leu Ala Thr Ala Tyr Asp
    130                 135                 140

Thr Leu Met Gly Gly Lys Arg Gln Trp Ala Ala Thr Thr Leu Ala Ala
145                 150                 155                 160

Leu Asp Trp Ser Arg Ser Arg His Val Val Asp Val Gly Gly Gly Asn
                165                 170                 175

Gly Thr Leu Leu Ser Cys Leu Leu Ala Ala His Pro His Leu Arg Gly
            180                 185                 190

Thr Val Val Asp Arg Pro Thr Ser Ala Lys Ala Ala Gly Ala Val Leu
        195                 200                 205

Ala Ser Ala Gly Val Ala Asp Arg Gly Glu Phe Arg Ala Gly Asp Phe
    210                 215                 220

Phe Glu Pro Leu Pro Val His Gly Ala Asp Thr Tyr Leu Leu Ser Ser
225                 230                 235                 240

Ile Leu His Asp Trp Asp Asp Ala Ala Ala Thr Ala Ile Leu Arg Arg
                245                 250                 255

Cys Ala Glu Ala Ala Ala Pro Gly Gly Arg Val Leu Leu Cys Glu Leu
            260                 265                 270

Val Ala Met Ala Gly Pro Asp Arg Arg Thr Val Thr His Met Asp Leu
        275                 280                 285

Cys Met Leu Val Tyr Phe Gly Gly Arg Glu Arg Thr Ala Ala Asp Phe
    290                 295                 300

Ala Ala Leu Ala Gly Ala Ala Gly Leu Glu Pro Arg Ser Val Thr Pro
305                 310                 315                 320

Leu Pro Pro His Asp Trp Gly Asn Ala Leu Val Glu Tyr Ala Val Pro
                325                 330                 335

Ala Ala Arg

<210> 56
<211> 204
<212> PRT
<213> nonomuraea sp.

<400> 56

Met Ala Lys Val Ile Ser Thr Leu Phe Ile Ser Ala Asp Gly Val Ala
1               5                   10                  15

Glu Ile Asp Pro Asp Trp His Phe Pro Tyr Phe Asp Glu Asn Met Gly
            20                  25                  30

Arg Ala Val Gly Glu Asp Tyr Asp Thr Ala Asp Val Leu Leu Ile Gly
        35                  40                  45

Arg Glu Thr Phe Asp Ser Phe Ala Gly Ala Trp Pro Asp Arg Glu Ala
    50                  55                  60

Ala Gly Gly Asp Asp Ala Pro Phe Ala Lys Gln Leu Gly Asp Met Arg
65                  70                  75                  80

Lys Val Val Val Ser Arg Gln Pro Leu Glu Phe Ser Trp Arg Asn Cys
                85                  90                  95

Glu Leu Ile Thr Gly Asp Leu Leu Asp Ala Val Thr Ser Leu Lys Ala
            100                 105                 110

Asp Ala Gly Ile Arg Gly Ile Leu Ile Pro Gly Ser Ile Ser Val Val
        115                 120                 125

Gln Gln Leu Leu Ala Ala Gly Leu Val Asp Glu Leu Arg Leu Leu Val
    130                 135                 140

His Pro Val Ala Ala Arg Lys Gly Arg Arg Leu Phe Asp Asp Gly Asp
145                 150                 155                 160

Ala Pro Tyr His Leu Ser Val Thr Ala Thr Glu Thr Phe Pro Thr Gly
                165                 170                 175

Val Ile Arg Val Ile Tyr Ser Pro Thr Glu Ala Pro Thr Lys Val Gly
            180                 185                 190

Tyr Asp Glu Val Glu Ala Gln Val Ser Thr Gly Glu
        195                 200

<210>  57
<211>  403
<212>  PRT
<213>  nonomuraea sp.

<400>  57

Met Thr Lys Pro Leu Leu Ile Tyr Ala Asn Val Pro Phe Cys Asn Ser
1               5                   10                  15

Lys Cys His Phe Cys Asp Trp Val Val Gln Val Pro Val Arg Asp Leu
            20                  25                  30

Arg Leu Gly Glu Gln Ser Pro Gly Arg Val Ala Tyr Leu Glu Ala Ile
        35                  40                  45

Arg Thr Gln Ile Arg Val Gln Ala Pro Ala Leu Arg Glu His Tyr His
    50                  55                  60

Pro Asp Ile Val Tyr Trp Gly Gly Gly Thr Ala Ser Ile Leu Gly Pro
65                  70                  75                  80

His Glu Ile Glu Ser Leu Tyr Gly Thr Leu Ser Ala Glu Phe Asp Leu
                85                  90                  95

Ser Thr Val Arg Glu Ala Thr Ile Glu Gly Ser Pro Glu Ser Leu Asp
            100                 105                 110

Arg Asp Lys Leu Arg Leu Leu Arg Glu Leu Gly Phe Asn Arg Ile Ser
        115                 120                 125

Ile Gly Val Gln Ser Phe Asp Asp Ala Arg Leu Arg Arg Leu Gly Arg
    130                 135                 140

Ala His Ala Ala Asp Gln Ala Leu Thr Ala Val Gln Asp Ala His Glu
145                 150                 155                 160

Ala Gly Phe Thr Asn Ile Asn Ile Asp Leu Ile Val Gly Phe Pro Gly
                165                 170                 175

Gln Thr Gly Gln Glu Val Ala Asp Ser Val Arg Thr Ala Leu Thr Leu
            180                 185                 190

Pro Ile Asn His Phe Ser Ile Tyr Pro Tyr Arg Ala Ser Pro Gly Thr
        195                 200                 205

Val Leu Arg Lys Gln Val His Arg Gly Ala Gln Leu Asp Leu Asn Leu
    210                 215                 220

Gln Leu Gln Ala Tyr Gly Ile Ala Arg Glu Leu Leu Glu Ala Ala Gly
225                 230                 235                 240

Phe Pro Glu Tyr Ala Met Ser Tyr Phe Gly His Pro Arg Cys Gln Ser
                245                 250                 255

Asp Glu Ala Tyr Tyr Gln Leu Arg Met Asp Trp Ile Gly Phe Gly Ser
            260                 265                 270

Gly Ala Asn Ser Leu Ile Gly Arg Arg Tyr Leu Ser Tyr Glu Lys Gly
        275                 280                 285

Lys Leu Ala His Tyr Asn Thr Asn Pro Leu Ala Phe Asp Ile Asn Ala
    290                 295                 300

Pro Ala His Ser Pro Gln Leu Thr Leu His Phe Leu Ser Gln Ala Leu
305                 310                 315                 320

Thr Thr Ala Glu Gly Leu Asp Ala Arg Leu Tyr Gln Gln Arg Thr Gly
                325                 330                 335

Val Pro Leu Arg Thr Ala Cys Ser His Pro Glu Val Met Thr Tyr Leu
            340                 345                 350

Glu Arg Met Asn Asn His Gly Arg Leu Ile Ala Asp His Asn Gly Ile
        355                 360                 365

Arg Leu His Arg Asp Asp Ile Ala Gln Thr Phe Ile Ala Leu Asn Trp
    370                 375                 380

Ile Pro Thr Pro Asp Thr Thr Pro Asn Glu Val Ile Pro Leu Thr Pro
385                 390                 395                 400

Ser Pro Thr

<210> 58
<211> 387
<212> PRT
<213> nonomuraea sp.

<400> 58

Met Thr Ala Pro Val Arg Pro Pro Ser Val Thr Asp Gly Gly Thr Ser
1               5                   10                  15

Leu Phe Ala Trp Leu Arg Glu Met Arg Asp Thr His Pro Val Trp Arg
            20                  25                  30

Asp Ser Tyr Gly Met Tyr His Val Phe Arg Tyr Asp Asp Val Arg Ala
        35                  40                  45

Val Leu Ala Asp His Glu Arg Phe Ser Ser Asp Arg Thr Arg Leu Met
    50                  55                  60

Gly Arg Gln Pro Phe Gly Gln Gly Gly Ile Thr Met Ile Asp Pro Pro
65                  70                  75                  80

Glu His Arg His Gln Arg Arg Leu Ile Thr Ala Ala Phe Thr Pro Ser
                85                  90                  95

Ser Val Ala Ala Leu Glu Pro Arg Ile Ala Ala Ile Ala Asp Glu Leu
            100                 105                 110

Leu Asp Ala Leu Pro Gly Pro Asp Phe Asp Leu Val Glu Ser Leu Ala
        115                 120                 125

Tyr Pro Leu Pro Val Thr Val Val Ala Glu Leu Leu Gly Val Pro Pro
    130                 135                 140

Ser Asp Arg Asp Leu Phe Arg Thr Trp Ser Asp Arg Leu Met Ser Leu
145                 150                 155                 160

Gln Val Pro Asp Phe Ala Asp Pro Ser Leu Ala Gly Arg Val Ala Ala
                165                 170                 175

Ala Met Ala Glu Met Asn Asp Tyr Leu Arg Glu His Cys Ala Asp Arg
            180                 185                 190

Arg Thr Arg Pro Arg Asp Asp Leu Leu Thr Arg Leu Val His Ala Glu
        195                 200                 205

Ile Asp Gly Glu Arg Leu Asp Ala Glu Gln Val Val Asn Thr Ala Ser
    210                 215                 220

Leu Leu Leu Leu Ala Gly His Val Thr Thr Thr Val Leu Ile Gly Asn
225                 230                 235                 240

Thr Val Leu Cys Leu Ala Asp Ala Pro Glu Ala Ala His Arg Thr Arg

245                 250                 255

Ala Asp Met Ser Leu Ile Pro Gln Ala Leu Glu Glu Ser Met Arg Leu
            260                 265                 270

Arg Ser Pro Phe Met Gln Ala Gly Arg Val Thr Thr Gln Asp Val Gln
        275                 280                 285

Val Ala Gly Glu Thr Ile Pro Ala Asn Arg Phe Val Met Ala Trp Leu
    290                 295                 300

Leu Ser Ala Asn His Asp Glu Arg His Phe Ala Asp Pro Glu Arg Phe
305                 310                 315                 320

Asp Leu Asp Arg Gln Leu Thr Gly Gln Leu Ala Phe Gly His Gly Val
                325                 330                 335

His Phe Cys Leu Gly Ala Gln Leu Gly Arg Leu Glu Gly Arg Ile Ala
            340                 345                 350

Leu Glu Arg Leu Leu Ser Arg Phe Thr Glu Leu Arg Pro Glu Lys Arg
        355                 360                 365

Ser Phe Tyr Glu Ser Gln Ile Phe Gly Val Arg Glu Met His Val Arg
    370                 375                 380

Gly Ser Ser
385

<210>  59
<211>  406
<212>  PRT
<213>  nonomuraea sp.

<400>  59

Val Asn Glu Thr His Val Pro Pro Asp Pro Thr Met Thr Met Pro Ile
1               5                   10                  15

Asp Ser Arg Pro Arg Ile Lys Asp Gly Gly Gln Thr Leu Leu Lys Trp
            20                  25                  30

Leu Gly Thr Met Arg Asp Glu Gln Pro Val Trp Arg Asp Ala Phe Gly
        35                  40                  45

Ile Phe His Val Phe Arg His Ala Ala Val Gln Arg Val Met Ser Asp
    50                  55                  60

Tyr Ala Ala Phe Ser Ser Asp Ile Asn Arg Leu Arg Pro Gly Gly Asp
65                  70                  75                  80

Pro Phe Ser Ala Gly Ser Leu Met Leu Thr Asp Pro Pro Glu His Arg
                85                  90                  95

Lys Leu Arg Lys Leu Ile Ser Gln Ala Phe Thr Pro Lys Met Ser Ser
            100                 105                 110

Asp Met Val Pro Arg Ile Ala Glu Leu Thr Thr Ala Leu Leu Asp Glu
        115                 120                 125

Ile Asp Glu Asp Glu Phe Asp Leu Val Glu Lys Ile Ala His Pro Leu
    130                 135                 140

Pro Val Met Val Ile Ala Glu Leu Leu Gly Ile Pro Ile His Asp Arg
145                 150                 155                 160

Glu Leu Phe Arg Thr Trp Ala Asp Arg Leu Val Ala Leu His Val Glu
                165                 170                 175

Asp Pro Arg Asp Ile Glu Ile Gly Arg Met Val Gly Gln Ala Met Arg
            180                 185                 190

Glu Met Gly Glu Tyr Val Leu Thr His Val Arg Glu Arg Arg Thr Arg
        195                 200                 205

Pro Gln Asp Asp Leu Val Ser Arg Leu Val Ala Ala Glu Val Asp Gly
    210                 215                 220

Asp Arg Leu Thr Asp Ala Glu Ile Val Asn Ser Ala Cys Leu Leu Leu
225                 230                 235                 240

Leu Ala Gly Gln Ile Thr Ser Thr Met Ala Leu Gly Asn Ala Phe Leu
                245                 250                 255

Cys Phe Arg Asp Ala Pro Gly Val Glu Ser Ala Val Arg Ala Asp Arg
            260                 265                 270

Glu Leu Ile Ala Pro Ala Phe Glu Glu Val Leu Arg Leu Arg Pro Pro
        275                 280                 285

Leu Thr Gln Ala Ala Arg Leu Pro Thr Ala Asp Val Glu Ile Asp Gly
    290                 295                 300

Thr Pro Ile Pro Ala Gly Ser Met Val Ile Asn Trp Leu Leu Ser Ala
305                 310                 315                 320

Asn Tyr Asp Glu Arg Gln Phe Pro Asp Pro Tyr Arg Leu Asp Pro Gly
                325                 330                 335

Arg Gln Pro Asn Arg Gln Tyr Ala Phe Gly His Gly Ile His Phe Cys
            340                 345                 350

Leu Gly Ala Pro Leu Ala Arg Val Glu Gly Lys Val Ala Leu Glu Leu
        355                 360                 365

Val Phe Asp Arg Phe Glu Gln Val Glu Ile Asp Pro Asp Ala Glu Leu
    370                 375                 380

Ser Tyr Tyr Glu Asp Pro Met Phe Gly Val Lys Ser Leu Pro Val Arg
385                 390                 395                 400

Val Lys Arg Ala Pro Arg
                405

<210>  60
<211>  243
<212>  PRT
<213>  nonomuraea sp.

<400>  60

Met Ser Thr Val Ser Thr Arg Ser Thr Gly Ala Arg Thr Ser Asp Asp
1               5                   10                  15

Arg Val Arg Arg Thr Leu Asp Val Thr Arg Ser Leu Leu Leu Arg Ser
            20                  25                  30

Gly Tyr Lys Arg Thr Thr Met Asp Glu Ile Ala Arg Arg Ala Asp Ile
        35                  40                  45

Gly Lys Gly Thr Ile Tyr Leu Ser Trp Asp Thr Lys Asp Asp Leu Ile
    50                  55                  60

Arg Thr Leu Val Ile Gln Glu Ile Val Gly Val Cys Gln Asp Ile Ser
65                  70                  75                  80

Arg Ile Ala Val Leu Arg Pro Ala Val Ala Arg Leu Ser Glu Phe Ser
                85                  90                  95

Arg Glu Leu Phe Thr Leu Val Phe Lys Tyr Pro Leu Phe Arg Ala Leu
            100                 105                 110

Tyr Thr Tyr Asp Lys Glu Thr Leu Gly Arg Ala Cys Asp Asp Pro Gln
        115                 120                 125

Leu Gly Phe Gln Cys Tyr Arg Phe Thr Thr Phe Thr Pro Phe Arg Asp
    130                 135                 140

Tyr Leu Arg Met Leu His Glu Ser Gly Met Trp Asp Pro Ser His Gly
145                 150                 155                 160

Phe Ala Leu Asp Ala Leu Leu Ser Gly Phe Ile Lys Leu His Leu His
                165                 170                 175

Ala Glu Ile Ala Gly Ala Lys Pro Asp Leu Ala Ala His Ala Asp Ser
            180                 185                 190

Leu Ala Gly Leu Ile Arg Ser Ser Phe Glu Pro Ala Asp Gln Val Pro
        195                 200                 205

Ala Glu Glu Leu Thr Asp Pro Ala Arg Arg Thr Val Glu Ile Phe Asp
    210                 215                 220

Gly Ala Ala Ala Lys Tyr Arg Ala Lys Leu Ile Pro Gln Pro Leu Ala
225                 230                 235                 240

Ala Ser Val

<210>  61
<211>  444
<212>  PRT
<213>  nonomuraea sp.

<400>  61

Met Thr Thr Glu Pro Phe Pro Ala Pro Thr Gly Pro Pro Ala Ala Pro
1               5                   10                  15

Thr Gly Pro Ser Pro Ala Leu Ala Glu Pro Ser Ala Ala Ser Pro Gly
            20                  25                  30

Pro Leu Ser Val Arg Gln Gly Pro Ser Pro Val Thr Ala Glu Leu Arg
        35                  40                  45

Asp Leu Ala Ile Phe Arg Ala Asp Leu His Ala His Pro Glu Leu Ser
    50                  55                  60

Trp Gln Glu Ala Arg Thr Ala Asp Arg Leu Ala Gly Arg Leu Ala Ala
65                  70                  75                  80

Ala Gly Tyr Glu Val Thr Thr Gly Met Gly Gly His Gly Val Val Gly
                85                  90                  95

Arg Leu Cys Arg Gly Asp Gly Val Thr Val Met Leu Arg Ala Glu Leu
            100                 105                 110

Asp Ala Leu Pro Val Lys Glu Glu Thr Gly Leu Ser Tyr Ala Ser Thr
        115                 120                 125

Ala Thr Ala Thr Thr Leu Asp Gly Arg Thr Val Pro Val Ser His Ala
    130                 135                 140

Cys Gly His Asp Leu His Leu Ala Cys Leu Val Gly Ala Ala Arg Arg
145                 150                 155                 160

Leu Ala Ala Cys Asp Asp Trp Arg Gly Thr Val Leu Val Ile Gly Gln
                165                 170                 175

Pro Ala Glu Glu Thr Leu Glu Gly Ala Ala Ala Met Leu Ala Asp Gly
            180                 185                 190

Leu Tyr Glu Arg Phe Gly Val Pro Asp Val Ala Leu Ala Gln His Val
        195                 200                 205

Ser Pro Phe Pro Ala Gly Leu Ile Ala Tyr Pro Glu Pro Pro Thr Ala
    210                 215                 220

Ala Gly Ala Glu Leu Arg Val Val Val Thr Gly Asp Gly Gly His Val
225                 230                 235                 240

Gly Asp Ile Gly Arg Ala Gly His Val Asp Arg Ala Val Gly Arg Asn
                245                 250                 255

Pro Val Ala Ala Val Ala Ala Leu Val His Arg Leu Asp Gln Thr Ala
            260                 265                 270

Phe Asp Gln Ala Ile Val Thr Val Gly Thr Leu His Ala Gly Glu Arg
        275                 280                 285

Ala Asn Val Ile Pro Thr Leu Ala Glu Ala Gly Ile Thr Val Arg Ala
    290                 295                 300

Ala Thr Glu Glu Ala Val Thr Arg Ala Val Ala Arg Val Ala Arg Leu
305                 310                 315                 320

Ala Glu Glu Thr Ala Gly Ala Gly Val Ile Val Val Ser Arg Val Pro
                325                 330                 335

Pro Gly Val Asn Asp Pro Ala Ala Thr Ala Leu Val Arg Arg Ala His
            340                 345                 350

Glu Ala Ala Leu Gly Ala Val Val Thr Ala Pro Gly Gly Ser Ala Cys
        355                 360                 365

Glu Asp Phe Pro Leu Tyr Gly Val Pro Ser Val Tyr Trp Tyr Val Gly
    370                 375                 380

Ala Ala Pro Pro Ala Gly Leu Val Gly Arg Pro His Thr Gly Thr Phe
385                 390                 395                 400

Arg Pro Asp Pro Val Pro Thr Leu Arg Ala Gly Val Thr Ala Met Gln
                405                 410                 415

Thr Ala Ala Leu Ala Val Leu Ala Asn Ala Ser Gln Phe Ala Pro Pro
            420                 425                 430

Ser Arg Tyr His Gly Pro Gly Ala Val Ala Glu His
        435                 440

<210>  62
<211>  22848
<212>  DNA
<213>  nonomuraea sp.

<400> 62
   gtgacggcgc ggcgacgcga cgtcgaacgg gtggatcgga tcgttgacgc ggcagcggaa 60
   ctgctgcctg agcggggata tcggcgactg acggtcgagg aggtcgcaag ccgggcgggc 120
   gtctccaaaa gcagcgtcta cctgcactgg aacaccaagg acgaaatctt ctacgccgtg 180
   ctggaccgcg agttcaccgt gctggcgcag gcggccgtgg accgggtcag gcgcgatcca 240
   gccgaggtgc tggcgcaccg gacggcggcc aacctcttcc agatcgtcgc ctgccggcca 300
   ctgctacagg cgttactcgt cgacgaccgg acaacgctcg gatcgctgca acccgcgaaa 360
   tctgctgttt tccgggccag cctcgcgaat ctcaacgaac tgatgtatcg ctatcttcac 420
   gcgctacgca taaacggact tctctgcccc gaggtcgacc cgcgtattat gcggaaagcg 480
   atttctgaaa tgctgcgcgg aatgacgttc tccgcgggcg cgaaaccgtt ggccgagaca 540
   cggctggcca agttgtccca ggtcgtcgcg gtgaccgtgc agcgcgcctt cgaaccgggc 600
   gacgtgcccg aactcgaccg gatcaacgcc gccgcggcgg aggtgttcaa ggcgttcgac 660
   gagctcatgc cggccgagga gacgatgcgc gccgaacgcc cggtggcgct gtgaccgtgg 720
   tgcgcgcgca cgtccgccag gagccggccg cgctcgtcgt cggtcgcggc ctgctcgccg 780
   accggctcgc ggcggtgacc ggccggctag accccggagt ggtgctcgcg gtgtcggacg 840
   cgccggaccc cgccgtgctc gcggaggcca gggcgacggg ccggcccgtg ctgcccgtgc 900
   tcgccgagcc gggccgggta cgggtcgggc cgctggaacg gccgggcgag ccgggctgct 960
   ccgactgcct gagcctgcgc aggcaccgcg ccgccagccg gtcggccgaa cgggccgccg 1020
   cctggctccg gcacggcccg gacctggccg ccacgccctc gccgctgctc gcgccggtgg 1080
   cgctcgacct ggtggccgag ctggtcaccg cgctggtcac cgcccccgcg ccggcccacc 1140
   gtggcatcct gctggccgac ctgtccgacc tgagcgtgac accgcacgcc ttcctgcccg 1200
   atccgttctg cccgcgctgc ggcaccctgc ccgacgacga cccggcgcgg gcgcggatca 1260
   cgctcgtgcc gcgcccgaaa ccgtcgccgg accggcaccg ggtgtgggac gccgaaggcc 1320
   ggctcgaccg gctcaggcag atctacgtcg acgaccactg cggcctggtc cactcgctca 1380
   acccggccgc gctgggctcg ctcgccgtcg ccggtgcgcc gatccggctg cgcggcacca 1440
   ccgccttcga acccggattc ggccgctccc gcagctaccg ccggagctcc gtcatcgcgc 1500
   tgctcgaagc cctggaacgg tacggcgcga tcggtcccgg tggcaggcgc ggcaccgtgc 1560
   gcgccagcta cgcctccctc gggaacctgg cggtggatcc caggtcgctg gggttgcacc 1620
   cgcccgagca ctacgcgctg ccgggcttcc cctaccggcc gttcaccccg gaagcggtgt 1680
   gccgctggct gtgggggcac tcgttcgcca ccggcggccc ggtgctggtg cccgagcgca 1740
   acgtccacta cggcccgtcc gacgacgatc ggccgttctg ctacgagctc gccaatggct 1800
   gcgctctcgg ctcgtgcctg gaggaggccg tcttccacgg cattctggag gtgctggaac 1860
   gcgacgcgtt cctgctgacc tggtacaccc gcgcccgcgc gccgcggctc gacctcggca 1920
   ccgcccgcga ccccacgatc ccgctggtcg ccgccgcgat cacggcggag accggctatc 1980
   tcgtcgagtg ctacgacatc acgcccgacc acggggtgcc ctgcgtgtgg gcgctggccc 2040
   gccatccttc cgacggcgag ccggccacga tcagcgccgc ggcggccggc cccggcctgg 2100
   aacccgccgc cgccggcgcg ctggccgagc tcggaccgat ggtgccgacc gtgcgcgacc 2160
   acttcccaca gcacgccggc cgggcgcggg agctggccgc ggacggccgg cgggtgcgat 2220
   ccatgatcga ccactatctc gtgtacggcg tgcggtcggc ggcacgccgc ttgtcgttcc 2280
   tcaccgacgg cacggaggag gtgcccttcg cgcccgcacc ggacggcttc cggcacgacg 2340
   acctcaccgc cgacctgagg ttcctcctcg accggctggc ggacagcggg ctcgacgtgg 2400
   tcgtggtgga cctgaccacg ccggagcacc gcgcgggcgg gcttcgctgc gtgaaggtgc 2460
   tggtgcccgg tacggtgccg atgacgttcg gcgagcagaa ccgccgcacg tggggcctgc 2520
   cgaggctgct cgacccggcc gtggtgcggg ggcgcggcat gccggtacgc ggccacgctg 2580
   atctgaaccc cgacccccac cccttcccgt gagccgccac gcgccgcccg ccgtcgcccg 2640
   ggtgggcggg ctgccggccg ccgcgctgca cggggtggcc tgccccgagg cgacccggct 2700
   cgccggccgc gtggtccacc tcaccgagga gctgacccga cgcgccgcgg tgctgtccga 2760
   cgctctctac gaggtgatcg gcgccgccgg cgcgcacaag ccgatcctgg tggcgatccg 2820
   ccgcgacctg cacgggctgc gccggcccaa acgcgtcgag gtgctgcccg cgccgttggc 2880
   cgagccggtc cggacgtgga tctcgctgtg ggaagagcgc gcccgcgtcc acgccgtgct 2940
   gccggacgtg ctcgcccgcg aggagcgctt ggcctgggag gcgctgcggg agctcgccgc 3000
   gaccccggcg gtgcggcacg ggctcgcgca cgccagcccg gacctctccg ccgacctgga 3060
   gaaatggctc gccgatacgg ggtggcggcc gcgaccgagc acgctggcga gcctgctgcg 3120
   gtacgtgaag cgcgtggcgg ccaagacgag ccccttcagc acgttcacca ccgtccacga 3180
   ggtccgctgg gtggacggcg gcgcggcctg gcaggtgccg gacacggcgc cgaccgtggt 3240
   ggtggaggcc gacgtggggc tccggctgct ggtcgagtcc gtgctgccgc gcatgcccga 3300
   ggtggccgcc gcgcgggtgg tgcggctgtc gccggccgcg tacgcgtcgg gggaccggct 3360
   ggcgttcccg gaaccgggcg ggcggatgcg cgcggtggac cggacctccg cactggacac 3420
   cctggtcgag ctgctgcgcg ccgagcagcg gtgggacgcg gcgatcgcca agctcaccgg 3480
   tgccggcctg gcaggccacg gagcagagga cggtacgcgt gacagcgcga gcgacggaac 3540
   ggcggcggcc gagcaggtgc tgagcgggct ggtgcgcggc gggctggtgg aggccgtcgt 3600
   gccggtgccc ggccaggcgg cccgtccgtt cgcgcgcctc gccgactggg cggcgcccgc 3660
   tgcgcccgcc ggcccgctgc accgtatcca ggtggccttg gaccaggccg ggccgctcgg 3720
   cgacggcgac ccgttggcgt cggcctgcgc ccacgtggcc cgccgcctca ccgccgaact 3780
   ccccgccctg ggcctgcccg tcatgccggt gcccgacctg cgccgtcgtg tgctgcgcga 3840
   gtcggcgctc ggcgcgccgg tcgcctgcgc gctgcgggag tggcgacccg tgctggccga 3900
   cctccaacgg gtccgccgct ggctcgccgt gcacgacccg atgctgccgc ttcgcctcac 3960
   cctggccgac cgggtacgcg actggttcgg ccccggctcg gccgcgcccc tgctggacgt 4020
   gtacgccagg gtccggaccg ccgaaccggg cacgcccctg gatcccgact tcctcgaaca 4080
   ccccgacccg ctcgccggcg tcaccgaccc gcgcctgacc cgcctgcgcg agctgcgcgc 4140
   cgcgtcggtc gccgcgctca ccggggggcg ggcggaggag gcgctgagtg agccgccggc 4200
   ctgggtacgc gatccggggc cggtcacctg ctatgtccag ccgttccagg gggaggacgg 4260
   cctgcggctg gtcctcaacg ccgcccacgg cggtcacggc cggggcatca cccgctggac 4320
   caggctgctg ggcgccgagc cgccgcccgg cgagcccccg gctgccgagt ccccgcccgg 4380
   tgagtccccg cccggtgagc tcccgcccgg tgcgtgcctg gtcgccgagc tgccgggcac 4440
   gttcggccac agcctcaacc tgcacgcgcc cgccaccggc tgggagctga cctacccggg 4500
   cgcggtgaac caggccccgc cggagcgccg cgtcccgctg gccgagctcc aggtccggca 4560
   cgacgccggg cgaggcgtcg tcgagctgtg gtggccgcgc gccggccggc gcgtcgtgcc 4620
   ggtgcacgcc ggcatgatgt cggagacgct gctgccgccc ctggcccgcc tgctcgtcga 4680
   ggcgttcggc accacccacc tcacccatcc gaccctgccg cccgtggtgc gggccgccgg 4740
   tccgcgcatc gacctgggcc gcgtcacgct cgccagggcc cagtggacgg cccgccgcga 4800
   ggagattccg cggcggggcg gcgacgacgc cgcccacctc gtcgccgtcc acgcctggct 4860
   gcgcgccgcc gggatcccgc gccgctgctt cgtccgcgtc cgcgaaccgc aggtgcggag 4920
   ggaccggctc gccttcgaca agcggcacaa gccggtgttc gtcgacttcg ggagctggcc 4980
   gtcggtgctg gagttcgacc ggatcgtcac ccgggccacc ggggacctgg aactgaccga 5040
   ggcactgccc gacggcgagc gggcggtgga gctggcgatc gagataggag cccggtgacg 5100
   cggtacccgc tgccccgccc ccaccccctc ggcatccacc ccggctacgc ccggctgcgc 5160
   gagggctgcc ccgtcgcgcg ggtcgactcg ccgtacggtc cggcctggct gatcacccgg 5220
   tacgccgatg tggcggcggt actcaccgac gcccggtaca gccgcgcggc ggcgacggag 5280
   gacgacggcg gcatcctgct caacaccgac ccgcccgagc acgaccggct gcgcaggctc 5340
   gtggtggcgc acaccggcgc cgccgccgtc gagcggctcc ggccgcgggc cgaggagatc 5400
   gccaccaccc tggcgaaacg catcccggcg gaggccgagt tcgtaagcgc gttcgccgag 5460
   ccgtactcac atcgggtgct cgcgctgttc gtcgggcacc tggtcggcct gccggccggc 5520
   gagctgagcc cgatggccac cgtgctcacg ctggcgcggg tgcccgaccg ggagcgggcg 5580
   gcggcgttcg ccgaactgcg ccggcggctg cggagccagg tggacgacga gacgctcgcc 5640
   gtggtgctca acatcgtctt cggcggccac gcggcggtgg tggccgcgct gggctactgc 5700
   ctgctcgcgg cgctggccga gccgctgccg agcctgaccg gcgatgtccg gcggatcgcc 5760
   gagctggtcg aggagacgct gcgcctggct ccgcccggcg accggaccct gctgcgccgc 5820
   accctcgagc ccgtcgagct gggcgggcgg gagctgcccg cgggagcgct ggtgatcccg 5880
   tcgatcgcgg cggccggccg ggatccggac cggcccaccg gcgggccggc gggccggcac 5940
   ctggcgttcg gccgcggccc gcacgcgtgc ctcggcatgg cgctggcccg gatggagctc 6000
   cgggtcgcgc tccaggcgct ggccgagcac gtccccggac tgcggccggc ggtcagcccg 6060
   gccacgctca ggcgtacgtg cgaggaattg gcggtcagcc cgcttgcggg aatcccgatt 6120
   cgggtgtagt gggcagaaaa ccgaataaac gaatttggtc agcgggtgcc cattccttgc 6180
   cgcggatcac gcccgggtca tactctcggt tccagaaatg gtgcacgcat tcggtgtgag 6240
   aaaccgaggg agaatcgtgt ccgcagaaca ccattatcgt gacaggctac gcgccctgtc 6300
   cgcatatcta cgcaaggtcg acaatgagcc ggaggccgcc gccgagctgc gcgctgaccc 6360
   ggaaaaggcc ctccgggagg ccggcgtcga ccaggcgttc gaccgccctg aagcgttccg 6420
   cgcatttgtc ggaaaactgg cggtattaag cggagaagca tggctcgcga ccgttcactc 6480
   gatgattgaa ctgtgtgaga tcggtgcgga tccgcagcct cctgctgggc ccaacatctc 6540
   attccgcata tcttccgacg gcggggtgac ggccatcgcc aatcgcggtg agatcgccaa 6600
   gaaggtgcag cccaacccct ttcacgccgg ctccgccgcc gcgtcgccgg gcggccggct 6660
   gcgcatctat cccggctacg ccaccagcga actgtcggcc cgcctcggtg agcgctacct 6720
   gtccacgttc taccagcgca ccctgctcaa gcgcgtcgtg ctcgacccgg gcaccgtggt 6780
   cgaggacgcc gacgccggtg agggcatcac cgtcaaccgg tcacagtacc gaggcgtcgg 6840
   gttcgaactg cacaccaggg ccgacggcgc ggaccgcgag atcgtcgccg cgctcgtgcg 6900
   ctgacccccg gcacgagatc acgacatcac accccacgga ggcgggatgc tgagcagcgc 6960
   gctcgaagta gacatcgacg aggcggccgt cgccgccgac ctcagggaac tggccgcggc 7020
   gctcgaccgc agcggctacg gcgagatcct gacgtgcttc ctgccgcaca aggcgcaggc 7080
   gcacatctgg gcgcagaccg ccgcgaagat cgacggtccg ctacggacac tgatggagct 7140
   cttcctgctc ggccgggcag tgccgcagga cgacctgccg gcccgggtcg gcgacctcat 7200
   ccccgggctc gccgccacgg gcctggtgca gaccgggcag ggcgcggtgt ggctgcccaa 7260
   ccttatcctg ctgcggccga tgggccagtg gctgtggtgt cagcgcccgc acccgtcgcc 7320
   cacgatgtac ttcggcgacg actcgctcgc gctggtgcac cgcatggtga cccatcgcgg 7380
   cggccacacc ctcgacctgt gcgcggggcc gggcgtgcag gcgctgaccg cctcgctgcg 7440
   cagcgaccgc gtcaccgccg tcgagatcaa ccccgtcgcc gccgcgctgt gccgcaccaa 7500
   catcgccatg aacggcctgg gcgaccggat ggaggtccgc ctcggcgacc tctacggcgc 7560
   ggtgccgggc gaggtcttcg acgacatcgt gtccaacccg ccgctgctgc cggtgcccga 7620
   ggacgtccag ttcgccttcg tcggcgacgg tggccgcgac ggcttcgaca tctcctggac 7680
   catcctcgac ggcctgcccg aacacctgtc cgatcgcggt gcgtgccgga tcgtcggctg 7740
   cgtgctcagc gacggctacg tgcccgtcgt gatggacggg ctcggcaagt gggccgtcga 7800
   acacgacttc gacgtgctgc tcaccgtcac ggcgcacgta gaggcgcacc gcgactcgtc 7860
   gttcctgcgc agcatgtcgc tgatgtccag cgcgatctcg ggcgagccgg ccgacgtcct 7920
   gcaggagcgc tacgccgccg actacgccga gctgggcggc tcccacgtgg ccttctacga 7980
   gctgtgcgcg cggcgcggcg gcggcacggc acggctcgcc gacgtctcgg cgaccaagcg 8040
   ctccgccgag gtctggttcg tctgacgcca cccaccgacc ggacgggacc gcgatgccga 8100
   ccacactccc gaagaccctg cgcgggtcgg acggcgcgct cgccgagcag ccgctgctca 8160
   tctacgtgaa tgtgccgttc tgcaactcaa aatgtcactt ctgcgactgg gtgaccgagg 8220
   ttccgctggc ggatctgcgg ctcacgccgg actcgtcgcc acgccgccgc tacgtcgccg 8280
   cgctggtcaa gcagatcgag acgcacgcgc cgacgctggg cggactcggc taccggcccg 8340
   aggtcatgta ctggggcggc ggcacggcca gcatcctcac catcgaggag atcgaggcgg 8400
   tggctggggc gctggcggcg cggttcgacc tcggcggcct ggtcgaggcc accatcgagg 8460
   gcagcccgga gtccatggac cccggcaagc tgaagctgtt ccgtgcgatc gggttcaacc 8520
   ggatcagcat cggggtgcag gcgttcgacg acgcgcgcct gcgccgcatc ggccgggtgc 8580
   acagcgccga gcaggcggag cgcgcggtcc ggatggccgc cgacgccggc ttcgacaaca 8640
   tcaacatcga cctcatcgtc gggttccccg ggcagggcgc cgacgaggtg tcgcgcacga 8700
   tccagcacgc ggtgacgctg ccggtcaacc acttctcggt ctacccctac cggccgaccg 8760
   gcggcaccgt gatgcgccgg caggtggccc gcggcatggg ccggatcgac gtggaggagc 8820
   agctccggtc gtacgaccac gcccgcgacc tgctcgcccg gcacgggttc gaggagtacg 8880
   ccaccgccta cttcggcgct ccccgctgcg agtccgacga ggtctactac aagctcacga 8940
   tggactggat cgggttcggc tccgggccca actccctcat cgggacccgc ttcctgctga 9000
   acgaccgggg cgggctgcac cgcttcagca cggccccgca ccgcttcgac tccgacaccc 9060
   cggcctcggc cccccacctg acacgccact tcctggccca ggcgctgacc acggtcgacg 9120
   gcatggacgc gcgcaccttc cagctgcgca ccggccgttc gctgcgggcg gcgtgcgagg 9180
   agcccgcggt gcgccggatg ctcgaacaga tcaaccgccg cggcaggctg atcgtcgact 9240
   cacgcggcat ccggctgcac cgcgacgaca tggcctcggc ctacatcacc atgaacagcg 9300
   tcgatctgta cgccgcgacc gagcagcccg gcggatgacc gcggtcctgc gcgcctgctc 9360
   acacagtctt cgtgaacccg tgtgcccttc ccacgtgggg acgggcgtgc cggtcgaccg 9420
   ccgcgggctc gcgaaggaat gagggtcaca acggaaagga gatgagcgag ttggaatcca 9480
   agctgaacct tagcgacctg cccatggacg tcttcgagat ggccgacagc ggcatggagg 9540
   tcgaatccct caccgcaggc cacgggatgc ccgaggtagg cgcttcctgc aactgcgtgt 9600
   gcggcttctg ctgctcctgc agcccgtccg cgtagcagtc aaggagtgcc gccggcccag 9660
   gccggcggca ctccgcccgg ggtgaggaga cagatcatgg aggtgggccg cgaaatcgtg 9720
   gtccgcgtgg cggggctgcc cgccgccgtg ctcgccgacc tgcgcctgcc gcacaccgcc 9780
   gagctcgtca cgcacctgtc cgctgagcgg cggcggctcg ccgccgaggc ggctgcgctg 9840
   tccggcgagc tgttcgacct gatcggcgcc gccggctccg cccgcgccgc cctggtgggc 9900
   ctgcggcgcg cgctcgcccc cgggcaccgc cccccgtcgc cgcgactgat cgggctgtgt 9960
   cccctgcccg ccccgctggc cgagcgggtc accgcctggg tgcgggcccg gtacgactgg 10020
   gaggaccggc gccgcgacct tgccggcacg ctggacaagg agcgcgccga cgcgctcgac 10080
   cgcgtacgcg ccgcctgcgt cgccccggcg ttccggcggg gtctggcgct gtccggtggg 10140
   gagctgacca gcacgctgga gcgctggctc gccgaccccg gccgtgcgcc ccggcagggg 10200
   aaggtgctcc gcctggtcaa gtacctgacc cgagctgcgg ccaagaccag cccgtacggc 10260
   tccttcatgg tcagcgcgct ggcggggcgg cccggcgacg accctcccat cccggagctg 10320
   ctcaccgtgg ccgagccgcc gggggcgttc ctcgacgccg tcggcgacgc gctgctcgcc 10380
   gacccggcac tggccggcca ggtgccgctg cgccccaacc ccagcctgac gtggaccgcc 10440
   caaggactgc ttttcgtccg gaccgtccgc gcggcggcgg gagagcaggc cgggccgaag 10500
   gaagagatcg ccaccgccgg gcgcgccgcc gcgctggagc tgtgcctgcg gcacgccgag 10560
   tcccggccca ccgcgccgca gctggcggag ctgctggccg aggcgggggc ggatccgggc 10620
   gaggcggccg cgttcgtcga ccggctggtg gcggcgcagc tcctgctgcc gtgtccgccg 10680
   gtgcacgacg acgatcccga cccgttcggt gcctgggccc gccaggtgtg cgccccggag 10740
   ctgcgcgagc tggcggccgc gtcgcggccc gtggccgccg ccgtggactc gcccggccag 10800
   cgccgcgcca ggatcgccga agccgccgcc gccgtggcgg accggctcgg catcgacccg 10860
   cccgccgatc cggcgcacga gcacgacgtc agcaccggcc gtccggcgcc gccaccgctg 10920
   cccgcgggcg tgctcgccga cctggacgcg gtccggcgct ggctcgccgt gttcgactgg 10980
   aaggtgcccg tccgggtggg ggtcggcgcg ttctgccgcg aacgtttcgg ctcagggtcc 11040
   cgcacgccct tcctggaggc gtgccgggcg gcgacggcgg cgctgccgca cctgttcggc 11100
   cccgccgcga tgccgtggtt cctcgacctg accggtgacg gtcgcctgcg tgagctggac 11160
   cgtctccgga cgcgcgcccg cgatctcgcc cgctccggcg tcctcgaccg gcggcgggtg 11220
   ctggctgaca cggccgactg gcccgcctgg ctgacctggc ccgcctccac tggtttctac 11280
   ctgcagaccc tgcccggtgg ggtggtgctg aacgcggtgc atgccgggca cggccgcgcg 11340
   gcgggtcgcg tccaccacct cctcgcccgg gcgggcgccg cgccgccgcg cccgccccgc 11400
   tccggcctgc cgcgtgccga gttcggcggc aggttcggct ccgcgctcaa cacccgcacg 11460
   ccgagcacgc ggtacgagat cgaccacccc ggcgcgacgt ccggccgcga cccccggcac 11520
   cgtgtcccgc tcggcacgct catggtggtg cacgacccgg acaccgacct ggtccacctg 11580
   cacagcgacc ggctcggccg cgtcgaaccg gtgcacctcg gcatgatggg cgagctgggg 11640
   ctgcccgccg tggccggttt cctcgaacgc gccttcgcgc ccacctacct gttccacccg 11700
   agcgtgccgc cgttcatctc gctgcgtgac ctggccggcg cctcctcggc gcggcgcttc 11760
   cccagggtga gtgtcggcga cgtggtcgtg cagcgcgccc gctggacggt gcccgccggc 11820
   ctggtgcccg cgcgtaccgg ggcggacggt gaccacctgc tcgcgctggc cgagtggcgg 11880
   cgcgagcacg gcatccccga gcggtgtttc gtgcgcggct ggaagccggg cgcagcgctg 11940
   ggcaaggcgc gcaagccgct ctacgtcgac ttcgcctcct ggcatctggt cgccctgttc 12000
   gaacgggagg cgcgaaccaa cgccgccctg gtcatcgacg aggccctgcc ggacccgctg 12060
   gccgacggcg cccccgcgca cgtcaccgag taccacgtcg agatcggcga ccgggagaga 12120
   gagcaatgac cgaacggccc ggctggttcg cggcgcatgt gcactaccgc ggcgacctcg 12180
   acgcgctgct gcgcgaagcg gtcgcgccgc tggtgcgcgc gcttgccgcc gacttcttct 12240
   tcctgcgcta ctgggacggg ggcagccacc tgcggctgcg gctgcgcggc ggggaccagg 12300
   tcgcggtcgc ccggcacctg gacgcctacc ttgccgccca ccccgcgccg gagaccacct 12360
   cccaggagga gtacgcccgc gtcgccccgg tgctggccgc ccgcgagggc atgacgggcc 12420
   acctgacgac gctgcggccg aacaacaccg tggagttcgc ggcgtaccgg ccggagaccg 12480
   cgaagtacgg caccggcgac gcgctacgcg ccgtcgagcg gcatttcgtc gagtccagcc 12540
   ggtacgcgct tgacgtcctt gaccgccgcc cgaccggcaa ccaacgcgag ctcgcagtgc 12600
   tcggcgtcct gctgctcgcc tggtacgccg cccgcccact cgatgagccg cctggtgagc 12660
   cggtcgatga cgagctgatc ggcgcggtcg aggcgctgtg ccggggctgg cgcggcggcc 12720
   gggacctgcc cgaggagctc gtggcggagg agtacggccg ggtgcgggag cgtgtggccg 12780
   ggctggccgg cgcgctgcgt gacctcgttc cggacccgga cgcgcccggc agcagcatgc 12840
   gcgcgtgggc ggcgacgttc gaccggctcg ccgccgcgct gcccccgccc gaccggctgc 12900
   gggtgctcga cacgtgcgcg catctggcgg ccaaccggct gggcgtgtcc atggcggccg 12960
   aggtgcggct gcggctgctc gccgcgcggg ccctgcgcga ggtcgcgccc gtggggaggc 13020
   gatgatgagc tggcgacgcg tcgacgtggc ctaccacgat cccgaccttg acgggctgat 13080
   cctggccacc cggccgctgc tggccgggac gcccggccgc ggctggttcc agcggcactg 13140
   ggtccgcggt ccgcacctgg agctgtggtt cgacgccgcg cagccgtcgt gggagcggat 13200
   ccgcgacgtg ctggagccct ggctgcgcgt caacccgtcg cgcgcccgga tcgaccgaga 13260
   ccggctgctg gcgcagcacc ggcacctcgc cgccgccgaa cggatcgacg agccgctgct 13320
   gccgttctac gccgacaaca cgctgcaccg ggccgctccg cgctcccgcg cacacgtgct 13380
   cggtggtccg gcggccgagg agctgttcca cgacttccac accaccgcca gcgccgtggc 13440
   ttacgacgag ctcgacgccg tgcgggcggg agagtcgcgg ctggtgatgg cactggacct 13500
   gatggtcgcc gccgcgcacg cgcacgccga gggcggcgtc cgcggcgggt tcgtgtcgtt 13560
   ccgctcgcac gcggaggcgt tcctggcgag cgcgcccggc ctgcgcgagc gctgggacgc 13620
   cgagtacgcc gcccgggcgg gggcgctgcg agcgcggatc accgcggtgg tcgccggaat 13680
   cccgcgcggc cgggcgtggg ccgggctgct cgaccggttc gcggaccgcg gcgacgagct 13740
   gatcgcctcc ggcgcgctgc tggtcgagcc cgccgggccg gacgcggtcg cccggcccga 13800
   caccgccttc caccgggcgc tgcgcggcaa ccggacctgg cacgaggagg tgctgcgctc 13860
   agcaccgttc cgccgctacc ggctgctgct gaacctgacc tacctgcagc tttcccggct 13920
   cggggtgaac gccgtgcagc gggcgctgct gtgccacttc gccgcgtcgg cggtcgagca 13980
   ggagtacggg gtgtccgcca tcgagatcgc catgggaggc gcgtgatgtc agtacaggtc 14040
   gagcccgcag tgacgcaccg gtgggcactg cggcccggcg tctaccgggc caccgcgcca 14100
   ggcggcgatc tcatgctcgc cgcctggccg cacgccacgc cgctcggccg ggccaccccc 14160
   gagctgctgg ccctgctgga cggcctggcc gccggatcgc tgccgctcga cgagcccggc 14220
   gacctcgcgc ccgccgtcga gctgctgcgc gcgggtggct ggctgcgcaa gacggtggcg 14280
   tacgcgggac gcgacctcta caccatcacc cccgtcgccg cccccaccga cgcgccgcca 14340
   ccgccgcggg acgaaccggc gctgtcgcgg ttcgcgctgc tgcgcaacac cgcggacggg 14400
   ctggtgctgg agatgcccgg ctcctggtgc gacgtccacg tgcacgaccc ggcggtggcc 14460
   gcgctgctcg ccgacccgtc cggcgacggc ggcctgcccg ccgaggccgc ggccgcgctg 14520
   cgcgccgacc tggccgccgc cggcatgctg atggagcggg aggagccgtt cgagcggcgg 14580
   cagtggagca cccacgagct gtggttccat gaacgcagcc ggctcggcaa ccggggctgg 14640
   ttcggcgcgc acttcggcgg cacgttctgg gcccgcggcc ggcacgaccc gccgcccgcg 14700
   cggccgtccc cctaccccgg cccggccgtc gagctggccc ggccggacct cgcggcgctg 14760
   cggcgcaccg accccacgct caccgccgtg ctggaggacc gcgaaagcgt ccgcgaccac 14820
   gacgacgacg atccgatcag cgctgagcag ctcggcgagc tgctctaccg gtgcgcgcgg 14880
   gtgcggctgc tgcgtcacct ggaaggcttc gagtacgccg gcaagccgta ccccgccggt 14940
   ggctcggcgt acgagctaga ggtgtacccg gtcgtgcggc tggcccgcgg cctcgacccc 15000
   ggcatgtacc actacgacgc gcacgaccat cggctgcgcc aggtccggcc cgccggccac 15060
   ccggcggtgc gcaggatgct gaaggtcgcc acggagtcgt ccgtcgccaa ggcgccgccc 15120
   caggtgctgc tggtgatcag cgcgagagtc ggccggatcc tgtggaagta cgaggcgatg 15180
   gggtacgcgc tcatgctgaa gcacgtcggc gtgctgcaac agacgctgta cgcggtcgcc 15240
   accgccatgg acctggcccc gtgcgcgctc ggcagcgggg acgatctcgc cttcaccgcc 15300
   gccaccggcc gggaccggct ggtggagtgc gcggtcggcg agttcatggt cggcagcagg 15360
   cggaaggagc ccgcggcatg ggagctctga acctcctcct gcgccccgac gcgcactacg 15420
   ccgaggtcga cggcggcgtc tacgtgctga gccaccaggg cgagaccttc atctcgggca 15480
   cctcggtgca ccggtggctc gaccgcctcg cgccgctgct cgacggcacc cgcacgctcg 15540
   accagctcac cgccggcctg cccgccgacc gggccggctt cgtggccaga cttgtcggca 15600
   cgctcaccga acgcggcctg gtgcgcgagg tcggcccggc ggtgtcggac acgctcaccg 15660
   acgccgaacg cggcgagcac cgtgccctgc tggcctacct gggctacttc cacgactcgc 15720
   ccggccgcgt cctcgaggac gtgcgcgaca ccccgaccgc gatcgtgggg tccggtccgc 15780
   tggccgccgc ggtggtccgc gcctgcgccg ccgccgggct gcgccgggtg gagatcggcg 15840
   aggaggcggg cgcggcccgg gtggtcgtcc acgtggccga gcgcgccgag cccgaacggg 15900
   cggcgcggct ggaacggcgg tgcgccgccg agggggtgct gctcgcacag gtcatgccgg 15960
   ggccggacgg catctggtgg cagcccgcgg cccgcggcgg ctgctgggcg agcgcgtggc 16020
   gccgccaccg ggcgctcgcg ggcgtggatc ggacgggcac gccgctcgat ccggtggcgg 16080
   tcgcggtggt ggccggccag gtggcacacg acgtcttccg cgtcctcgcc ggactgcgcg 16140
   aggaggcgcc gccccggttg gtggtgctcg atccgcggac gctggcgagc accgcgcacc 16200
   cggtggtggc gcacccgttc gagctgcccg ccgcgccgct ggacgaggcg gcgttcctcg 16260
   accgggtcgc cgcgctgcgg gcagcgcccg cgctgagcga ggcggagttc tcccggcggg 16320
   ccaaggggct catggactcg acagtggggc tgttcgccga gatcgacgag ggcgacctcg 16380
   cgcagttgcc gctgcacgtc acggcgacca cggtggccga cccgtgcggg ctgatcggct 16440
   ccgcgccgcg gcccgtggtc accggcgcgg ggctcacgtt cgaggaggcc cgctaccgcg 16500
   cggcgcgcgc cgcgctcgcc ctggccggca ctctcgcgct cgaccggcgg aggctggtcg 16560
   acggccgggt gtgggcgtac gaccttgcgg acggggccgc ccggctggtt ccggccgacg 16620
   aggtcttcgc aagcacggcg ggcgggagca cggcgggcgt gccgctcggc gcggcggcag 16680
   cctactcgtg ggccgaggcg gtcgcggagg gcctggtcgc gcaggccgcc gcgctcactc 16740
   tcgccggcgc cgggcacgcg ggcgagccgt atggacgggc ggacctcgcc ggggcgcctg 16800
   cgtatctcct cgccatggtg cgggcgctcg gcgaggagct gaccgtgcac gacgtcaccg 16860
   ggccgctggg cgtgccggtc gtggtgggga cgctgtccgg cggggcgacc gcctgcggcg 16920
   cgggcatgac cacggacgcc gccgtcaccg cgtgcctgcg cgacctgctg ctgctgcgcc 16980
   aggccgagat caacgaccag cccgtctacg ccccaccatc cggcaggccc ttgcccgggc 17040
   atctacgcgg gaaccacccg gcgcccgccg tgcccggcac ggacgccgac cggctggcgg 17100
   cccgcctggc cgagcggggg caccgccctc tggcggtgcc gctggatcat gaccaggccg 17160
   tgcacgcggt cctgcccttc gccgtccggg tggtgtgccg atgatccgcc tgcccgacca 17220
   cggcgagctt gccgtgccga tcggcgacgg gccgatgctg ccgctgcgca ccgaactcgg 17280
   ccgggtggtc atcggcccgc tggagcggcc cggcgtgccc ggatgccggg cctgcgtgcg 17340
   catccgcgcg gcgcgggtcc agcccgacgc ccgcaaggcc gaggccgtac gcgcccggca 17400
   cgcccccgcg cccagccagt ggctcacccc gctcgccacc gacctggtgc gcgtcctggt 17460
   cgcgagcgag agcgctgccc tggcagccga caccgacccc cgcaccgcgg gtgccgtgct 17520
   ggagatcgac ctcgccacgc tggaaatcac caggcaccgg ttcctgcccg acccgctctg 17580
   cccgcactgc ggcggtctgc cgccggacgg cccggccgag ctgaccctgc ggccccgccg 17640
   caagctcggc ggcagtcccc ggaccaggcc ggtcgagctg aacgcgctgc tggagacgta 17700
   cgtggacggc cgggtcggga tgatccggcc gctgaagtcg ggcgtgcagg gcggactgac 17760
   ggtggccagc gccatgctgc cgatccgcgc gggccacggc ctggaaccgg gggtcggccg 17820
   tacccgcagc tacaccgcga gcaggctggt cgccgtgctg gaggcgctgg agcgctatgg 17880
   cggcgtcagc cccggcggcc gccgcaccag cgttaccgcc gcctaccgcg acatctccgg 17940
   ccacgccgtc catcccgaca ccttcggcac acaccccgag gagaactacg acaggcccgg 18000
   cttcgccttc cggcgcttca ccgaggacac cgtgtgccgg tgggtgtggg gctactcctt 18060
   cgccaaggcc cagcccgtgc tggtgccgga gaaccaggtc tactactacg ccagaaacct 18120
   gccggacggc gagcagccgt tcgtcttcga ggtctccaac ggctgcgcgc tcggctcgtg 18180
   cctggaggag gcgatcctgc acggcctgct cgaggtggtc gagcgggacg cgttcctgct 18240
   cacctggcac gcccggcggg aggttccggt gctgaacccc gcgctggcga gcgacccggt 18300
   cctgcccatg caggccgccg cgatcaccgc cgagaccggg caccgggtgc tgtgcttcga 18360
   caccaccgcc gagcacggca tccccagcgt gtgggcgatg gccgtggacg tcgaacaccg 18420
   gtcggaccgg cccgccaccg cccacgccgc cggcgcggcg ctcacgctgg agcaggccgc 18480
   catgaacgcg ctgagcgagc tcggcccgct gctggccgac gtgatccgcc gctaccccga 18540
   ggagcgggaa cgggccgagg ccatggtccg cgaccccgag caggtcgcca ccatgcacga 18600
   ccactccctg ctctacgcgg ttcccgccgc ggccgaacgg ctgtcgttcc tcaccggccg 18660
   cgccgacggc ccgcggaccg gcttcggctc ggcccggttc accggcgacg acctcaccgc 18720
   cgatctgcgg gccatgatcg acgcggtgat cgacgccggg atggacgtgg tggtcgtcga 18780
   ccagaccacg ccggagcacc tggcgggcgg gttccgctgc gtgaaggtgc tggtgcccgg 18840
   cgcgctgccg atgacgttcg gccaccagta ccggcggctg gacaacctgc cgcggctggc 18900
   ggccgcgcgt accaccgacc cgcacccgtt cccatgagcg ggcgcccgga cagcggaaag 18960
   gagccgtcgt gtacgtggtg atcgtcgcgt tcgacctgaa ggagtcctcc gtcgacttcg 19020
   ccgaactgcg cgcctgggtg cgggaccggg cggcggacga ctactcccgg ctgccgggga 19080
   tgcgcttcaa gacgtggttc tccgacgagc gaaaacggct ctggggcgcg gtgtacctgg 19140
   tggagtcgat gttgtcgttc gaccgggaca acctcccgct gctgcccgac ggcaggaccg 19200
   ggccggtcgg cacccggccg acgtcggtca tggtgctgga gttggaggcg ttcgtcaccg 19260
   ggccggacgg cctggacggg atcgaggcgc tcgcccggca agggctgagc atgacgggag 19320
   gcggccatga ccactgaggc gacgactgcg gtgaccactc gtgcgaacgc cggagcgctg 19380
   tccctgccgg cgcgtggcac ggaccggccg ctgctgctct acgtcaacat cccgttctgc 19440
   aactccaaat gccacttctg cgactgggtg gtggacgtgc ccgtctccga cctgcggctc 19500
   gcccccgcgg cgccgcgccg ggtcgactac ctggccgcgc tgtgcaccca gatccgcacg 19560
   cacgcccccg ccttgcgggc cgccggttac cgcggtgagg tcatgtactg gggcggcggc 19620
   accgccaccg tcctcaccgc ggcggagatc gagcaggtcc acgcctgcct cgcggccgag 19680
   ttcgacctga cggcgctggc cgaggcgacg atcgagggca gccccgagtc ggtggacccg 19740
   gccaagctga ggctgctgcg ggacctcggc ttcgaccggg tgagtctcgg cgtgcagtcg 19800
   ttcgacgaga ccaggctgcg ccgcatcggc cgcgcccact ccgcgggcca ggccgtgcgc 19860
   agcgtcgagg acgcgcacgc ggcgggcttc gacaacatca acatcgacct gatcgtgggc 19920
   ttccccgacc agtcgctcgc cgaggtggag cagaccgtgc ggcgcgccct cgacctgccg 19980
   gtcaaccact tctcggttta ctcctaccga gcgaccgagg gcacggtgat gcgcaggcag 20040
   atcgaacgca gcggcacggc catcgagctg gagcatcagc tccagtcgta ccggctggcg 20100
   ggcgagatgc tcgcggcggc gggacatccc gagtacgccg tctcctactt cggtgcgccg 20160
   cgctgcctcg ccgacgaggc gtactaccgg ctcagcatgg actggatcgg cttcggcacg 20220
   ggcgccaact cgctgatcaa ccagcgctac ctgctcaacg gccgcggccg gatgcgtgac 20280
   ttcaccagca ggccgggcga gttcgaggtg gacctgccgg ccggagtgcc gtcactcacc 20340
   gtgcagtggc tgcccagggc gctgggcacc gccgagggca tcgacgcggc gaccttccag 20400
   cgtcgcaccg gcacgtccct gcgtgcggcc tgcgaggaac ccgagctgaa cgcgttcctc 20460
   acccgggtga acgagttcgg ccggctcgtc gtggaccgcg acggcatccg cctggccgac 20520
   gagaaccgct cggacgtgct gtcgcgcacg ttcgccgcga tgggctgggt gtcctgacgt 20580
   ggaggcgatg cgcacgctgc tcgcggccgc cgacctggtc acgccgatgg cgctgcgggt 20640
   gtcggcgacg ctgcggctcg ccgaccacct ggccgccggc acgaccggcg ccgccgagct 20700
   cgccgcggcc gccggggtcg acgaggcggc gttgacacgg ctgctgcgct acctggtcgc 20760
   gcgcggcgtc ttcaccgagc ccgcgcccgg tgagttcggc ctcaaccccg ccgccgaact 20820
   gctgcgcgac ggccgccccg accggctgcg cgactggctc gacctcaccg ggccgatcgg 20880
   ccgcgccgac ctggcgttcg ggtcgctgct ggacgtcgta cgcaccggca agcccggtta 20940
   cccgatgatc cacggccggg gcttctggga cgacctcgcc gccgagccgg cgctcgccac 21000
   cgcgtacgac accctgatgg gcggcaagcg ccagtgggcc gcgaccacgc tcgccgccct 21060
   ggactggagc cgctcccggc acgtcgtgga tgtcggcgga ggcaacggca cgctgctgtc 21120
   gtgcctgctg gccgcgcacc cgcacctgcg cggcaccgtc gtggaccggc ccacctccgc 21180
   gaaggccgcc ggagcagtac tggcctcggc cggggtggcg gaccgtggcg agtttcgcgc 21240
   gggcgacttc ttcgagccgc tgccggtgca cggcgccgac acatacctgc tcagctcgat 21300
   cctgcacgac tgggacgacg ccgcggctac ggcgatcctg cgccgctgcg cggaggccgc 21360
   cgcgccgggc gggcgggtgc tgctctgcga gctggtggcc atggccgggc cggaccggcg 21420
   gacggtgacg cacatggatc tgtgcatgct cgtgtacttc ggcggccggg agcgcaccgc 21480
   cgccgacttc gccgcgctgg ccggggcggc cgggctggag ccgcgctcgg tgacgccgct 21540
   cccgccccac gactggggca acgccctcgt ggagtacgcg gtccccgccg cccgctgacc 21600
   ggaaaagacc cctgccgacg gcgacggtca gcgcggcgcg gctccgttga tcgcgcccgc 21660
   ctgggccgca cgatgagctg gagcgctggt cccactcgac cgcaatcgcc gtggaggtgt 21720
   gggcacttcc cggcgagccc tgtccggcga cgtcgcggag ctcgtccatg ccacgatccc 21780
   cgacgtactc gacggctcgg gacgggacgg atcggtgcgg tgcgcgggca tcgcgctcac 21840
   cgcgggcgcc cgcgggctgc gcctgaccac cagccacgac ggctccggct cgccgcgctg 21900
   gggttcaaac tcactaccca gggccggtac gggatgagtt tcgtccgctt ctgtgaggaa 21960
   cgcggcgctg accgggtggc ggcgcgccgg ttcggcctac atcaaggagc tgatgcccga 22020
   catctggatg gcaccatcga gccggggcaa atgttcgacg ccacgaccga tgcgatgtcc 22080
   cgcgaccggc cgtcgcggtg aggcgtgccg atgcatcacg acgagctcgg aataccgagt 22140
   ctgcgtcggg gtggatttgt ggctggtcga tgagtttggg atgctgcgca ggtctgcctg 22200
   ccgataccac ctgactggaa ggggatcacc gcgatggcga aggtcatctc tacgttgttc 22260
   atctctgctg acggtgtggc cgagatcgac cccgattggc acttcccgta cttcgacgag 22320
   aacatgggcc gcgccgtcgg cgaggactat gacaccgctg acgtgctgtt gatcggtcgc 22380
   gagaccttcg acagcttcgc cggagcgtgg cccgaccgcg aggcggcggg tggggacgac 22440
   gcaccgttcg ccaagcaact cggggacatg cgcaaggtgg tcgtctcccg ccagccgctg 22500
   gagttctcct ggcgcaactg cgaactgatc acgggcgatc tcctcgatgc cgtcacctcg 22560
   ctcaaggccg atgccggcat caggggcatc ctcatccccg gttcgatctc cgtggtgcag 22620
   cagttgctcg ccgcagggct ggtcgacgag ttgcgcctgc tggtgcaccc ggtggcggcg 22680
   cgtaagggcc gcaggctgtt cgacgacggc gacgcgccgt accacctgag cgtgacggcg 22740
   acggagacgt ttccgacggg cgtgatccgc gtgatctact cgccgaccga ggcacccacc 22800
   aaagtcggct acgacgaggt cgaggcccag gtgtccaccg gggaatag 22848

<210> 63
<211> 15586
<212> DNA
<213> nonomuraea sp.

<400> 63
ctaggtgggt gagggggtta gggggattac ctcgttgggg gtggtgtcgg gggtggggat 60

ccagttgagg gcgatgaagg tctgggcgat gtcgtcgcgg tggaggcgga tgccgttgtg 120

gtcggcgatg agccggccgt ggttgttcat gcgttccagg tacgtcatga cctccgggtg 180

agagcaggcc gtgcggaggg ggacgccggt gcgttgctgg tagaggcggg cgtccaggcc 240

ctcggcggtt gtcagtgcct gggacaggaa gtggagggtg agctgtgggg agtgggccgg 300

ggcgttgatg tcgaaggcca gcgggttggt gttgtagtgg gccagcttgc ccttctcgta 360

gctcaggtag cggcggccga tcagggagtt ggcgcccgag ccgaagccga tccagtccat 420

gcggagctgg tagtacgcct cgtccgactg gcagcggggg tggccgaagt aggacatggc 480

gtattcgggg aagccggctg cttccagcag ttccctggcg atgccgtacg cctggagctg 540

gaggttgagg tcgagctggg cgccccggtg gacctgcttg cgcagcaccg tgccggggct 600

ggcgcggtag gggtagatcg agaagtggtt gatgggcagg gtcagggccg tgcggaccga 660

gtcggcgacc tcctggccgg tctgcccggg gaagccgacg atcaggtcga tgttgatgtt 720

ggtgaagccg gcctcgtggg cgtcctggac ggcggtgagg gcctggtcgg cggcatgggc 780

gcggccgagg cggcgcaggc gcgcgtcgtc gaaggactgg acgccgatgc tgatccggtt 840

gaagcccagc tcgcgcagga ggcgtagttt gtcccggtcg agggattccg ggctgccctc 900

gatcgtggcc tccctgaccg tgctcaggtc gaactcggcg gacagcgtgc cgtacaggga 960

ctcgatctcg tgggggccga ggatgctggc cgtgccgccg ccccagtaga cgatgtcggg 1020

gtggtagtgc tcgcgcaggg cgggggcctg gacgcggatc tgggtcctga tggcctccag 1080

gtacgcgacg cggcccgggc tctgctcgcc cagccgcagg tcgcgtacgg ggacctggac 1140

cacccagtcg cagaagtggc acttggagtt gcagaagggg acgttcgcgt agatcagcag 1200

gggcttggtc atgaggaacc tctcacgtgc atctccctga cgccgaagat ctggctctcg 1260

tagaaggagc gcttttcggg acggagctcg gtgaagcggc tcagcaggcg ctccagcgcg 1320

atgcggccct ccaggcgccc gagctgggcg cccaggcaga agtgcacgcc gtggccgaac 1380

gcgagctggc cggtgagctg ccggtccagg tcgaagcgtt cggggtcggc gaagtggcgt 1440

tcgtcgtggt tggcggacag gagccaggcc atgacgaacc tgttggccgg gatggtctcg 1500

ccggccacct gtacgtcctg cgtggtcacc ctgcccgcct gcatgaacgg cgagcgcagg 1560

cgcatgctct cctccagcgc ctgggggatg agcgacatgt cggctcttgt ccggtgggcg 1620

gcctcggggg cgtccgccag gcagagcacg gtgttgccga tcagcacggt ggtggtgacg 1680

tgcccggcca gcaggagcag gctcgcggtg ttgacgacct gctccgcgtc gagccgctcg 1740

ccgtcgatct cggcatggac gaggcgggtg agcaggtcgt ccctgggacg ggtacggcgg 1800
tccgcgcagt gctcgcgcag gtagtcgttc atctccgcca tggccgccgc cacccgcccg 1860
gccagcgacg ggtcggcgaa gtcgggcacc tggagcgaca tgagccggtc cgaccaggtg 1920
cggaacaggt cgcggtcgga cggcggcacg ccgagcagct cggcgacgac ggtgacgggc 1980
aggggatagg ccaggctctc gaccaggtcg aagtcagggc cgggcagggc gtcgaggagc 2040
tcgtcggcga tcgcggcgat gcgtggttcg agggccgcga cggagctcgg ggtgaaggcc 2100
gcggtgatga gcctgcgctg gtggcggtgt tcgggcgggt cgatcatcgt gatgccgccc 2160
tggccgaacg gctgccgtcc catcagcctg gtgcggtcgg aggagaaccg ctcgtggtcc 2220
gccagaacgg cgcgtacgtc gtcgtagcgg aagacgtggt acatcccata ggaatcccgc 2280
cagaccgggt gcgtgtcgcg catctcgcgc agccaggcga acagggacgt cccgccgtcc 2340
gtgaccgaag gtggccttac gggggccgtc atcggggcgc ccgcttgacg cgtaccggca 2400
ggctcttgac gccgaacatg gggtcctcgt agtacgacag ctccgcgtcc gggtcgatct 2460
cgacctgctc gaaccggtcg aacaccagct ccagcgccac cttcccctcc accctcgcca 2520
gcggcgcgcc caggcagaag tggatgccgt ggccgaaggc gtactggcgg ttgggctggc 2580
ggccggggtc gagccggtac gggtcgggga actggcgctc gtcgtagttc gcggacagca 2640
gccagttgat gaccatggaa ccggccggga tcggcgtgcc gtcgatctcg acgtcggccg 2700
tggggagccg ggcggcctgg gtgagcggcg ggcgcagcct gagcacctcc tcgaaggcgg 2760
gtgcgatcag ctcccggtcg gcccgtacgg cgctctccac gccgggcgcg tcgcggaagc 2820
acaggaacgc gttccccagg gccatcgtgc tggtgatctg cccggcgagc agcagcaggc 2880
aggcggagtt gacgatctcg gcgtcggtga gccggtcgcc gtccacctcg gccgcgacca 2940
gccggctgac gaggtcgtcc tgcggacggg tgcggcgctc gcgcacgtgg gtgaggacgt 3000
attcccccat ttcgcgcatg gcctgaccga ccatccgccc gatctcgatg tcccgcgggt 3060
cctcgacgtg cagtgcgacc agccggtcgg cccaggtgcg gaacagctcg cggtcgtgga 3120
tcggaatgcc gagcagctcg gcgatcacca tcacgggcag cgggtgggcg atcttctcga 3180
ccaggtcgaa ctcgtcctcg tcgatctcgt ccagcagcgc cgtggtgagc tcggcgatcc 3240
gcggcaccat gtccgacgac attttcgggg tgaacgcctg gctgatgagt ttgcggagct 3300
ttcggtgctc cggcgggtcg gtgagcatga ggctgcccgc gctgaacgga tcgccgccgg 3360
gccggagccg gttgatgtca gaggagaacg ccgcatagtc cgacatcacc cgctgtacgg 3420
ccgcatgccg gaaaacgtgg aagatgccga atgcgtcgcg ccagaccggt tgttcgtccc 3480
gcatcgtgcc gagccacttg aggagggtct ggccgccgtc cttgatgcgc ggccgggagt 3540
cgatcggcat cgtcatcgtc gggtccggcg ggacgtgtgt ctcgttcaca attgactcct 3600
cagacggagg cggccagggg ctgggggatg agtttcgccc ggtatttcgc cgcggcgccg 3660
tcgaagatct cgacggtacg gcgggcgggg tcggtcagtt cttcggccgg gacctggtca 3720
gcaggttcga aagagctcct gatgaggccg gccaggctgt cggcgtgcgc cgccagatcg 3780
ggtttcgccc cggcgatctc ggcgtgcagg tgaagtttga tgaacccgga aagcagggca 3840

tcgagcgcga aaccgtggga cgggtcccac attccgctct cgtggagcat ccggagataa 3900
tcgcgaaatg gcgtgaacgt ggtgaagcgg taacactgga agccgagctg tggatcgtcg 3960
caggcccttc cgagcgtttc cttgtcatag gtgtagagtg ctcggaagag tggatacttg 4020
aacacgagtg tgaacagctc gcgtgagaac tcggagagcc gcgcgacggc aggacgaagc 4080
accgctattc gcgaaatatc ctggcagact ccgacaattt cctgaatgac gagcgttctt 4140
atgagatcgt ctttcgtgtc ccagctgaga taaatggtgc cctttccgat gtccgcccgc 4200
cgggcgatct cgtccatcgt ggtccgcttg tagccggatc gcaggagcag gctcctggtg 4260
acgtccagag tccggcgtac gcgatcgtcg ctggtcctgg cacccgtgct gcgtgtcgag 4320
acggtgctca tggctgggtg gctccttccc cgtaagagct ggtgcgcatg aaccggtatt 4380
ccctaccggc tagggggatt ttggtgggag atgtgctctg ggcccgtcag agctggagat 4440
ttcgaagata ttcgaaatgc tcagatttca acagtcaggt gccccctccc tgactggctg 4500
accgattttc gattttagtc agccagttcg cattcttgac cgcgttccat atgtacacct 4560
agcgtcggcg atgtcgcaag ggtgttccta taccaggcga tgacctccgt gcgatgcaca 4620
tcagtgcgtt tgcctggaat gtcgatgggg cgtttacccc cggaaggaga gtccgcatgg 4680
acctcagcga tctgcccatg gacgtcttcg aactcgccga tgacggtgtt gcggtcgagt 4740
cccttactgc tggtcacggc atgaccgaag tgggagcttc gtgtaactgc ttctgctaca 4800
tctgctgctc ctgctcgagc gcctgacgcg gtacgcgggc gtcgccacgg cggcgcccgc 4860
gcctgttgcc ttttggcgcc aataaagcga atggggctca tgtaatggcg gttattgagc 4920
gggaagaaag cgacggcccg ccggtcgctg tccgcacgtg cggggtcgcg gccgcggagc 4980
tggaatccct gcgatgggac gagtcctatg cgctggtgac ccggctgctc gcggagaccg 5040
cccgggtggc ggcggacggg cggcggctgg ccgtggcgct cggtgacgtc atcggagggc 5100
tcggcccgtc cgggtcccgg cccgtcctcg tggggctgcg gcgggcgctg cacaccgggc 5160
gtctcccctc cgggcgggag tgggggcccg aggccggggc ggcgctgccg tacgggctga 5220
ggggcgaggt ggaggagtgg gtacggcgtg cgcgcgagtg cgccgggctg cgcgcccgcc 5280
tgcccgaggt cgtcgcccgg gagtccctgg agaaggagga acggctgcgc gccgccgcgg 5340
ccgaccccgc cttccgcagg gggctggcgc tggccggcgg cgagctggcg gtcgacctgg 5400
agacgtggct cgcggacccc gcgcggcgcc ccaagccgca gaagctgctc aggctggcca 5460
aatacctggt cagggccgcc gtgaagacca gcccgtacag cacgttcacg agcaccgggc 5520
gggcggtgtg gggcggtggc gagcggcacg tggagcgcgt ggtgccggtg ctggaactcg 5580
acggcgtcca gacgttctcg gacgatcggg tcagggtgaa tccgtcggtc accgtcgcgg 5640
gcgggacggc ggagttcatc gggccgcctc ccggggaggc gctggtgtcc atcggggtga 5700
cggaggcggt ggccgcgtgc ctgcgcgtcg cggagggcgg ggagtgggtg ccgcggggcc 5760
ggttcgcgga ggctctgggg gctgagcctg ccgcagtcac gaaatttctg gacaagttgc 5820
ttgcggttgg gctcctggag gcccggccgg acggcgaccc gcccccgcac ctgctggacg 5880

ggatccggcg cacggaacgc gccaccgacc cgtccacctt ccgccacgaa ctcggccgcc 5940
tgatcgccgc cgcccgcgcc gccgcccgcg ccaccgcccc cggcgctgac acggccaaga 6000
acgccgcccc tgccgggcgc gccacccgcg gcgatggcgg tgctgatgcc gagcgcgcca 6060
cccgcggcga tagcgatgct gatgacgggc gcgccacccg gggcgacggc ggtgctgatg 6120
acaggggcgc cgcgcggggc gacggcggtg tggggcgggc ggttgtgcac gaggtcgcgg 6180
tctgcgtcga gcccgtggcg aggctggatc cggcgcgatg gcgtggcggg cttgccgatc 6240
tcgacgtcgt acgccgctgg ctggccgttt tcgacgccaa gcaccccatg cgcctcgcgg 6300
tcgccgccta cctcaccacc cggtacggcc ccgacccggc cgtccccttc ctcacgctcc 6360
accgccacat ccagcgcgag ctggccggca ccggcgcggc aggccgggag ttgcgcgcgt 6420
tcctcggctc gtcggccgcc tggacccagc cgctaggcac ctccgccctg cccaggatca 6480
gggagctgga gcgcctcaga gccgaggcga gaagcctggc gctggacgcg gaggaccccg 6540
acggcacctg ccgcgtcacc cccggacagc tggccgcgca gctggagtcg tggccgtctt 6600
ggatcgcgat ccccgcctcc tcggcctgct acgtacaggc ggtggccggc accctggtgc 6660
tcaacgtcgt ccacggcggg cacggccgag ccctgcgcag gctcgaccac ctcctcgaac 6720
gcgccggcgg ccaccccgag ccgccccgga tcgaggaccc ggacggcgcg gtctacgccg 6780
agttctccgg cgacctgggc tccacgctca acgcgcgccc gccgagcacc cgatacgaga 6840
tcgactaccc ccactcgccc ggcacgcgcc cgcccgacct gcggctgccg ctcacggacc 6900
tgcacgtgac gctctgcccg gacagcggcc tgcccgagct gcgttccaag cggctcggcc 6960
gccgggtcgt gccgttgcat ctcgggctgg ccgccgagtt ccggcttccg ccggccgcgc 7020
ggttcatcga acgcgtcttc ggccccggct acctgctcca tcccagctcg ccgccgctgg 7080
tgcggatggg ccgggtcccc tcggaggtca cccggtatcc acgcgtcgag gccgggcgcg 7140
tggtggtgca gcggcgtcgc tggctggcgc ctgcggccac gctgccggtg cgcgcgaagg 7200
gcgagggcga cgcggcgtac ctcacgcggc tgatcgcctg ggcggacgag tacggcgtac 7260
cgcaccgatc gttcgtccgc gcctggccgg agcagaccgg cgacaaaggg caggacaagg 7320
cgcgcaaacc gctcttcctg gacctggcga acctgttcct ggtcaagaac ttcgagcggc 7380
agatacgtgg ctgcgcgttc gcgctcttcg aggaggcgct gcccgacccg ggaccggagc 7440
gggtgaccga atacctgatc gaggtgggag gccgatgagc tgggtgagcg tgcacgtctt 7500
ctcgcagggg ccgctcgacg acatgatcac gggcctggcc gggccgctcc tcggcgacga 7560
cggcttcttc ctgcgctact gggagggcgg cccgcatctg cggatcaggg tcaggtccca 7620
cccggcggag caggtcgtcg agcgggccga ggcgtatctg cgcgagcacc cgtcagaggc 7680
ggtgatcgac ccggaggagt acgccaggct cgccgcgacc ctcgccgccc gcgaaggcgt 7740
ccccggctat acgcggcgcc tctatccgaa cgactccgcc cacctcatcc cgtaccagcc 7800
ggaacacgac cgctacggca cgggccgctc gctcgacgcc gtggagcggc acttccacga 7860
ctccagcgtg atcgcactcc agctgatcgg ggcggggctc ggacacgagc ggcggagcat 7920
ggcgtggctg accatgctgc tggtcgcctg gcaggtcgcg ggggagggca gggcgttcgg 7980

cccgcccgct gacgccgggg ttcaggtgcc ccacgagcgg atggcggcgc tggtcgagca 8040
cgccagggcg gtgacacagg agcctgcgcc gagcgagccc cgtggcgtgg tggccgcctg 8100
gtacgcctcg atcgcgcggc tggtggagga actggcggcg gcggggttcg acgcggcgcg 8160
gacgggggcg accgtcgatc tggccgcgca tctgctggcc aacaggctgg ggatccgggt 8220
gcaggaggag gcccggctgc gtcacctggc gtccggggcc acgagggagt cggaggtggg 8280
ggtcggatga cctggacgcg gctgcgggtg gactatcacg acggcccgat ggacgacctc 8340
atcctggacg cgctccgccc ggcctggcat gaaatacggg gatatttcct gcgccactgg 8400
gtgtgcggcc cgcacctgcg gatcttcgtg gacggcgacg cgacgccgat cgtccgggcg 8460
gtcgagcgcc acctggccgc ctgcccgtcc gccggcacca ccgacccgca ggcgctgctc 8520
cccctgcacg agcgcctggc cgagctggaa ggcgaacgag gcccgctgct gccatgggcc 8580
cccgacaaca ccgtcaccgc cgaacccccg gggctcgaca ccgagctcga ccggttcctc 8640
gcggacttct acgccgacac caccgaggcc gcgttcgacg ccctcggccg ggtcagagcc 8700
ggcacgccgc tgccgggcat cgccttcgac ctggtggtcg ccaccgcgca cgacctgtcg 8760
gagggcggcc tgcccactgc caggacctcg ctgcgctcgc acgccgaggc ttacctgtcg 8820
cgcctgccgg gaggcgtccg cgccaagtgg caggcgcact acgaacgcaa ccaggagccg 8880
ctgaccgcca ggatcaaggc cctgaccggc gccggagagc ccggcgcgtg gctgcgtacg 8940
atccgggcca cccgcgaccg ggccgtaccc ctcatcgacg agggacggct gagcctcggc 9000
tacgccaccg acggcccttc cacgcggccc ccgctcgccg cggtctcccc cttccaccgg 9060
aacctggaga ccgacgaacg ctggctcgcc ctcaaggaca cccccgcctt cgccgcctac 9120
cgcctggccc tgaactgcgc gtacctccac ctcacccggc tcggcctcac gcccgaccag 9180
cgcttcctga tctgccacct cgccgcgagc gccgccgacg acgtctaccg ggccgtggcc 9240
tcatgaccgg ggaacggtac gagctgcgcg ccggcgtgca cctcgcccgg ctggacgcgg 9300
gggtccgcct gttcgcctgg ccgcacgccg agaccctcgg cgagctcacg cgggatgagc 9360
tcgacctgct ccgcgacctg gccgccggac cccgcaccgc cgggggaccg ctcgtcgaac 9420
gcctgcgcgc cggaggctgg ctcaagatca cgattacccg ggacggcgtg cccctctaca 9480
cgctcgcccc gctgcgcccg ccgccgccac ggccggcggt gccggagcgg ccggttctgt 9540
ccaggttcgc cacgctgcgg cgcgaggggg acgcgctggt cgccggctcc ccgctggcct 9600
ggtgcgacct gtacgtccac gaccccggcc tggtcggcga cgtggtccgg cccggcggcg 9660
aggtcctggc cgacctggtg tgggccgggc tggccgtgcc cgaggaggcc gaggaccggg 9720
agctgcggtt ccggcagtgg gcgccgcacg agctggagtt ccatgaccgg agccgggtgg 9780
ggcaccgcgg ctacctgggc gatgggttcg gcgggacgtt ctgggcccgc ggccggttcg 9840
acccgctgcc cgcccggccc cggccctacc ccggccgggc gatcgagctg cccacgcccg 9900
acctggacga actgcggctc aaggacccct cgctcacgga cgtgctggag ggccgcaggt 9960
ccgtacgcga gcacgacgac gagcgcccga tcaccctcga cctgctcggc gagctgctgt 10020

accgcacggc cagggtgcgg gagtgctgga cggccgacgg cgtcgaatac acccgcaagc 10080
cgtacccctc cggagggtcc gtctacgagc tggagatcta ccccgtcgtc aggaacgtgg 10140
ccgggctcga acccggcatg taccactacg acgggcacga ccacgtgctc aggccggtac 10200
gtgaggccgg ccatcccgcc gtacgccggc tgctgaccgt cgccgggcac ggcgccgcca 10260
ccggccgccg cccccaggtc ctgctcgcgg tctccagccg cgccggccgg ctgctgtgga 10320
agtacgaagg catggggtac gcgctgacgc tcaagcacgt gggtgtgctc taccagacgc 10380
tctactgcgt ggccacggcg atgggcctgg cggcctgcgg gctcggcagc ggcgactcgg 10440
ccgcgttcgc cgaggcgacc gggcgggacc cgttggagga gtgcgcggtc ggcgaattca 10500
tgatcggaac cgcggaggca ccacgttgaa cgacttcaaa cccagactcc ccgcggacat 10560
acgcttcgcg cccacctccg gcggtgtcgc cttcctcacc cgcgaaggcg tcgtgcggat 10620
gaacggggcc tccgtccacc tgtgggtgga gcggctggcc gtgcacctcg acgggagcag 10680
gaccgtggcc gagctgaccg cggagctgcc cgacgagcgg cgggcgttcg tgacgagcct 10740
gctgaccacg ctgcgcgagc gcgggctcgt ggaggacgcg ggagagcgcg gcgtgctgcc 10800
cgtggcggcc ggcaccgcgt gctcgctgct ggccgacgcg gaccggttcg gcgagctggc 10860
gcccgccctg gccgacgccc tgcgcaggct ggggctggag gtgctgacag agccgtcggg 10920
cggggcgctc gtgcacgtcg cggcggccga cgcggacggg ctggccgccg ccgggcgcct 10980
cgaccggctg agcgccgagc gcgggctgcc catcgcgcac gtgctgctgc gcgagggcga 11040
ggcgtggtgg agcgagaccg gcgtcgtcgg gcgcgacgtc ccgccgtggg cggccgggtg 11100
gcggcggctg gcggccatgg aggccgtccc gcgtaccgcc gggcacgtct ccgtgtcgcc 11160
cgtcgccgcg gccgtggtcg ccgggcaggt ggtggcggac ctggggcgct gcctgtccgg 11220
aacgccccgc gtgtccggcg gtccgcggct gcacgtggtg gacctggacg ggttcgggat 11280
gcggacgcac tccttcctgc cgcacctgtc ggcgctgccg cccggcacgg gggtgagcgc 11340
ggggccgctc ggcgaggagg agttctcgcg gcgggcggcc aggttgatgg actcgcggac 11400
gggggtgttc gccgagatcg aggaggggga gtacgggcag ctcccgctgc acgtgtcggt 11460
gaccacggtg gccgatccga tgggggccct cgggggtgcg ctggcgcggg tgatcggcgt 11520
gggggcggac ttcgccaccg cgcgataccg ggcggcgctt aaggcactga ccgtgcacgg 11580
gctgctcgcc ctcgaccccc gccgcctggt cacggccgag gggacgcccc tgaccggcgc 11640
gggggaaccc gccagcggct tcccggctga tccgggcgcg agggaacggc cgatcggtgg 11700
cttttcggct gatccgggtg cgggtgagcg gccggacgac ggtctcctgg acgacccgct 11760
ggtgggcgac gtcgcggcgg gacgggtcga ggcgttcgtg cgcggccgtg acctcgtcga 11820
gggcggtgag gtgctgctgc ccgcccgcgc ggtgttcctg gatccgcacg ccaccggggc 11880
cgccgcgggg tactcggccg aggaggcgct ggtcgcgggg ttactggacc actgtgcggc 11940
actggccggg ctcgacggga cgctgccgcc cgtggacgtg ggcggcgcgc gggcggccga 12000
cgacgagacc gcgcggtcgg cggcgctgct ggacgcgatc gggctgccgt accaggtgct 12060
ggacgccacc ggcccgtcgg gcgtgcccgt gcacgcgggg acgctcggcg ggcggctggt 12120

ggcgcgggcg gcgggcgtca cgcccctggc cgcgctgcgg gccacgctcg aagggatcct 12180
ggccgcgtac cagggcattc cgggccccgc acccctgccc gacctgtgcc cggccgtcgc 12240
gcaggagccg ccggtcgtcc tcgacacggg ggcggccgtg ctcggcgccg gtgagctcgc 12300
cgccgccctg gccggcgccg ggctgcgggt cagcgccgtt ccgctggatc acgaccggga 12360
ggtacacgcg ctcatgccgc acgtcgtccg cgtcgtcacg gagccgctcc atggctgagg 12420
tcgtggggac gggcctgctg gccgaggaga tcacgatcgc gctgaaggac cgcgacgggc 12480
gggcagaggt gacgatcacc gcctgcgacg gctgggcgcg cggcccccgg cgcgacgggg 12540
cggcgtggtt gcccgtgcgg gtcgagtacg gggtcgccgt catcgggccg ctggaacgcc 12600
ccggcgaacc cggctgcgtg acctgcttcg agacccgccg ggaccgggtc aggaccgaca 12660
gcgccgatcg ggccgccgtc ctggccgtcc acggccccga actcgccggg cggcctcccc 12720
cgtacctcgg ccgcctggcc gcccgcacga tcgcggccct ggccgcgcac gccgccgagg 12780
gccccggatg cgccgtctgg tacgtcgacc tgcgcacgct caccgccgaa cgccacacgt 12840
tcctgccgga accccactgc cccgactgcg gcgacctgcc ggacgacgac cgcgcgtccg 12900
ccgccttcga gcccggcgcc gccccgcgac ctcccggcta ccgggtcagg aacgtgccgg 12960
ccgagcacgg cgcgctgctc gacacgtacg tggacggcga atgcgggctg atcaggcccc 13020
tcgtccggga cacgctcggc ggcctggtga tcgccggggc gatgctgccg ctgcggttcg 13080
agggcggttc ggagcccggc gtgggccgca cccgcggcta ccgggccagc gaggtcaccg 13140
ccgtgctcga agcgctcgaa cggtggggcg gcgtcgagcc cggcggcaag cgcacccgcg 13200
tgcgggcctc gtacgcggag gtggccgcgg acgcgctcga ccctcgaacg ctgggcgagc 13260
acgacacctc ctcctacgag atggaggggt tcgcgttccg gccgttcgcc gaggacgccg 13320
tctgcgactg ggtgtggggg tactccttcg cccggcgctc gccgatcctc gtgccggaga 13380
gcctggccta ttactacgta cgggggcggg agcggccgtt cctcttcgag atctcgaacg 13440
ggtgcgcgct cggcggcagc atggcggagg ccctgctcta cgggctgctg gagacggtcg 13500
agcgggacgc ctttctcatg acctggtacg ggcggctccc ggtgccgcgg atcgaccccg 13560
gcacggcacg ggaccgcacc atcccgctcc aggccgccgc gatcatcgcg gagaccggtt 13620
atcgggtgga gctgtacgac acgacgatgg agcacggcat ccccagcgtg tgggcgatgg 13680
gcgtgcggca ggacggcgat ccgggcaggc cgcgcctggt gtgcgccgcc ggggcccacc 13740
tggtgccgga gaaggccgtg ctcagcgcgc tgagcgagct ggggccgctg ctggccgacc 13800
tgatccggcg gtatccggac gaggtgcacc gagcccagga gatggtgaca aatcctgatc 13860
tggtggcgac gatgcacgac cactccaccc tgtacggcgc cgacgcggcc ttcgaccggc 13920
tctcgttcct gaccggcggc acgggcgtgc gggggctgcc cgacatggag gcgttcaccg 13980
tgcccggtga tctcgacgtc atgagggtgg tggaccgctt cctcgccgag gggatggacg 14040
tcgtcgtcat cgaccagacc acgccggagc atcgggcccg cggtttctcc tgcgtcaagg 14100
tgctggtgcc cggcacgctc ccgatgacct tcggccaccg caaccgcagg gtgcggggcc 14160

tgacccgccc gctggagctg ccgtaccggc tcggccaccg cgacacgccc ctgacccccg 14220
ccgacctgaa ccccgaccca caccccttcc catgaccacc gagccgtttc ccgcgccgac 14280
ggggccgccc gcagcgccga cggggccgtc tcccgcgctg gcggagccgt ctgctgcatc 14340
gcccggcccg ttgtccgttc ggcaggggcc gtctcctgtg accgccgagc tgcgtgacct 14400
ggcgatcttc cgtgccgatc tgcatgccca ccccgagctg tcctggcagg aggccaggac 14460
ggccgatcgc ctggcggggc ggctggcggc ggccgggtac gaggtcacga ccgggatggg 14520
cggtcatggg gtggtcggga ggctgtgccg tggcgacggg gtcacggtca tgctcagagc 14580
cgagctggac gccctccccg tcaaggagga gaccggcctg tcatacgcca gcaccgccac 14640
cgccacgacc ctcgacggca ggacggtgcc ggtctcccac gcctgcggcc acgacctcca 14700
tctcgcctgc ctggtcggcg ccgcccggcg gctggccgcc tgcgacgact ggcggggcac 14760
ggtgctcgtg atcggccagc ccgccgagga gacgctggag ggcgccgccg cgatgctcgc 14820
cgacggcctg tacgagcggt tcggcgtccc ggacgtggcg ctggcccagc acgtctcgcc 14880
gttccccgcg ggcctgatcg cctacccgga gccgcccacc gcggccggcg ccgaactccg 14940
cgtcgtcgtc acgggggacg gcggccacgt cggagacatc ggccgggctg gtcacgtcga 15000
tcgagccgtg gggcgcaatc cggtcgcggc cgtcgccgcg ctcgttcacc ggctcgatca 15060
gacggcgttc gaccaggcga tcgtcaccgt ggggacgttg cacgcgggcg agcgcgccaa 15120
cgtgatcccc accctcgccg aggccgggat cacggtcagg gccgcgacgg aggaggcggt 15180
gacccgggcc gtcgcccgcg tcgcccggct ggcggaggag acggcgggag ccggcgtcat 15240
cgtggtctcc agggtgccgc ccggcgtgaa cgaccccgcc gccaccgccc tggtacgccg 15300
ggcccacgag gccgcgctcg gcgcggtcgt caccgccccc ggcggctcgg cctgcgagga 15360
cttccccctc tacggggtgc cctccgtcta ctggtacgtg ggcgcggccc cacccgccgg 15420
cctggtcgga cgcccgcata ccgggacttt ccgccccgat cccgtgccca cgttgagggc 15480
aggggtcacc gccatgcaaa cggctgccct ggcagtcctt gcgaacgcgt cacagttcgc 15540
ccctccatcc cgatatcatg gacccggagc agtagccgag cactga 15586

<210> 64
<211> 60
<212> PRT
<213> nonomuraea sp.

<400> 64

Met Ser Asn Ala Ser Ile Gly Gln Glu Ile Gly Val Glu Gly Leu Thr
1               5                   10                  15

Gly Leu Asp Val Asp Ala Leu Glu Ile Ser Asp Tyr Val Asp Glu Thr
            20                  25                  30

Leu Leu Asp Gly Glu Asp Leu Thr Val Thr Met Ile Ala Ser Ala Ser
        35                  40                  45

Cys Thr Thr Cys Ile Cys Thr Cys Ser Cys Ser Ser

50                    55                    60

<210>  65
<211>  180
<212>  DNA
<213>  nonomuraea sp.

<400>  65
atgagtaatg cctccatcgg tcaggagatc ggcgtcgagg gcctgacggg cctggacgtc      60

gacgctctgg agatcagcga ctacgtggac gagacgctgc tcgacggcga ggacctcacg     120

gtgacgatga tcgcctccgc ctcgtgcacc acctgcatct gcacgtgcag ctgcagctcc     180

## Description

The present invention relates to the precursor proteins for thiopeptide biosynthesis, and the corresponding structural genes and uses thereof. The present invention also relates to methods for genetically manipulating the thiopeptide precursor protein or host cells expressing a gene encoding said thiopeptide precursor protein to produce thiopeptide compounds or their derivatives. The present invention further relates to the cloning and characterization of genes involved in thiopeptide biosynthesis and their use in thiopeptide compounds production.

The thiopeptides are naturally occurring, sulphur-rich, highly modified, macrocyclic peptides, many of which have potent antibiotic activity. These complex natural products are grouped as thiazolyl peptides according to Berdy's chemical classification of antibiotics (Berdy J. 'Recent developments of antibiotic research and classification of antibiotics according to chemical structure. 'Adv Appl Microbiol. 1974;18(0):309-406.) and include thiostrepton, nosiheptide, micrococcin, nocathiacin, amythiamicin, GE2270A etc. The thiopeptides share a number of common structural features: a tri- or tetrasubstituted nitrogen heterocycle clustered in a central polyazole domain that is part of a macrocyclic framework consisting of modified heterocyclic residues, including thiazoles, oxazoles, and indoles, and dehydroamino acids. It is this macrocyclic signature of the thiopeptides that distinguishes them from other thiazolyl compounds, such as bleomycin, bacitracin, or microcin B17. It was Hensens who first suggested grouping thiopeptide antibiotics according to the structure and oxidation state of the central heterocyclic domain (O.D. Hensens and G. Albers-Schoenberg. 'Total structure of the peptide antibiotic components of thiopeptin by 1H and 13C NMR spectroscopy.'Tetrahedron Lett. 1978, 3649). A recent review by Mark Bagley and colleagues from Cardiff University has extended Hensens' classification system to describe five distinct central heterocyclic domains: tetrasubstituted dehydropiperidine or saturated piperdine, dihydroimidazopiperidine, trisubstituted pyridine and tetrasubstituted hydroxypyridine (Bagley et al. 'Thiopeptide antibiotics.' Chem Rev. 2005 Feb;105(2):685-714).

Despite considerable structural homology, the sites and modes of action (inhibition of protein synthesis) of the thiopeptide antibiotics can be categorized into two functional classes: those that bind to a region of the 23S ribosomal RNA known as the L11 binding domain and those that bind to the Ef-Tu protein complex involved in the elongation cycle. Thiostrepton, which was first isolated in 1954 as a secondary metabolite of *Streptomyces azureus,* is often regarded as the archetypal compound of the thiopeptide family (Donovick et al. 'Thiostrepton, a new antibiotic.' Antibiot Annu. 1955-1956;3:554-9 and Bagley et al. 'Thiopeptide antibiotics.' Chem Rev. 2005 Feb;105(2):685-714).

It was postulated that thiopeptides are synthesized by a nonribosomal process that utilizes large, multi-enzyme complexes, termed nonribosomal peptide synthetases (NRPS) (Mocek et al. 'Biosynthesis of the modified peptide antibiotic thiostrepton in Streptomyces azureus and Streptomyces laurentii. J.Am.Chem.Soc.1993, 115, 7992 - 8001). An example of an NRPS-assembled macrocycle is the immunosuppressant peptide cyclosporine which is produced by the fungus *Tolypocladium inflatum.* NRPS-assembled peptides are not restricted to the common 20 L-amino acids typically found in proteins. Instead these enzymes have the biosynthetic capability of incorporating a broader repertoire of hundreds of unusual amino acids and derived residues.

Preliminary experiments conducted by Heinz Floss et al., University of Washington, suggest that the thiopeptides are indeed produced by a nonribosomal pathway. This group treated cultures with chloramphenicol at levels that inhibited ribosomal protein synthesis. However, *de novo* production of thiopeptide continued. Oligonucleotides derived from the amino acid sequence representing either thiostrepton or the related nosiheptide failed to hybridize to any homologous sequences when used to probe Southern blots of genomic DNA from the respective producing organisms (T. M. Smith, Y.-F. Jiang, H. G. Floss. 'Thiopeptide Antibiotics" in "Biotechnology of Antibiotics" W. R. Strohl, Ed., 2nd Ed., Marcel-Dekker, New York, 1997, pp 393-413).

Additionally, a NRPS gene fragment that is postulated to encode a module of the micrococcin synthetase complex has been identified in the producing strain (Carnio et al. 'Pyridinyl polythiazole class peptide antibiotic micrococcin P1, secreted by foodborne Staphylococcus equorum WS2733, is biosynthesized nonribosomally.' Eur J Biochem. 2001 Dec;268(24):6390-401.).

However, to date there has been no published description of a thiopeptide biosynthetic pathway.

Therefore, there is a need to identify genes involved in thiopeptide biosynthesis that will provide tools for efficient thiopeptide production and possibilities of generating alternative thiopeptide structures. The present invention fulfills this need and discloses for the first time a chromosomally encoded backbone for thiopeptide biosynthesis, as well as the core biosynthetic enzymes involved in thiopeptide biosynthesis.

### 1. A chromosomally encoded backbone as starting material for thiopeptide biosynthesis

The inventors have indeed found clear evidence that a chromosomally encoded backbone is the starting material for thiopeptide biosynthesis, for example in actinomycete species.

In a first aspect, the invention relates to a thiopeptide precursor protein comprising an amino acid sequence selected among the group consisting of
(i) SEQ ID NO:1;
(ii) SEO ID NO:5;
(iii) SEQ ID NO:11; and
(iv) a variant of any of said amino acid sequences.

As used herein, the term "thiopeptide" refers to thiazolyl peptides according to Berdy's chemical classification of antibiotics that are characterized, contrary to other thiazolyl compounds, such as bleomycin, bacitracin, or microcin B17, by a tri- or tetrasubstituted nitrogen heterocycle clustered in a central polyazole domain that is part of a macrocyclic framework consisting of modified heterocyclic residues, including thiazoles, oxazoles, and indoles, and dehydroamino acids.

The term "thiopeptide precursor protein" refers to a gene encoded polypeptide backbone which could be used as a starting material for in vitro or in vivo thiopeptide synthesis. Preferably, said thiopeptide precursor is a precursor for an inhibitor of the bacterial elongation factor Ef-tu. Ef-tu inhibitors are described for example in the review by Hogg T, Mesters JR and Hilgenfeld R. ( 'Inhibitory mechanisms of antibiotics targeting elongation factor Tu.' Curr Protein Pept Sci. 2002 Feb;3(1):121-31). Examples of well-known Ef-tu inhibitors are GE2270A (as defined in Selva et al. 'Antibiotic GE2270A: a novel inhibitor of bacterial protein synthesis. I. Isolation and characterization.' J Antibiot (Tokyo). 1991 Jul;44(7):693-701.), GE37648A (as defined in Stella et al. 'Antibiotic GE37468 A: a new inhibitor of bacterial protein synthesis. I. Isolation and characterization.'J Antibiot (Tokyo). 1995 Aug;48(8):780-6. Erratum in: J Antibiot (Tokyo) 1995 Dec;48(12):C-3.), and Amythiamicin (as defined in ('Novel antibiotics, amythiamicins.' J Antibiot (Tokyo). 1994 Jun;47(6):668-74, 1145-52 and 1153-9.). Novel Ef-tu inhibitors are also described in International Patent Application Number PCT/US07/025955 filed on 19 December 2007. Such novel Ef-tu inhibitors include more specifically compounds of formulas I through XI (including pharmaceutically acceptable salts thereof, as well as enantiomers, stereoisomers, rotamers, tautomers, diastereomers, or racemates thereof) as represented below:

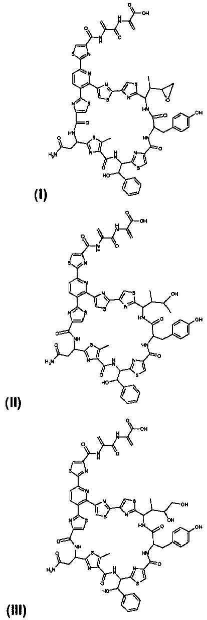

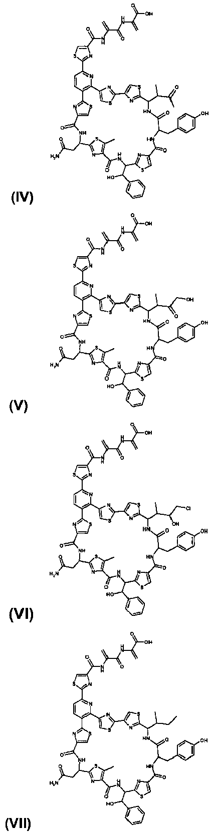

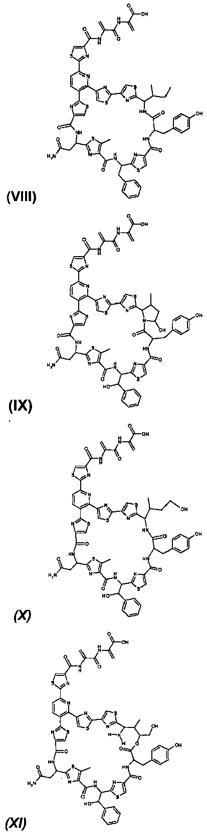

The amino acid sequence of such precursor protein is critical for the final structure of the thiopeptide. As this will be described below in detail, the thiopeptide precursor proteins of the invention can be used to reproduce the backbone of a newly identified thiopeptide or to generate novel derivatives of known thiopeptides, for example with improved properties.

The invention therefore not only relates to the precursor protein of SEQ ID NO:1, SEQ ID NO: 5 or SEQ ID NO: 11 but also to any variant which could be useful for biosynthesis of thiopeptide derivatives. The one skilled in the art will know how to design said variant depending on the amino acid backbone of the thiopeptide to be synthesized. In SEQ ID NO:1, the six cysteines found within the 14 amino-acid backbone are the precursors of the thiazole heterocycles that decorate these molecules. In addition, two serine residues are involved in the formation of the pyridine ring system at the juncture of the macrocycle and the side-chain. In the thiopeptide side-chain of the GE2270A molecule is found an additional oxazoline and in the novel Ef-tu inhibitors of formulas (I) to (XI) are found dehydroalanine residues formed by the heterocyclization and dehydration of serine amino-acids respectively.

In a specific embodiment, a variant of SEQ ID NO:1, SEQ ID NO 5 or SEQ ID NO 11 has no more than 1, 2, 3, 4, 5, 6 or 10 deleted, inserted or substituted amino acids when compared to original sequence. Substituted amino acids can be either natural amino acid with equivalent functional group or with different functional group or non-natural amino acid.

The following table 1 gives an indication of the thiopeptide precursor protein sequence (SEQ ID NO:1-14) that can be used for biosynthesis of corresponding specific thiopeptides.

**Table 1. Primary sequence alignment of thiopeptide amino acid backbones. The alignment is centered around the macrocycle that is bounded by two invariant serine molecules that form the signature multi-substituted nitrogen heterocycle. Thiostrepton has an additional amino extension of 4 amino acids.**

| **Thiopeptide** | | **Primary Amino Acid Sequence** | |
|---|---|---|---|
| | | **11 aa Macrocycle** | Side Chain |
| GE2270A | | SCNCVCGFCCS | CSP (NO:1) |
| GE37468A | | SSNCFCYPCCS | CSS (NO:2) |
| Novel EF Tu inhibitors | | SCNCFCYICCS | CSS (NO:3) |
| Amythiamicin | | SCNCVCGVCCS | CSP (NO:4) |

| | | **10 aa Macrocycle** | Side Chain |
|---|---|---|---|
| Micrococcin | | SCTTCVCTCS | CCT (NO:5) |
| Thiostrepton | IASA | SCTTCICTCS | CSS (NO:6) |
| Nosiheptide | | SCTTCECCCS | CS (NO:7) |
| Thiocillin | | SCTTCVCVCS | CCT (NO:8) |
| Thiocins | | SCVGSACASS | SSS (NO:9) |
| Nocathiacins | | SCTTCXCXCS | CS (NO:10) |

| | | **13 aa Macrocycle** | Side Chain |
|---|---|---|---|
| Berninamycin | | SCTTSSVSSSSSS | SS (NO:11) |
| Sulfomycins | | SCTTSGCTSSSSS | SSS (NO:12) |
| A10255B | | SCTTSGSACSSSS | SSSS (NO:13) |
| Radamycin | | SCVGSACACSSSS | SS (NO:14) |

The following standard code letter is used in the instant application for describing any amino acid, peptide and protein sequence: A, Alanine; R, Arginine; N, Asparagine; D, Aspartic acid; C, Cysteine; Q, Glutamine; E, Glutamic acid; G, Glycine; H, Histidine; I, Isoleucine; L, Leucine; K, Lysine; M, methionine; F, phenylalanine; P, Proline; S, Serine; T, Threonine; W, Tryptophane; Y, Tyrosine; V, Valine.

More generally, in one specific embodiment, said variant of amino acid sequence SEQ ID NO:1 has the following formula : SXNCXCXXCCSCSX, wherein X can be any amino acid. More preferably, said variant of SEQ ID NO:1 comprises the following formula : SX₁NCX₂CX₃X₄CCSCSX₅, wherein X₁ is C or S, X₂ is V or F, X₃ is G or Y, X₄ is a F, P, I or V and X₅ is P or S. Examples of such variants are the precursor proteins of SEQ ID NO:2-4.

In another specific embodiment, said variant of amino acid sequence SEQ ID NO:5 comprises the following formula: SCX₁X₂X₃CX₄CX₅X₆X₇X₈ wherein X₁ is T or V, preferably T, X₂ is T or G, preferably T, X₃ is C or S, preferably C, X₄ is any amino acid, preferably V, I, E or A, X₅ is any amino acid, preferably T, C, V or A, X₆ and X₇ is, independently C or S, preferably C, X₈ is C or S, and X₉ is either no amino acid residue or T or S. Examples of such variants are the precursor protein of SEQ ID NO:6-10.

In another specific embodiment, said variant of amino acid sequence SEQ ID NO:11 comprises the following formula: SCX₁X₂SX₃X₄X₅X₆SSSSSS wherein X₁ is T or V, preferably T, X₂ is T or G, preferably T, X₃ is S, G or A, X₄ is V, C or A, X₅ is S, T or A, X₆ is C or S. Examples of such variants are the precursor protein of SEQ ID NO:12-14.

### 2. Nucleic acids of genes encoding the thiopeptide precursor protein

The invention further provides nucleic acids of genes and/or open reading frames encoding the thiopeptide precursor protein. More specifically, the invention provides a nucleic acid comprising the nucleotide sequence encoding the thiopeptide precursor protein as defined above.

A "nucleic acid" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid, and in particular DNA or RNA molecule, refers only to the primary and secondary structure of the molecule, and does not limit it to any particular tertiary forms. Thus, this term includes double-stranded DNA found, inter alia, in linear (e.g., restriction fragments) or circular DNA molecules, plasmids, and chromosomes.

A "recombinant DNA molecule" is a DNA molecule that has undergone a molecular biological manipulation.

A "polynucleotide" or "nucleotide sequence" is a series of nucleotide bases (also called "nucleotides") in a nucleic acid, such as DNA and RNA, and means any chain of two or more nucleotides. A nucleotide sequence typically carries genetic information, including the information used by cellular machinery to make proteins and enzymes. These terms include double or single stranded genomic and cDNA, RNA, any synthetic and genetically manipulated polynucleotide, and both sense and anti-sense polynucleotide (although only sense stands are being represented herein). This includes single-and double-stranded molecules, i.e., DNA-DNA, DNA-RNA and RNA-RNA hybrids. This also includes nucleic acids containing modified bases, for example thio-uracil, thio-guanine and fluoro-uracil.

The nucleic acids herein may be flanked by natural regulatory (expression control) sequences, or may be associated with heterologous sequences, including promoters, other ribosome binding site sequences , regulatory response elements, signal sequences, and the like. The nucleic acids may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.).

The term "gene", also called a "structural gene" means a DNA sequence that codes for or corresponds to a particular sequence of amino acids which comprise all or part of one or more proteins, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription.

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG or GTG) and a stop codon.

A coding sequence is "under the control of" or "operatively associated with" expression control sequences in a cell when RNA polymerase transcribes the coding sequence into RNA, particularly mRNA, and translated into the protein encoded by the coding sequence.

The term "heterologous" refers to a combination of elements not naturally occurring. For example, heterologous DNA refers to DNA not naturally located in the cell, or in a chromosomal site of the cell. Preferably, the heterologous DNA includes a gene foreign to the cell. For example, the present invention includes chimeric DNA molecules that comprise a DNA sequence and a heterologous DNA sequence that is not part of the DNA sequence. In this context, the heterologous DNA sequence refers to a DNA sequence that is not naturally located within the thiopeptide biosynthetic gene cluster sequence. Alternatively, the heterologous DNA sequence can be naturally located within the biosynthetic gene cluster at a location where it does not natively occur. A heterologous expression regulatory element is an element operatively associated with a different gene than the one it is operatively associated with in nature. In the context of the present invention, a gene encoding a protein of interest is heterologous to the vector DNA in which it is inserted for cloning or expression, and it is heterologous to a host cell containing such a vector, in which it is expressed.

The term "expression control sequence" refers to a promoter, any enhancer element, or suppression elements (e.g., an origin of replication) that combine to regulate the transcription of a coding sequence. The terms "express" and "expression" mean allowing or causing the information in a gene or DNA sequence to become manifest, for example producing a protein by activating the cellular functions involved in transcription and translation of a corresponding gene or DNA sequence. A DNA sequence is expressed in or by a cell to form an "expression product" such as a protein. The expression product itself, e.g., the resulting protein, may also be said to be "expressed" by the cell. An expression product can be characterized as intracellular, extracellular or secreted. The term "intracellular" means something that is inside a cell. The term "extracellular" means something that is outside a cell. A substance is "secreted" by a cell if it appears in significant measure outside the cell, from somewhere on or inside the cell.

The term "transformation" means the introduction of a "foreign" (i.e. extrinsic or extracellular) gene, DNA or RNA sequence to a cell, so that the host cell will express the introduced gene or sequence to produce a desired substance, typically a protein or enzyme coded by the introduced gene or sequence. The introduced gene or sequence may also be called a "cloned" or "foreign" gene or sequence, may include regulatory or control sequences, such as start, stop, promoter, signal, secretion, or other sequences used by a cells genetic machinery. The gene or sequence may include nonfunctional sequences or sequences with no known function. A host cell that receives and expresses introduced DNA or RNA has been "transformed" and is a "transformant" or a "clone." The DNA or RNA introduced to a host cell can come from any source, including cells of the same genus or species as the host cell, or cells of a different genus or species.

The inventors were successful in identifying small structural genes in the genomes of Ef-tu inhibitor thiopeptide-producing *Nomomuraea species,* which encoded the whole peptide backbone of SEQ ID NO:1 and SEQ ID NO:3. The predicted size of the precursor preproproteins are 57 and 49 amino-acids respectively and are depicted in SEQ ID NO:19 and SEQ ID NO:20 respectively. However, there are a number of initiation codons that may indicate alternative translation start sites. The 14 amino-acid thiopeptide precursor sequence is at the C-terminus.

A similar structural gene for the unrelated thiopeptide thiostrepton has also been identified from *Streptomyces azureus* ETH28555 sp. as shown in the example 4 and which encodes the whole peptide backbone of SEQ ID NO:6. The predicted size of the precursor preproprotein for thiostrepton is 60 amino acids and is depicted in SEQ ID NO:65.

In one embodiment, said nucleic acid of the invention comprises the nucleotide sequence of SEQ ID NO:15 encoding the 14 amino acids thiopeptide precursor of SEQ ID NO:1. In another embodiment, said nucleic acid of the invention comprises the nucleotide sequence of SEQ ID NO:16 encoding the 14 amino acids thiopeptide precursor of SEQ ID NO:3. The invention further includes any nucleotide sequence encoding the thiopeptide precursor of SEQ ID NO:1, SEQ ID NO:5 and SEQ ID NO:11 and any variant thereof, such as those precursor variants depicted in Table 1. Examples of such nucleotide sequences are depicted in SEQ ID NO:17 and 18 or SEQ ID NO:65, encoding respectively thiopeptide precursor protein for SEQ ID NO:1 and SEQ ID NO:3 and SEQ ID NO:6.

Methods to isolate and identify such nucleotide sequence of the invention from known thiopeptide producing strain starting from probes or primers derived of SEQ ID NO:15-18 are well known in the art and one example of such method is shown in Example 4. For example, genomic DNA of a thiopeptide producing strain can be amplified using primers flanking the region encoding the 14 amino acid precursor protein and determined from SEQ ID NO:17 or SEQ ID NO:18 as disclosed herein.

In another embodiment, said nucleic acid of the invention comprises the nucleotide sequence of SEQ ID NO: 17 or SEQ ID NO:18 encoding the 57 amino acid or 49 amino acid preproprotein respectively, or any fragment thereof comprising at least SEQ ID NO:15 or SEQ ID NO:16.

Also included are modifications of said nucleic acids. Such modifications include, for example, labels which are known in the art, methylation, and substitution of one or more of the naturally occurring nucleotides with a degenerate nucleotide. These modifications may be to increase expression, yield, and/or to improve purification in the selected expression systems, or for another desired purpose.

In one embodiment, the nucleic acid of the invention is operatively linked to heterologous transcriptional and translational control sequence. More preferably, it is an expression vector.

As used herein, the terms "expression vector" refer to the vehicle by which a nucleic acid can be introduced into a host cell, resulting in expression of the introduced sequence. In one embodiment, vectors comprise a promoter and one or more control elements (e.g., enhancer elements) that are heterologous to the introduced nucleic acid but are recognized and used by the host cell. In another embodiment, the sequence that is introduced into the vector retains its natural promoter that may be recognized and expressed by the host cell. In one embodiment, the vectors compatible with the present invention are the shuttle vectors pSET152, pOJ436, pOJ446 (Bierman et al. 'Plasmid cloning vectors for the conjugal transfer of DNA from Escherichia coli to Streptomyces spp.' Gene. 1992 Jul 1;116(1):43-9), pHM11a (Motamedi et al. 'Integrative vectors for heterologous gene expression in Streptomyces spp.' Gene. 1995 Jul 4;160(1):25-31) and pIJ8600 (Sun et al. 'Green fluorescent protein as a reporter for spatial and temporal gene expression in Streptomyces coelicolor A3(2).'Microbiology. 1999 Sep;145 ( Pt 9):2221-7) and their derivatives. In another embodiment, the vector is a cosmid.

A "promoter" or "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase. The promoter may be operatively associated with other expression control sequences, including enhancer and repressor sequences.

A common type of vector is a "plasmid", which generally is a self-contained molecule of double-stranded DNA (which may be circular), usually of bacterial origin, that can readily accept additional (foreign) DNA and which can readily be introduced into a suitable host cell. A plasmid vector often contains coding DNA and promoter DNA and has one or more restriction sites suitable for inserting foreign DNA. Recombinant cloning vectors will often include one or more replication systems for cloning or expression, one or more markers for selection in the host, e.g. antibiotic resistance, and one or more expression cassettes.

Vector constructs may be produced using conventional molecular biology and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, N.Y. (herein "Sambrook et al., 1989"); DNA Cloning: A Practical Approach, Volumes I and II (D. N. Glover ed. 1985); F. M. Ausubel et al. (eds.), Current Protocols in Molecular Biology, John Wiley & Sons, Inc. (1994). Alternatively, vector constructs could be partially or fully synthesized using DNA synthesis technology of a constructive biology company such as CodonDevices (Cambridge, MA, USA; http://www.codondevices.com/) or Blue Heron Biotechnology (Bothwell, WA, USA; http://www.blueheronbio.com/).

### 3. Core biosynthetic enzymes for producing core macrocycles from thiopeptide precursor

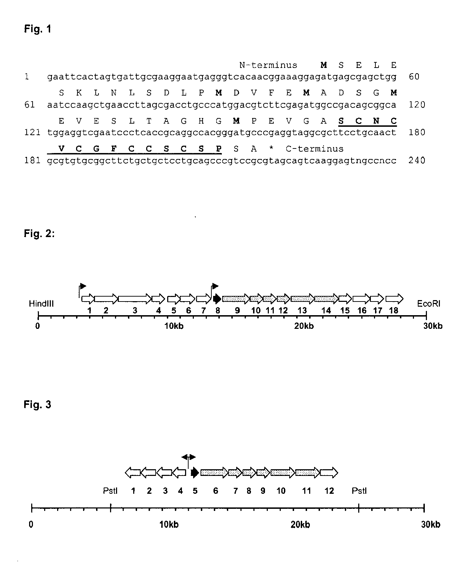

The characterization of two thiopeptide biosynthetic gene clusters from two distinct thiopeptide producing strains allows the inventors to identify genes that are highly conserved among the two strains, therefore, suggesting that these genes encode enzymes required for the synthesis of a core thiopeptide molecule from the thiopeptide precursor. Figures 2 and 3 show the position of Open Reading Frames (ORFs) of the biosynthetic gene cluster characterized from two distinct thiopeptide producing strains.

In another aspect, the invention relates to a polypeptide for the biosynthesis of thiopeptide, comprising an amino acid sequence selected among the group consisting of:
(i) any one of SEQ ID NO:23-34; and.
(ii) a variant of an amino acid sequence listed in (i), having no more than 1,2, 3, 4, 5, 6 or 10 deleted, inserted or substituted amino acids when compared to the corresponding wild type amino acid sequence listed in (i).

These polypeptides may be used either in vitro or in vivo for performing one or more reaction steps using the thiopeptide precursor protein as a starting material for the synthesis of a thiopeptide molecule. The variant polypeptide may retain substantially the same catalytic activity as the wild type corresponding sequence or have improved or modified catalytic activity. Solely for ease of reading, these polypeptides will be referred as the "core biosynthetic enzymes" in the text hereafter.

The following table 2 describes examples of core biosynthetic enzymes (SEQ ID NO:23-34) that can be used for thiopeptide biosynthesis and their proposed function in the biosynthetic pathway. The proposed function is further detailed below in Examples 5.1 and 5.2.

**Table 2: Examples of core enzymes for thiopeptide biosynthesis starting from thiopeptide precursor protein**

| **ORF#** | **Polypeptide SEQ ID** | **Nt SEQ ID** | **Coordinates of genomic BAC** | **Homology and Proposed function** |
|---|---|---|---|---|
| ORF9-I | NO:23 | NO:35 | 9646(GTG)-12129(TGA) of NO:62 | Lantibiotic dehydratase / Serine → |
| ORF6-II | NO:29 | NO:41 | 4866(TTG)→7479(TGA) of NO:63 | Dehydroalanine |
| ORF10-I | NO:24 | NO:36 | 12126(ATG)-13025(TGA) of NO:62 | Lantibiotic dehydratase / Serine → |
| ORF7-II | NO:30 | NO:42 | 7475(ATG)→8291(TGA) of NO:63 | Dehydroalanine |
| ORF11-I | NO:25 | NO:37 | 13025(ATG)-14026(TGA) of NO:62 | Lantibiotic biosynthesis |
| ORF8-II | NO:31 | NO:43 | 8287(ATG)→9247(TGA) of NO:63 | |
| ORF12-I | NO:26 | NO:38 | 14023(GTG)-15390(TGA) of NO:62 | Thiazoline oxidase / Subtilisin-like protease |
| ORF9-II | NO:32 | NO:44 | 9243(ATG)→10530(TGA) of NO:63 | |
| ORF13-I | NO:27 | NO:39 | 15378(ATG)-17204(TGA) of NO:62 | Conserved hypothetical protein |
| ORF10-II | NO:33 | NO:45 | 10526(TTG)→12419(TGA) of NO:63 | |
| ORF14-I | NO:28 | NO:40 | 17201(ATG)-18937(TGA) of NO:62 | Adenylation/ heterocyclisation |
| ORF11-II | NO:34 | NO:46 | 12411(ATG)→14256(TGA) of NO:63 | |

### 4. Genes encoding the core biosynthetic enzymes

The invention further provides nucleic acids of genes and/or open reading frames encoding the core biosynthetic enzymes. More specifically, the invention provides a nucleic acid comprising the nucleotide sequence encoding any one of the core biosynthetic enzymes as defined above.

The inventors were successful in identifying the structural genes in the genomes of Ef-tu inhibitor thiopeptide producing Nomomuraea *species,* which likely encode essential enzymes for the biosynthesis of corresponding Ef-tu inhibitors.

In one embodiment, said nucleic acid encoding an enzyme for the biosynthesis of Ef-tu inhibitors comprises a nucleic acid selected among the group consisting of ORF9, ORF10, ORF11, ORF12, ORF13 and ORF15 of the genomic fragment of SEQ ID NO:62 as described in Table 2. In another embodiment, said nucleic acid of the invention comprises a nucleic acid selected among the group consisting of ORF6, ORF7, ORF8, ORF9, ORF10 and ORF11 of the genomic fragment of SEQ ID NO:63 as described in Table 2. The nucleotide position (coordinates) of the ORF (Open Reading Frame) of each gene in the corresponding genomic fragment (from 5' to 3') are also reported in Table 2.

Methods to isolate and identify such nucleotide sequence of the invention, from known thiopeptide producing strains and using probes or primers derived of SEQ ID NO:35-46, are well known in the art. For example, genomic DNA of a thiopeptide producing strain can be amplified using primers flanking the region encoding the conserved region of each gene and determined from the disclosed nucleotide sequences.

Also included are modifications of said nucleic acids. Such modifications include, for example, labels which are known in the art, methylation, and substitution of one or more of the naturally occurring nucleotides with a degenerate nucleotide. These modifications may be useful to increase expression, yield, and/or to improve purification in the selected expression systems, or for another desired purpose.

In one embodiment, the nucleic acid of the invention is operatively linked to heterologous transcriptional and translational control sequence. More preferably, it is an expression vector suitable for expression in a host cell of whole or part of the core biosynthetic enzymes.

### 5. Production of thiopeptide precursor protein and corresponding thiopeptides:

The determination of a chromosomal encoded backbone and consequently a ribosomal route for thiopeptide biosynthesis by the inventors permits, in one embodiment, one of ordinary skill in the art to clone and express the thiopeptide biosynthesis pathway, i.e the biosynthesis gene cluster, and thus, produce a thiopeptide compound, in a modified host cell or a heterologous organism. The invention also permits the production of thiopeptide precursors to be expressed in a heterologous host cell, i.e., another strain, than the natural producing strain. Although the examples illustrate use of a bacterial strain, any organism or expression system can be used, as described herein. The choice of organism is dependent upon the needs of the skilled artisan. For example, a strain that is amenable to genetic manipulation may be used in order to facilitate modification and production of thiopeptide compounds.

Thus, in another aspect, the invention relates to a host cell comprising one or more nucleic acids as described above encoding thiopeptide precursor protein and/or core biosynthetic enzymes, wherein said nucleic acid is not naturally found in the genome of said host cell and/or said host cell does not naturally produce corresponding thiopeptide.

Alternatively, the production of thiopeptide or thiopeptide derivatives is rendered possible by providing a culture medium comprising an appropriate amount of thiopeptide precursor protein as defined above, and culturing microorganisms in said culture medium, wherein said microorganism further comprises other genes required for thiopeptide biosynthesis, for example, the genes encoding the core biosynthetic enzymes.

As used herein, the term "host cell" or "microorganisms" means any cell of any organism that is selected, modified, transformed, grown or used or manipulated in any way for the production of thiopeptide precursor or thiopeptides and their derivatives by the cell. For example, a host cell may be one that is manipulated to express a particular gene, a DNA or RNA sequence, a protein or an enzyme. Host cells can further be used for screening or other assays that are described infra. Host cells may be cultured in vitro or one or more cells in a non-human animal (e.g., a transgenic animal or a transiently transfected animal).

The host cell or microorganism itself may be selected from any biological organism, including prokaryotic (e.g., bacterial) cells, plant cells, and eukaryotic cells, including, insect cells, yeast cells and mammalian cells. Representative examples of appropriate host cells include bacterial cells, such as, *E. coli, Streptomyces* and *Bacillus subtilis* cells; fungal cells, such as yeast cells, such as Pichia or Saccharomyces cells, filamentous fungi such *Trichoderma* or *Aspergillus* cells; and insect cells such as *Drosophila* S2 and *Spodoptera* Sf9 cells. Preferably, said host cells are selected from host cells known to synthesize thiopeptide derivatives or described to be resistant to said thiopeptide such as strains of Streptomyces ramocissimus and *Streptomyces coelicolor* (Olsthoorn-Tieleman et al. 'Elongation factor Tu3 (EF-Tu3) from the kirromycin producer Streptomyces ramocissimus is resistant to three classes of EF-Tu-specific inhibitors.' J Bacteriol. 2007 May;189(9):3581-90.). In some embodiments, the host cell is selected from a strain which is rendered resistant to thiopeptides. Methods to render resistant a strain are well known in the art [Kieser T, Bibb MJ, Buttner MJ, Chater KF, Hopwood D. Practical Streptomyces Genetics. John Innes Foundation, Norwich (2000)] and an example of such method is given in the examples below.

In some embodiments of the production method, said host cell further comprises other genes required for thiopeptide biosynthesis. Other genes required for biosynthesis can include for example one or more genes encoding the core biosynthetic enzymes as described above. Said host cells are for example selected among the group of species consisting of *Nonomuraea* sp. , *Planobispora* sp., *Amycolatopsis* sp. and *Streptomyces* sp. Specific host cell organisms contemplated herein include, without limitation, organisms of the actinomycete suborder Streptosporangineae including the families Nocardiopsaceae, Streptosporangiaceae and Thermomonosporaceae , of which preferred genera include *Acrocarpospora, Actinomadura, Herbidospora, Microbispora, Microtetraspora, Nocardiopsis, Nonomuraea* ((Nonomuria sic, corrected by Chiba et al (1999) to Nonomuraea) a reclassified genus as reported by Zhenshui Zhang, Yue Wang and Jisheng Ruan in the International Journal of Systematic Bacteriology (1998), 48, 411-422)), *Planobispora, Planomonospora, Planopolyspora, Planotetraspora* or *Streptosporangium.* More generally, the terms are intended to encompass all organisms containing genetic information necessary to produce a thiopeptide compound. An example of such host cell includes Nonomuraea microbial strain Bp3714-39, a deposit of which was made on 2006-11-30, under Accession No. DSM 18831.

Suitable production techniques for protein such as the thiopeptide precursor protein of the invention are well known to those of skill in the art. See, e.g., Sambrook et al, Molecular Cloning: A Laboratory Manual, Cold Spring Harbor Press (Cold Spring Harbor, N.Y.). The sequences of any of the amino acid sequences provided herein can be readily generated using a variety of techniques. These and other suitable production methods are within the knowledge of those of skill in the art.

In one aspect, the amino acid sequences of the invention are produced by expression of one or more of the ORFs or genes in a selected host cell. The invention therefore relates to a method for producing a thiopeptide precursor protein, said method comprising the step of culturing a host cell capable of expressing a nucleic acid encoding a thiopeptide precursor protein as described above, and optionally, one or more nucleic acids encoding a core biosynthetic enzyme, under conditions appropriate for production of said thiopeptide precursor protein. In such method, said host cell is not the natural producing strain of said thiopeptide precursor protein, or when said host cell is the natural producing strain of said thiopeptide precursor protein, said nucleic acid encoding a thiopeptide precursor protein is a recombinant or heterologous nucleic acid. Depending on the host cell that is used, thiopeptide compound biosynthesis can be achieved by said host cell from the thiopeptide precursor. For that purpose, the person skilled in the art can either use a host cell that naturally synthesizes the enzymes required for post-translational modification of the thiopeptide precursor protein or introduce said genes required for post-translational modification for thiopeptide biosynthesis in the producing strain. In one specific embodiment, the methods defined above further comprise isolating said thiopeptide precursor or thiopeptide compound in a substantially pure form.

In a specific embodiment, the invention relates to the use of a host cell of the invention for the production of a thiopeptide compound selected among the group consisting of : GE2270A, GE37648A, Amythiamicin and the novel Ef-tu inhibitors as represented in formulas I to XI above, micrococcin, thiostrepton, nosiheptide, thiocillin, thiocins, nocathiacins, berninamycin, A10255B and radamycin.

In one aspect, the present invention provides methods of producing thiopeptide derivatives, comprising
i) synthesizing an altered thiopeptide precursor in a host cell by gene expression of the sequence encoding said altered thiopeptide precursor in said host cell,
ii) synthesizing said thiopeptide derivatives from said altered thiopeptide precursor; and/or,
iii) modifying said altered thiopeptide precursor by use of one or more of the core biosynthetic enzymes.

As used herein, an "altered thiopeptide precursor" is a thiopeptide precursor that is not naturally found in the strain producing it, i.e the producing strain.

Method for synthesizing altered thiopeptide precursor are further described below. Preferably, said altered thiopeptide derivative precursor is a variant of SEQ ID NO:1, SEQ ID NO:5 or SEQ ID NO:11 as described above. Step ii) is performed either *in vitro,* ie, outside of the host cells; or, *in vivo,* in the same host cell as step i). In the latter, one or more of the core biosynthetic enzymes are also synthesized by said host cells, either naturally or from recombinant DNA.

In a method of producing thiopeptide derivatives, the culture media inoculated with the host cell of the invention may be incubated under aerobic conditions using, for example, a rotary shaker or a stirred tank fermentor. Aeration may be achieved by the injection of air, oxygen or an appropriate gaseous mixture to the inoculated culture media during incubation. As soon as a sufficient amount of the thiopeptide derivatives have accumulated, they may be concentrated and isolated from the culture in conventional and usual manner, for example by extraction- and chromatographic methods, precipitation or crystallization, and/or in a manner disclosed herein. As an example for extraction, the culture can be mixed and stirred with a suitable organic solvent such as n-butanol, ethyl acetate, cyclohexane, n-hexane, toluene, n-butyl acetate or 4-methyl-2-pentanone, the thiopeptide derivatives in the organic layer can be recovered by removal of the solvent under reduced pressure. The resulting residue can optionally be reconstituted with for example water, ethanol, methanol or a mixture thereof, and re-extracted with a suitable organic solvent such as hexane, carbon tetrachloride, methylene chloride, dichloromethane or a mixture thereof. Following removal of the solvent, the compounds may be further purified for example by chromatographic methods. As an example for chromatography, stationary phases such as silica gel or aluminia oxide can be applied, with organic eluting solvents or mixtures thereof, including ethers, ketones, esters, halogenated hydrocarbons or alcohols, or reversed-phase chromatography on modified silica gel having various functional groups and eluting with organic solvents or aqueous mixtures thereof, like acetonitrile, methanol or tetrahydrofuran at different pH. Another example is partition-chromatography, for example in the solid-liquid or in the liquid-liquid mode. Also size exclusion chromatography may be applied, for example using Sephadex LH-20 (Sigma-Aldrich) and eluting with different solvents, preferably with alcohols.

As it is usual in this field, the production as well as the recovery and purification process may be monitored by a variety of analytical methods, including bioassays, TLC, HPLC or a combination thereof, and applying different detection methods, for TLC typically UV light, iodine vapor or spraying coloring reagents, for HPLC typically UV light, mass sensitive or light scattering methods. For example a HPLC technique is represented by using a reversed-phase column with a functionalized silica gel and applying an eluent which is a linear gradient mixture of a polar water miscible solvent and water at a specific pH, and a detection method with UV light at different wavelengths and a mass sensitive detector.

The resulting purified compounds are free of cells and cellular materials, by-products, reagents, and other foreign material as necessary to permit handling and formulating of the compound for laboratory and/or clinical purposes. It is preferable that purity of the compounds used in the present invention have a purity of greater than 80% by weight; more preferably at least 90% by weight, even more preferably greater than 95% by weight; yet even more preferably at least 99% by weight. In one embodiment, the invention provides compositions containing the compounds of the invention, regardless of how such compounds are produced.

The compounds biosynthesized by the host cells may optionally be subjected to random and/or directed chemical modifications to form compounds that are derivatives or structural analogs. The compounds may optionally be modified using methods known in the art and described herein.

### 6. Mutant microorganism capable of producing thiopeptide precursor protein for thiopeptide derivatives production

Following the teaching of the present invention, it is now possible to genetically manipulate microorganism capable of producing thiopeptide precursor protein, for example with the aim of improving thiopeptide production or modulating thiopeptide structure. Accordingly, in a further aspect, the invention provides a mutant microorganism, wherein said mutant microorganism has a mutation in the gene encoding the thiopeptide precursor protein and/or in one or more of the genes encoding core biosynthetic enzymes. Said mutation can be a single or multiple nucleotide deletion, insertion or substitution. It can also be a deletion of a fragment of the gene or the whole gene encoding the thiopeptide precursor protein. Preferably, the mutant microorganism no longer expresses the gene encoding the thiopeptide precursor protein of the invention when compared to the corresponding wild-type microorganism. Preferably, to avoid polar affects said mutation is an in-frame deletion within the gene encoding the thiopeptide precursor protein.

Said mutant organisms is for example of the actinomycete suborder Streptosporangineae including the families *Nocardiopsaceae, Streptosporangiaceae* and *Thermomonosporaceae,* of which preferred genera include *Acrocarpospora, Actinomadura, Herbidospora, Microbispora, Microtetraspora, Nocardiopsis,* Nonomuraea ((Nonomuria sic, corrected by Chiba et al (1999) to Nonomuraea) a reclassified genus as reported by Zhenshui Zhang, Yue Wang and Jisheng Ruan in the International Journal of Systematic Bacteriology (1998), 48, 411-422)), *Planobispora, Planomonospora, Planopolyspora, Planotetraspora* or *Streptosporangium.*

In a specific embodiment, said mutant microorganism is a *Nonomuraea* sp., for example, Nonomuraea microbial strain Bp3714-39, a deposit of which was made on 2006-11-30, under Accession No. DSM 18831, and said mutation is a disruption of the gene comprising SEQ ID NO:15 or SEQ ID NO:16, for example a mutation of the gene comprising SEQ ID NO:17 or SEQ ID NO:18.

In another particular embodiment, the mutant microorganism of the present invention can be further transformed with a nucleic acid encoding any of the thiopeptide precursor proteins as described above. This method allows to provide with microorganisms capable of thiopeptide biosynthesis from any thiopeptide precursor protein, including the variants of SEQ ID NO:1, SEQ ID NO5 or SEQ ID NO:11 as described above. In one further embodiment, said mutant microorganism further expresses either naturally or recombinantly at least one or more genes encoding the core biosynthetic enzymes, for example one or more genes encoding a polypeptide selected among SEQ ID NO:23-34. In one embodiment, the mutant microorganism expresses either naturally or recombinantly the genes encoding at least the polypeptides of SEQ ID NOs:23-28. In another embodiment, the mutant microorganism expresses either naturally or recombinantly the genes encoding at least the polypeptides of SEQ ID NOs:35-46.

### 7. Screening of novel strains producing thiopeptide compounds

The genes identified by the inventors can further be used as a tool to identify other microorganisms capable of producing thiopeptide derivatives. For example, the invention relates to any method that allows one to identify cells having a gene substantially similar to either,
(i) the gene of SEQ ID NO:17 or SEQ ID NO:18 and more preferably the fragment of SEQ ID NO:15 or SEQ ID NO:16, or
(ii) the core genes as defined in any of SEQ ID NOs:35-46.

In a specific embodiment, two DNA sequences are "substantially homologous" or "substantially similar" when at least about 80%, and most preferably at least about 90% or 95% of the nucleotides match over the defined length of the DNA sequences, as determined by sequence comparison algorithms, such as BLAST, FASTA, DNA Strider, etc. An example of such a sequence is an allelic or species variant of the specific genes of the invention. Sequences that are substantially homologous can be identified by comparing the sequences using standard software available in sequence data banks, or in a Southern hybridization experiment under, for example, stringent conditions as defined for that particular system.

The terms "sequence identity" "percent sequence identity" or "percent identical" in the context of nucleic acid sequences refers to the residues in the two sequences which are the same when aligned for maximum correspondence. The length of sequence identity comparison may be over the full-length of the genome, the full-length of a gene coding sequence, or a fragment of at least about 500 to 5000 nucleotides, is desired. However, identity among smaller fragments, e.g. of at least about nine nucleotides, usually at least about 20 to 24 nucleotides, at least about 28 to 32 nucleotides, at least about 36 or more nucleotides, may also be desired. Similarly, "percent sequence identity" may be readily determined for amino acid sequences, over the full-length of a protein, or a fragment thereof. Suitably, a fragment is at least about 8 amino acids in length, more preferably at least about 14 amino acids, and may be up to about 700 amino acids. Examples of suitable fragments are described herein.

In one embodiment, the method to identify cells capable of producing thiopeptide derivatives, comprises the following steps of
(i) incubating genomic DNA or RNA from isolated cells with a nucleic acid probe of SEQ ID NO:15 or SEQ ID NO:16 or specific fragments thereof for specific hybridization of the probe to homologous DNA region; and,
(ii) identifying the cells which comprise genomic DNA region or RNA that hybridizes specifically with said probe according to step (i).

A nucleic acid molecule is "hybridizable specifically" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., Molecular Cloning: A Laboratory Manual, Second Edition (1989) Cold Spring Harbor Laboratory Press, Cold Spring Harbor, N.Y. (herein "Sambrook et al., 1989"). The conditions of temperature and ionic strength determine the "stringency" of the hybridization. For preliminary screening for homologous nucleic acids, low stringency hybridization conditions, corresponding to a Tm (melting temperature) of 55° C., can be used, e.g., 5×SSC, 0.1% SDS, 0.25% milk, and no formnamide; or 30% formamide, 5×SSC, 0.5% SDS). Moderate stringency hybridization conditions correspond to a higher Tm, e.g., 40% formamide, with 5× or 6×SCC. High stringency hybridization conditions correspond to the highest Tm, e.g., 50% formamide, 5x or 6×SCC. SCC is a 0.15M NaCl, 0.015M Na citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., supra, 9.50-9.51). For hybridization with shorter nucleic acids, i.e. , oligonucleotides, the position of mismatches becomes more important, and the length of the oligonucleotide determines its specificity (see Sambrook et al., supra, 11.7-11.8). A minimum length for a hybridizable nucleic acid is at least about 10 nucleotides; preferably at least about 15 nucleotides; and more preferably the length is at least about 20 nucleotides.

In a specific embodiment, standard hybridization conditions are used. The term "standard hybridization conditions" refers to a Tm of 55° C., and utilizes conditions as set forth above. In a preferred embodiment, the Tm is 60° C.; in a more preferred embodiment, the Tm is 65° C. In a specific embodiment, "high stringency" conditions are used.

Suitable hybridization conditions for oligonucleotides (e. g., for oligonucleotide probes or primers) are typically somewhat different than for full-length nucleic acids (e.g., full-length cDNA), because of the oligonucleotides' lower melting temperature. Because the melting temperature of oligonucleotides will depend on the length of the oligonucleotide sequences involved, suitable hybridization temperatures will vary depending upon the oligoncucleotide molecules used. Exemplary temperatures may be 37° C. (for 14-base oligonucleotides), 48° C. (for 17-base oligoncucleotides), 55° C. (for 20-base oligonucleotides) and 60° C. (for 23-base oligonucleotides). Exemplary suitable hybridization conditions for oligonucleotides include washing in 6×SSC/0.05% sodium pyrophosphate, or other conditions that afford equivalent levels of hybridization.

In one preferred embodiment of the method, oligonucleotide specific of the gene encoding thiopeptide precursor are designed and used for identification of novel cells capable of producing thiopeptide compounds. Such oligonucleotides can be used for PCR amplification of a fragment of the gene encoding thiopeptide precursor protein. Preferably, lower eukaryotic cells are screened, and more preferably cells from actinomycetes species.

### 8. Genetic manipulation of gene encoding thiopeptide precursor protein and use for screening novel thiopeptide derivatives

In another embodiment, the present invention provides methods of modifying one or more of the nucleotide sequence of the genes and/or open reading frames encoding thiopeptide precursor protein. For example, such modifications or alterations can be for the purpose of improving expression in a selected expression system or generating novel thiopeptide derivatives. Other alterations can be made to extinguish, modify, or enhance function of thiopeptide compounds, including improving antibiotic function or decreasing undesired properties.

In one aspect, synthesis of an altered thiopeptide precursor is achieved from modification of the nucleic acid sequence encoding thiopeptide precursor protein. In one embodiment, the altered nucleic acid sequences can be provided to a heterologous host cell as described above via a suitable vector in a selected host cell and used to express the corresponding product. Alternatively, the alteration can be made directly in the natural gene carried by strain producing thiopeptide, for example, by genetic manipulation of said strain.

The present invention contemplates any method of altering any of the nucleic acid sequences encoding the precursor proteins of the present invention. More specifically, the invention contemplates any method that inserts amino acids, deletes amino acids or replaces amino acids in the proteins of the invention. The modifications may be performed at the nucleic acid level. These modifications are performed by standard techniques and are well known within the art.

Accordingly, the present invention provides a method to generate a nucleic acid library encoding precursor of thiopeptide derivatives, said method comprising the steps of generating a variety of nucleic acid with altered nucleotide sequence by performing nucleotide substitution in at least one codon of the sequence encoding of the sequence encoding any of SEQ ID NO:1-14, for each nucleic acid. Such nucleic acid library can be used advantageously to screen for novel thiopeptide derivatives, for example, Ef-tu inhibitor, with improved properties.

The altered nucleic acids or nucleic acid library can then be used to transform host cells for thiopeptide production as described above.

Preferably, each nucleic acid of the nucleic acid library has a single nucleotide substitution so that only one codon of the sequence encoding any of SEQ ID NO:1-14 is mutated compared to wild type corresponding sequence. Method to generate site-directed mutagenesis or nucleic acid libraries are well known in the art and for example described in an article by Hogrefe et al published in Biotechniques ('Creating randomized amino acid libraries with the QuikChange Multi Site-Directed Mutagenesis Kit.' 2002 Nov;33(5):1158-60, 1162, 1164-5).

In one specific embodiment, said nucleotide substitution is performed in one or more of the codons encoding amino acid residues of positions 2, 5, 7, 8, 14 of any of SEQ ID NO:1-4.

Preferably the host cells are capable of synthesizing thiopeptide compounds, ie, further comprising other genes required for thiopeptide biosynthesis. For example, other genes required for thiopeptide biosynthesis may comprise one or more genes encoding a polypeptide selected among SEQ ID NO:23-34. More preferably, the altered nucleic acids are transformed with an expression vector, so that, corresponding thiopeptide precursor protein is synthesized after transformation from said expression vector. The resulting expression library can be used as a tool to screen novel thiopeptide derivatives, for example, novel Ef-tu inhibitors.

### 9. Cloning of tailoring biosynthetic genes

When characterizing the biosynthetic gene clusters from two strains producing distinct thiopeptide compounds, the inventors identified ORFs that are likely involved in thiopeptide biosynthesis but that are strain-specific. It is therefore proposed that these genes likely encode tailoring polypeptides, mainly enzymes and transcriptional regulators, involved in gene regulation and enzymatic modification of the core thiopeptide structure to produce the final strain-specific thiopeptide compounds.

The Tables 3 and 4 describes the nucleic acid and corresponding polypeptide sequences of these tailoring polypeptides, according to the invention.

**Table 3: Examples of tailoring polypeptides for thiopeptide biosynthesis from Strain I**

| **ORF#** | **Polypeptide SEQ ID** | **Coordinates of genomic BAC** | **Homology and Proposed function** |
|---|---|---|---|
| ORF1-I | NO:47 | 1(GTG)-714(TGA) of NO:62 | Transcriptional regulator / Regulation gene expression |
| ORF2-I | NO:48 | 711(GTG)-2612(TGA) of NO:62 | Adenylation-heterocyclization / Oxazoline formation in Tail |
| ORF3-I | NO:49 | 2609(GTG)-5098(TGA) of NO:62 | Lantibiotic dehydratase / Serine -> Dehydroalanine |
| ORF4-I | NO:50 | 5095(GTG)-6129(TAG) of NO:62 | Cytochrome P450 monooxygenase / Hydroxylation |
| ORF5-I | NO:51 | 6218(ATG)-6904(TGA) of NO:62 | ? |
| ORF6-I | NO:52 | 6947(ATG)-8065(TGA) of NO:62 | N-Methytase / Methylation of asparagine |
| ORF7-I | NO:21 | 8094(ATG)-9338(TGA) of NO:62 | Coproporphyrinogen III oxidase / Methylation of thiazole |
| ORF15-I | NO:53 | 23718 (ATG)- 25454 (TGA) of NO:62 | ? |
| ORF16-I | NO:54 | 19327(ATG)- 20577(TGA) of NO:62 | Coproporphyrinogen III oxidase / Methlyation (methoxy formation) |
| ORF17-I | NO:55 | 20579(GTG)- 21598(TGA) of NO:62 | O-methylase/ Methlyation (methoxy formation) |
| ORF18-I | NO:56 | 22234(ATG)- 22848(TAG) of NO:62 | Deaminase Reductase / Removal N-terminus - |

| | | | Pyridine |
|---|---|---|---|
| Table 4: Examples of tailoring polypeptides for thiopeptide biosynthesis from Strain II | | | |
| **ORF#** | **Polypeptide SEQ ID** | **Coordinates of genomic BAC** | **Homology and Proposed function** |
| ORF1-II | NO:47 | 1(TAG)←1213(ATG) of NO:63 | Coproporphyrinogen III oxidase / Methylation of thiazole |
| ORF2-II | NO:48 | 1209(TGA)←2373(ATG) of NO:63 | Cytochrome P450 monooxygenase / hydroxylation |
| ORF3-II | NO:49 | 2369(TGA)←3590(GTG) of NO:63 | Cytochrome P450 monooxygenase / hydroxylation |
| ORF4-II | NO:50 | 3600(TGA)←4332(ATG) of NO:63 | Transcriptional regulator / regulation gene expression |
| ORF12-II | NO:51 | 14252(ATG→15587(TGA) of NO:63 | Peptidase / Amidohydrolase / removal C-terminal alanine |

In one embodiment, one or more of the polypeptides listed in Table 3 or 4 are used for *in vitro* or *in vivo* synthesis of a compound of formula (I) to (XI) as defined above.

The invention relates also to any functional variant of such enzymes reported in Tables 3 or 4, retaining substantially the same enzymatic activity. The invention also relates to any functional variant of any transcriptional regulator reported in Tables 3 or 4, retaining substantially the same transcriptional activity. In one embodiment, such polypeptide contains not least than 1, 2, 3, 4 or 5 amino acids deleted, inserted or substituted when compared to the original polypeptide listed in the table above. In another embodiment, such functional variants are at least 80 or 90% identical to one of the polypeptide listed above.

These sequences can be used to enable the generation of mutant strains that lack specific tailoring steps, for example, to avoid the production of undesired side compounds. In one embodiment, the invention relates to a mutant strain capable of producing thiopeptide that is deficient in the expression of one or more of the tailoring genes listed in the table above. In one specific embodiment, the mutant strain is deficient in the expression of a gene encoded by ORF2-II or ORF3-II (respectively SEQ ID NO:58 and SEQ ID NO:59) or ORF4-I (SEQ ID NO:50).

As used herein, "deficient expression" means that the corresponding polypeptide is no longer expressed by the mutant strain when compared to the wild type strain, or at least has steady state mRNA amounts decreased by more than 50% or more than 90%, compared to wild type strain steady-state mRNA as measured in conventional methods for quantifying mRNA expression. For example, the gene is disrupted or partially or fully deleted so that a functional protein is no longer synthesized.

These sequences can further be used in combination with the gene encoding precursor protein and genes encoding core enzymes to engineer host cells capable of producing specific thiopeptide derivatives. In one embodiment, the invention relates to a host cell that contains recombinant open reading frames ORF1-II to ORF12-II, as described in Tables 2, 3 and 4, or functional variants thereof having at least 80%, or at least 90% identity to the corresponding wild type sequence, and capable of producing an EF-Tu inhibitor. In another embodiment, the invention relates to a host cell that contains recombinant ORF1-I to ORF18-I as described in Tables 2, 3 and 4 or functional variants thereof having at least 80%, or at least 90% identity to the corresponding wild type sequence, and capable of producing an EF-Tu inhibitor.

In another embodiment, the isolated tailoring enzymes can be used either individually or in combination as a catalyst in a chemical reaction step in an in vitro method, for example using a thiopeptide precursor protein as a starting material, for example, in a method for producing a thiopeptide compound, for example, in a method for producing an E-FTu inhibitor.

### BRIEF DESCRIPTION OF THE FIGURES

**Fig 1****. Thiopeptide structural gene.** Potential initiation sites in bold. The 14 amino-acid backbone is underlined.
**Fig. 2****. Biosynthetic gene cluster from thiopeptide producing Strain I.** Arrows represent hypothetical promoters. Open arrows represent open reading frames: the black filled arrow indicates the seriesl structural gene and grey shaded arrows are syntenous genes shared by both the seriesI and seriesII thiopeptide gene clusters. Hindlll and EcoRI are unique restriction sites flanking the cluster.
**Fig. 3** **Biosynthetic gene cluster from thiopeptide producing Strain II (strain Bp3714-39).** Arrows represent hypothetical divergent promoters. Open arrows represent open reading frames: The black filled arrow indicates the seriesll structural gene and grey shaded arrows are syntenous genes shared by both the seriesI and seriesII thiopeptide gene clusters. The gene cluster is flanked by Pstl restriction sites..

### EXAMPLES

The invention is also described by means of particular examples. However, the use of such examples is illustrative only and in no way limits the scope and meaning of the invention or of any exemplified term.

### 1. Identification of a small structural gene in the genome of the thiopeptide producing Nonomuraea sp., which encoded the whole peptide backbone

A PCR method was employed to isolate a chromosomal sequence that encodes for the thiopeptide backbone. Genomic DNA was purified from the thiopeptide producing *Nonomuraea* strain originating from Hubei province, China and digested with the restriction enzyme *Nar*I*.* The digested chromosomal DNA was purified by passing it over a QiaQuick DNA clean-up column (Qiagen). An adaptor-ligated library was generated by ligating to the fragmented DNA a 500-fold molar excess of the following adapter: 5'-CGACCACGACCA (phosphorylated on the 5'terminus and includes a 3' C6-TFA-amino modification) and 5'-AGTCTCGCAGATGATAAGGTGGTCGTGGT. The thiopeptide structural gene was amplified from this library by using the adapter primer (5'-GTCCAGTCTCGCAGATGATAAGG) and a degenerate primer designed on the thiopeptide macrocycle (CFGCVCNC: 5'-CARAAICCRCAIACRCARTTRCA). Inosine was included in the oligonucleotide to reduce the degeneracy. HotStar polymerase mix (Qiagen) was used to obtain a specific PCR product with the cycling conditions as follows: 95°C for 15 min; 30 cycles of 94°C for 30 sec, 55°C for 30 sec, and 72°C for 1 min; and 72°C for 10 min. The 3'amino-modification on the adaptor blocks extension and prevents adaptor-adaptor amplification by the adaptor primer. Amplification can only take place if the degenerate oligonucleotide anneals and primes a polymerase extension that generates the complementary sequence for the adaptor primer.

Experiments were successful in identifying a small structural gene in the genome of the thiopeptide producing *Nonomuraea sp.,* which encoded the whole peptide backbone (Fig 1). The predicted size of this precursor protein is 57amino-acids. However, there are a number of initiation codons that may indicate alternative translation start sites. The 14 amino-acid thiopeptide sequence is at the C-terminus and establishes the direction of synthesis. The primary amino acid sequence of the thiopeptide backbone begins with a serine that is incorporated into the pyridine ring and continues in an anticlockwise direction around the macrocycle and finishes with the final amino-acid at the end of the side-chain. Homology searches in the public databases revealed no close homolog.

### 2. Genetic disruption of the gene encoding thiopeptide precursor protein

The suicide vector pSET152-Hind can replicate in *E.coli,* confers apramycin resistance and carries an origin of transfer (*oriT*) that allows intergeneric conjugation from *E.coli* into actinomycete species. pSET152-Hind is a derivative of the broad host range vector pSET152. The gene that allows site-specific intergration *(int)* was deleted from pSET152 by removing a *Hind*III fragment. The gene encoding the thiopeptide precursor protein may be disrupted in either of two ways; insertional inactivation or deletion. The first method requires that an internal fragment of the gene without an initiation or stop codon be cloned into pSET152-Hind. This plasmid may then be introduced into the thiopeptide producing strain by conjugation. Selection for apramycin will identify mutants that have the vector backbone inserted into the thiopeptide structural gene. Such an event will inhibit transcription and translation and prevent the production of thiopeptide. The second method requires a mutant allele be constructed in pSET152-Hindlll and then transferred into the producing strain. The mutant allele will contain the sequence upstream and downstream the structural gene but will have the open reading frame deleted preferably in-frame. This deletion may be marked/replaced with a gene that confers resistance to an antibiotic such as hygromycin or thiostrepton. On conjugation into the producing strain selection for the plasmid antibiotic marker will select for strains that have both the wild type and mutant allele in the chromosome. Merodiploidy results from homologous recombination between the plasmid and the chromosome in either the upstream or downstream sequence. Selection for the loss of the vector antibiotic marker and subsequent PCR screening for deletion or antibiotic selection for the marked mutant allele will identify the desired second recombination event, which will remove the wild type allele and leave the deleted allele of the structural gene.

Alternatively, advances in DNA synthesis technology now allow the synthetic build-up of large pieces of DNA and such services are commercially available. The thiopeptide gene cluster maybe reengineered by de *novo* chemical synthesis for heterologous expression and thiopeptide production in a surrogate host. Such a host will have well established genetic tools eg. *Streptomyces coelicolor* or *Streptomyces lividans* and must be thiopeptide resistant or be rendered thiopeptide resistant. Resistant strains are isolated by plating 10¹⁰ to 10" cells/spores on agar plates containing concentrations of thiopeptide in excess of the thiopeptide minimum inhibitory concentration. Rare spontaneous mutants pre-existing in the population conferring resistance will be identified by colonial growth on the selection plates. The frequency of mutation rate may be increased by exposing cells to chemical mutagen. Chemical synthesis of the thiopeptide gene cluster permits the introduction of better regulatory elements, deletion of genes, removal or introduction of restriction sites and alterations in codon usage. A functional synthetic copy of the gene cluster cloned on the integrative shuttle vector pOJ436 or episomal shuttle vector pOJ446 will have restriction sites introduced into the structural gene to permit the generation of an in-frame deletion.

In addition, a cloned copy of the thiopeptide gene cluster or synthetic version may be precisely manipulated by homologous recombination in strains of E.coli which express phage-derived protein pairs, either RecE/RecT from the Rac prophage, or Redα/Redβ from λ phage. This technique is referred to as Red/ET Recombineering or λ-mediated recombination (Muyrers, J.P.P., Zhang, Y., Stewart, A.F. ET cloning: Think recombination first. Genetic Engineering, Principles and Methods (Ed. J.K. Setlow), 22, 77-98 Kluwer Academic/Plenum Publishers, NY. (2000)).

### 3. Heterologous expression of the structural genes to generate alternative thiopeptide structures.

A native producing strain or heterologous host expressing the thiopeptide core and tailoring biosynthetic genes but with an in-frame deletion in the precursor structural gene are useful tool strains. These tool strains may be used to produce thiopeptides with alternative structures

Site-directed mutagenesis of the structural gene may be employed to replace or introduce new amino acids into the thiopeptide. The mutated version of the structural gene cloned in pHM11a or pSET152 is reintroduced by conjugation or transformation into the tool strain expressing the thiopeptide biosynthetic enzymes.

Alternatively, a library can be created encoding alternative amino acids in every position of the thiopeptide backbone. This variant library can be generated chemically by gene synthesis or by degenerate PCR. The PCR method requires amplification of a thiopeptide structural gene to incorporate variations in the thiopeptide encoding backbone and restriction sites for cloning and expression in a plasmid such as pHM11a or pSET152. One of the PCR primers will be degenerate to incorporate all amino acid substitutions. The degree of degeneracy can altered to allow substitutions in selected positions of the backbone ie. not in positions encoding amino acids deemed invariant such as the cysteines that form the thiazoles of the thiopeptide macrocycle. Both primers will be tagged with restriction sites to allow direct cloning of the PCR product into the expression vector. All E.coli transformants will be pooled and DNA isolated to generate the variant library. The library will be transformed into the tool strain and a bioassay may be employed to identify clones harboring structural genes that support production of active thiopeptides with alternative structures.

### 4. Isolation of the gene encoding thiostrepton backbone polypeptide

A PCR method was employed to isolate a chromosomal sequence that encodes for the thiostrepton backbone. Genomic DNA was purified from the thiostrepton producing strain *Streptomyces azureus* ETH28555 and digested with the restriction enzyme *Nar*I. The digested chromosomal DNA was purified by passing it over a QiaQuick DNA clean-up column (Qiagen). An adaptor-ligated library was generated by ligating to the fragmented DNA a 500-fold molar excess of the following adapter: 5'-CGACCACGACCA (phosphorylated on the 5'terminus and includes a 3' C6-TFA-amino modification) and 5'-AGTCTCGCAGATGATAAGGTGGTCGTGGT. The thiostrepton structural gene was amplified from this library by using the adapter primer (5'-GTCCAGTCTCGCAGATGATAAGG) and a degenerate primer designed on the thiostrepton macrocycle (CTTCICTC: 5'-CAC GTG CAG ATR CAN GTN GTR CA-3'). The degenerate primer was designed based on the CODEHOP principles with a 5' consensus clamp and a 3' degenerate core (Rose et al. CODEHOP (COnsensus-DEgenerate Hybrid Oligonucleotide Primer) PCR primer design. Nucleic Acids Res. 2003 Jul 1;31(13):3763-6). HotStar polymerase mix (Qiagen) was used to obtain a specific PCR product with the cycling conditions as follows: 95°C for 15 min; 40 cycles of 94°C for 30 sec, 55°C for 30 sec, and 72°C for 1 min; and 72°C for 10 min. The 3'amino-modification on the adaptor blocks extension and prevents adaptor-adaptor amplification by the adaptor primer. Amplification can only take place if the degenerate oligonucleotide anneals and primes a polymerase extension that generates the complementary sequence for the adaptor primer. This strategy proved successful in identifying a genomic fragment that encoded the thiostrepton macrocycle. Gene specific primers were then used to walk both up and downstream to identify the full thiostrepton structural gene.

### 5. Biosynthetic gene clusters from thiopeptide producing Strains I and II

### 5.1 Strain I

Figure 2 describes the ORFs positions in one isolated BAC (SEQ ID NO:62) from Strain I genomic DNA comprising the biosynthetic gene cluster for thiopeptide synthesis.

Without being bound by any preferred model, the following pathway defines putative function of the individual polypeptides characterized from the cloned biosynthetic gene clusters in the synthesis of thiopeptide derivatives.

Synthesis Scheme for SeriesI:
(A) ORF9, ORF10, ORF11, ORF12, ORF13 and ORF 14 encode the core biosynthetic enzymes that likely forms a complex, which functions processively by binding the precursor peptide. The corresponding modifications are introduced as the complex moves along the peptide. Thiazoles are introduced by cyclodehydration of the cysteine thiol group with the preceding carbonyl and subsequent oxidation of thiazoline ring. Dehydration of two serine residues form the dehydroalanine residues that are the substrates for the aza-Diels-Alder cycloaddition reaction that yields the central pyridine heterocycle. 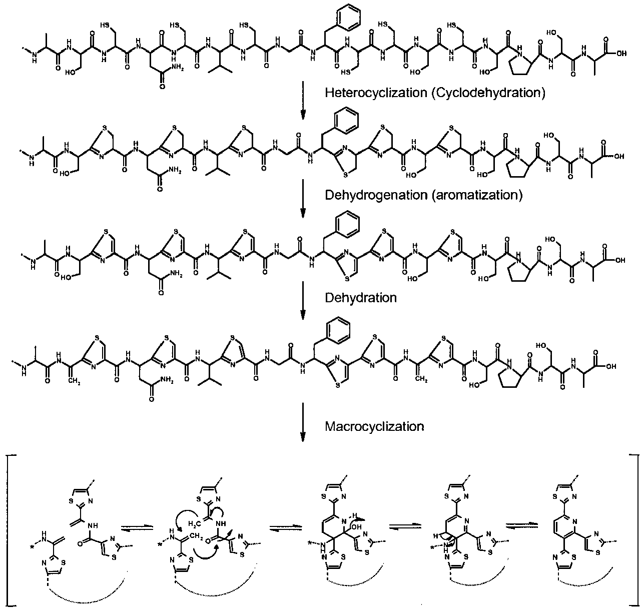
(B) ORF2 and ORF3 encode enzymes likely involved in the modifications incorporated into the tail. Cyclodehydration of a serine residue yields an oxazoline ring. Dehydration of the additional serine in the tail to dehydroalanine may require a separate step due to the presence of a proline in the tail, which likely causes a conformational kink in the peptide. ORF18 may be involved in elimination of terminal sequence leaving amide group. 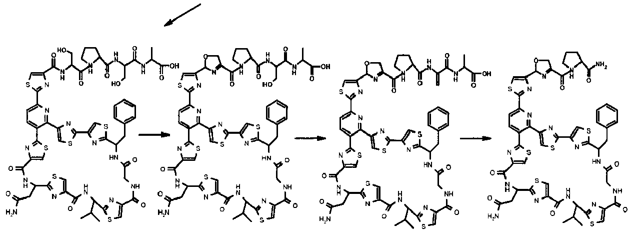
(C) ORF4, ORF6, ORF7, ORF16 and ORF17 encode enzymes likely involved in tailoring modifications. ORF4: hydroxylation of phenylalanine. ORF5: methylation of asparagine. ORF7: methylation of thiazole. ORF16 and ORF17: addition of methoxyethyl group to thiazole.

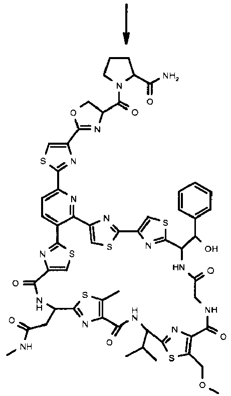

### 5.2 Strain II

Figure 3 describes the ORFs positions in one isolated BAC (SEQ ID NO:63) from Strain II genomic DNA comprising the biosynthetic gene cluster for thiopeptide synthesis.

Without being bound by any preferred model, the following pathway defines putative function of the individual polypeptides characterized from the cloned biosynthetic gene clusters in the synthesis of thiopeptide derivatives.

Synthesis Scheme for SeriesII:
(A) ORF6, ORF7, ORF8, ORF9, ORF10 and ORF 11 encode the core biosynthetic enzymes that likely forms a complex, which functions processively by binding the precursor peptide. The corresponding modifications are introduced as the complex moves along the peptide. Thiazoles are introduced by cyclodehydration of the cysteine thiol group with the preceding carbonyl and subsequent oxidation of thiazoline ring. Dehydration of two serine residues form the dehydroalanine residues that are the substrates for the aza-Diels-Alder cycloaddition reaction that yields the central pyridine heterocycle. 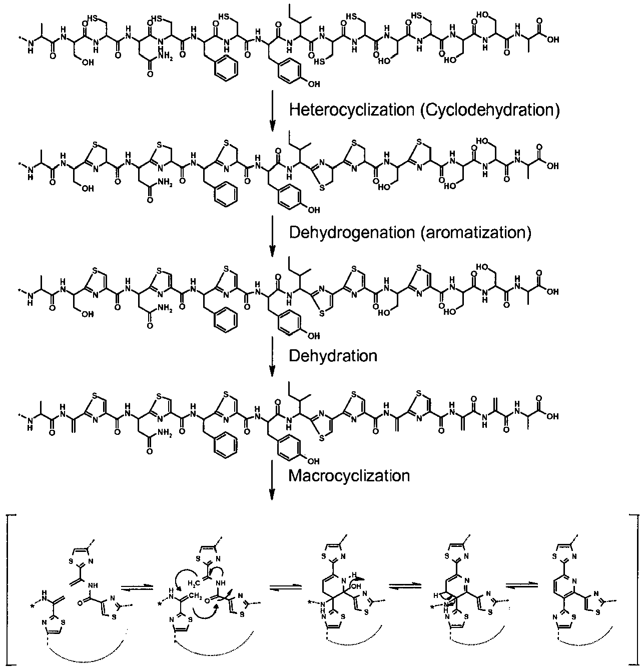
(B) ORF 12 encodes a peptidase that cleaves the terminal alanine 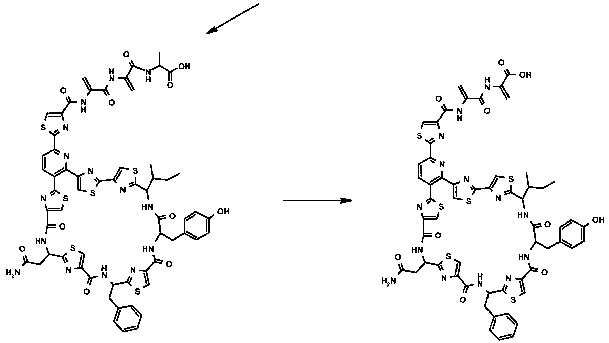
(C) ORF1, ORF2 and ORF3 encode enzymes likely involved in tailoring modifications. ORF1 methylation of thiazole with ORF2 and ORF3 involved in the hydroxylation of phenylalanine and isoleucine. The isoleucine is hydroxylated twice and decomposes to generate an epoxide.

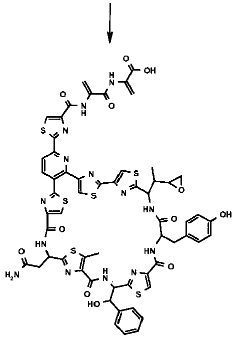

### 6. Production of thiopeptide derivatives

Media composition

| | *(a) Seed medium* |
|---|---|
| Substance | Concentration [g/l] |
| Agar | 1 |
| Trace solution | 1 mL |
| Glycerol | 7.5 |
| NaCl | 0.05 |
| CaCO₃ | 0.05 |
| KH₂PO₄ | 0.25 |
| K₂HPO₄ | 0.5 |
| MgSO₄ x 7 H₂O | 0.1 |
| Yeast Extract | 1.35 |
| N-Z Amine A | 2.5 |
| Malt extract | 5.85 |
| L (-) Asparagine x 1 H₂O | 1 |
| Soy protein | 2.5 |
| Starch | 7.5 |
| Glucose | 7.5 |
| Adjust to pH 7 | |
| | |

| | *(b) Production medium A* |
|---|---|
| Substance | Concentration [g/l] |
| Agar | 1 |
| Trace solution | 1 mL |
| Glycerol | 7.5 |
| NaCl | 0.05 |
| CaCO₃ | 0.05 |
| KH₂PO₄ | 0.25 |
| K₂HPO₄ | 0.5 |

| Substance | Concentration [g/l] |
|---|---|
| MgSO₄ x 7 H₂O | 0.1 |
| Yeast Extract | 1.35 |
| N-Z Amine A | 2.5 |
| Malt extract | 5.85 |
| L (-) Asparagine x 1 H₂O | 1.0 |
| Soy protein | 2.5 |
| Starch | 7.5 |
| Glucose | 7.5 |
| Antifoam emulsion | 0.2 mL |
| Adjust to pH 7 | |
| | |

| | *(c) Trace solution* |
|---|---|
| Substance | Concentration [g/l] |
| ZnSO₄ x 7 H₂O | 4 |
| Boric acid H₃BO₃ | 0.1 |
| FeSO₄ x 7 H₂O | 5 |
| KJ | 0.05 |
| CoCl₂ x 6 H₂O | 2 |
| CuSO₄ x 5 H₂O | 0.2 |
| MnCl₂ x 4 H₂O | 2 |
| H₂SO₄ 95 - 97 % | 1 mL |

A frozen suspension (1.5 mL) of a host cell as described in example 3 is inoculated into a two liter non-baffled shake flask containing 500 mL of seed medium. The flask is incubated for 3 days at 30°C on a rotary shaker at 200 rpm and with 50 mm amplitude. The second seed stage is developed by inoculating 40 mL each, of the first stage seed into eight two liter non-baffled shake flasks each containing 500 mL of seed medium. The flask is incubated for 2 days at 30°C on a rotary shaker at 200 rpm and with 50 mm amplitude. A third seed stage is developed by inoculating 4 liters each, of the second stage seed into two 150 liter scale stirred tank fermentors containing each 100 liters of seed medium. The 150 liter scale fermentors are operated for 3 days with the following parameters: Temperature = 30°C, agitation = 80 rpm, airflow = 25 slpm, and pressure = 0.5 bar. Excess foam formation is prevented by controlled addition of silicon oil-based antifoam agent. pH is monitored but not controlled.

A 5500 liter scale stirred tank fermentor containing 3500 liters of production medium A is inoculated with 200 liters from the third seed stage. Operating parameters of the 5500 liter scale fermentor are: Temperature = 30°C, airflow = 1050 slpm, and pressure = 0.5 bar. Agitation is controlled at 60 rpm and, after 44 hours, increased to 80 rpm. Excess foam formation is prevented by controlled addition of silicon oil-based antifoam agent. pH is monitored but not controlled. The fermentor containing 3500 liters of broth is harvested after 5 days of incubation.

### 7. Isolation of thiopeptide derivatives

The fermentation broth are harvested and extracted over night in a stirred tank by addition of ethyl acetate. During extraction the mixture is passed through a continuous Dispax^{®} reactor (Jahnke & Kunkel, Germany) for maximum sheer force and optimal mixing. After separating the two phases on a continuous Westfalia separator SA20 (Westfalia Separator AG, Oelde, Germany) the ethyl acetate phase is concentrated by evaporation under reduced pressure. During evaporation a precipitate is formed which is separated by filtration.

The precipitate obtained from the extraction of the culture broth according to the procedure described above is dissolved in Dioxane/water 95:5 and filtered to remove insoluble ingredients. The filtrate is concentrated under reduced pressure in the presence of diatome 8 (Isolute®, International Sorbent Technology Ltd., Hengoed Mid Glam, UK) The obtained powder is applied to a chromatographic column of silica gel (e.g. 0.040-0.063 mm, column size 5x25 cm) prepared in dichloromethane/methanol/acetic acid 90:10:0.5. The column is developed with a mixture of dichloromethane/methanol/acetic acid 90:10:0.5 at a flow rate of 35 mL/min. Fractions of 30 mL are collected, which are analyzed by HPLC. To the pooled fractions containing compound I 20 mL Isopropanol is added and concentrated under reduced pressure until the compound precipitates from the remaining Isopropanol. After separating of the solvent from the precipitate through centrifugation the residue is dried under reduced pressure yielding semi-purified thiopeptide derivatives.

## Claims

1. A thiopeptide precursor protein comprising an amino acid sequence selected among the group consisting of
(i) SEQ ID NO:1;
(ii) SEQ ID NO:5;
(iii) SEQ ID NO:11;
(iv) or a variant of said amino acid sequences having no more than 1, 2, 3, 4, 5, 6 or 10 deleted, inserted and/or substituted amino acids when compared to SEQ ID NO:1,SEQ ID NO:5 or SEQ ID NO:11.

2. The thiopeptide precursor protein of Claim 1, wherein said precursor is a biosynthetic precursor for an Ef-tu thiopeptide inhibitor.

3. The thiopeptide precursor protein of Claim 2, wherein said Ef-tu inhibitor is selected among the group consisting of GE2270A, GE37648A, Amythiamicin or a compound as represented in any of Formulas I - XI below:

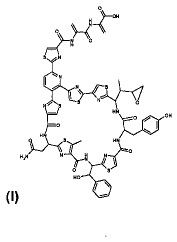

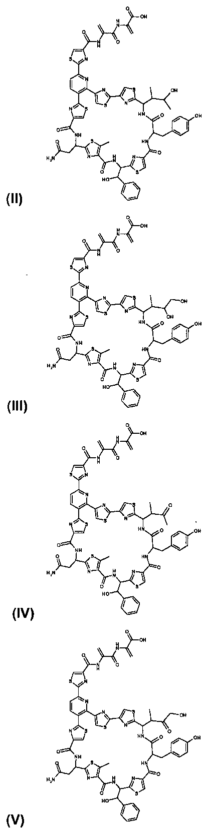

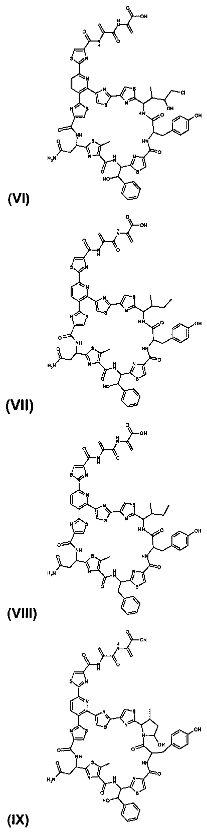

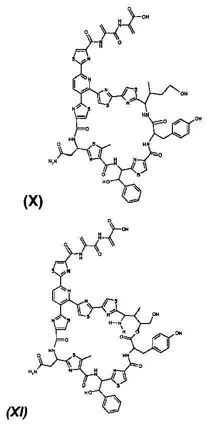

4. A nucleic acid comprising the nucleotide sequence encoding the thiopeptide precursor protein according to any of Claims 1-3.

5. The nucleic acid of Claim 4, comprising the nucleotide sequence of SEQ ID NO:5.

6. The nucleic acid of Claim 5, comprising the nucleotide sequence of SEQ ID NO: 6 or any fragment thereof comprising at least SEQ ID NO:5.

7. The nucleic acid according to any of claims 4-6, wherein said nucleic acid sequence is operatively linked to heterologous transcriptional and translational control sequence.

8. The nucleic acid of Claim 7, wherein said nucleic acid is an expression vector.

9. A polypeptide for the biosynthesis of thiopeptide, comprising an amino acid sequence selected among the group consisting of:
(i) any one of SEQ ID NO:23-34,
(ii) a variant of an amino acid sequence listed in (i), having no more than 1, 2, 3, 4, 5, 6 or 10 deleted, inserted or substituted amino acids when compared to the corresponding wild type amino acid sequence listed in (i) and retaining substantially the same enzymatic function.

10. A nucleic acid comprising a nucleotide sequence encoding a polypeptide of Claim 9.

11. The nucleic acid of Claim 10, wherein said nucleic acid sequence is operatively linked to heterologous transcriptional and translational sequence.

12. A host cell comprising the nucleic acid according to any of Claims 1-86, wherein said nucleic acid is not naturally found in the genome of said host cell.

13. The host cell of Claim 12, further comprising other genes required for thiopeptide biosynthesis.

14. The host cell of Claim 13, wherein said other genes required for thiopeptide biosynthesis comprises a nucleic acid as defined in any of Claims 10-11.

15. The host cell of any of Claim 13 or 14, which is selected among the group of species consisting of *Nonomuraea* sp., *Planobispora sp., Amycolatopsis* sp., *Escherichia coli,* Corynebacterium sp., Bacillus sp. and *Streptomyces* sp. such as *Streptomyces lividans, Streptomyces coelicolor, Streptomyces albus, Streptomyces ramocissimus Streptomyces collinus, Streptomyces fradiae, Streptomyces azureus* or *Streptomyces griseus,* and wherein said host cell is rendered resistant to said thiopeptide.

16. A mutant microorganism, wherein said mutant microorganism no longer express a gene encoding the thiopeptide precursor protein as defined in any of Claims 1-8 when compared to the corresponding wild-type microorganism.

17. The mutant microorganism of Claim 16, wherein said mutation is a disruption of the gene encoding the thiopeptide precursor protein as defined in any of Claims 1-8 in the corresponding wild-type microorganism.

18. The mutant microorganism of Claim 17, wherein said microorganism is a *Nonomuraea* sp. and said mutation is a disruption of the gene comprising SEQ ID NO:15 or SEQ ID NO:16.

19. The mutant microorganism of any of Claims 16-18, wherein said mutant is further transformed with a nucleic acid according to any of Claims 1-8.

20. A mutant microorganism, wherein said mutant microorganism no longer express one or more genes encoding one or more polypeptides as defined in Claim 9.

21. A polypeptide for the biosynthesis of thiopeptide, comprising an amino acid sequence selected among the group consisting of:
(i) any one of SEQ ID NO: 47-60,
(ii) a variant of an amino acid sequence listed in (i), having no more than 1, 2, 3, 4, 5, 6 or 10 deleted, inserted or substituted amino acids when compared to the corresponding wild type amino acid sequence listed in (i) and retaining substantially the same enzymatic or regulatory function
(iii) a variant of an amino acid sequence listed in (i), having at least 80% or at least 90% identity with one of the polypeptide listed in (i) and retaining substantially the same enzymatic or regulatory function.

22. A nucleic acid comprising a nucleotide sequence encoding a polypeptide of Claim 21.

23. The nucleic acid of Claim 22, wherein said nucleic acid sequence is operatively linked to heterologous transcriptional and translational sequence.

24. A host cell comprising the nucleic acid according to Claim 22 or 23, wherein said nucleic acid is not naturally found in the genome of said host cell.

25. A host cell according to Claim 24, further comprising other genes required for thiopeptide biosynthesis.

26. The host cell of Claim 25, wherein said other genes required for thiopeptide biosynthesis are selected among one or more of the group consisting of the nucleic acid encoding a thiopeptide precursor protein according to any of Claims 4-8, and the nucleic acid according to Claims 10-11.

27. A mutant microorganism, capable of producing thiopeptide that is deficient in the expression of one or more of a tailoring gene encoding one or more polypeptides as defined in Claim 21.

28. A method for producing a thiopeptide precursor protein, said method comprising the step of culturing a host cell according to claim 12, 13, 15 , or 24-26 under conditions appropriate for production of said thiopeptide precursor protein.

29. A method for producing thiopeptide compound, said method comprising the step of culturing a host cell according to claim 12, 13, 14, 15, or 24-26 under conditions appropriate for production of said thiopeptide compound.

30. The method of Claim 28 or 29, further comprising isolating said thiopeptide precursor or thiopeptide compound in a substantially pure form.

31. The method of any of Claim 29 or 30, wherein said thiopeptide compound is selected among the group consisting of : GE2270A, GE37648A, a compound as represented in any of Formulas I - XI as represented in Claim 5, amythiamicin, micrococcin, thiostrepton, nosiheptide, thiocillin, thiocins, nocathiacins, berninamycin, A10255B and radamycin.

32. A method of producing thiopeptide derivatives, comprising
(i) synthesizing an altered thiopeptide precursor in a host cell by gene expression of the sequence encoding said altered thiopeptide precursor in said host cell,
(ii) synthesizing said thiopeptide derivatives from said altered thiopeptide precursor.

33. The method of Claim 32, wherein said altered thiopeptide derivative precursor is a variant of SEQ ID NO:1,SEQ ID NO:5 or SEQ ID NO:11 as defined in any of Claim 1-11.

34. The method of Claim 32 or 33, wherein step ii) is performed *in vitro.*

35. The method of Claim 32 or 33, wherein step ii) is performed *in vivo* in the same host cell as step i).

36. A method of producing thiopeptide or thiopeptide derivatives, comprising
(i) providing a culture medium comprising a thiopeptide precursor protein as defined in any of 1-11,
(ii) culturing microorganisms in said culture medium, wherein said microorganism further comprises other genes required for thiopeptide biosynthesis.

37. The method of Claim 36, wherein said other genes required for thiopeptide biosynthesis are selected among those encoding a polypeptide selected among SEQ ID NO:23-34 and SEQ ID NO:47-60.

38. The method of Claim 36 or 37, wherein said microorganism is selected among the group of species consisting of *Nonomuraea* sp., *Planobispora sp., Amycolatopsis* sp. and *Streptomyces* sp. such as *Streptomyces lividans , Streptomyces coelicolor, Streptomyces albus, Streptomyces ramocissimus Streptomyces collinus, Streptomyces fradiae, Streptomyces azureus* or *Streptomyces griseus, wherein said microorganism is a strain selected for resistance against said thiopeptide or thiopeptide derivative*
